# EUROPÄISCHE PATENTSCHRIFT

(11) **EP 2 051 975 B1**
(45) Veröffentlichungstag und Bekanntmachung des Hinweises auf die Patenterteilung: **12.09.2012**
(21) Anmeldenummer: 07729083.1
(22) Anmeldetag: 14.05.2007
(51) Int. Cl.: C07D 401/12, C07D 401/14, A61P 7/02, A61K 31/435, C07D 471/04

(54) **SUBSTITUIERTE PROLINAMIDE, DEREN HERSTELLUNG UND DEREN VERWENDUNG ALS ARZNEIMITTEL**
SUBSTITUTED PROLINAMIDES, PRODUCTION THEREOF AND THEIR USE AS DRUGS
PROLINAMIDES SUBSTITUÉS, PROCÉDÉ DE PRODUCTION ET UTILISATION DE CEUX-CI COMME MÉDICAMENTS

(30) Priorität: 16.05.2006 EP 06113977; 16.02.2007 EP 07102566
(43) Veröffentlichungstag der Anmeldung: 29.04.2009
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: GERLACH, Kai, 88441 Mittelbiberach (DE); PRIEPKE, Henning, 88447 Warthausen (DE); PFAU, Roland, 88400 Biberach (DE); WIENEN, Wolfgang, 88400 Biberach (DE); SCHULER-METZ, Annette, 89081 Ulm (DE); DAHMANN, Georg, 88448 Attenweiler (DE); NAR, Herbert, 88416 Ochsenhausen (DE)
(74) Vertreter: Hammann, Heinz
(86) Internationale Anmeldenummer: PCT/EP2007/054631
(87) Internationale Veröffentlichungsnummer: WO 2007/131982

(56) Entgegenhaltungen:
- WO-A-2004/087695
- WO-A-2004/110433
- WO-A-2005/058817
- WO-A-2005/092849
- WO-A2-2004/087646

## Beschreibung

WO 2004/087646 beschreibt Carbonylverbindungen mit Faktor Xa inhibierende Eigenschaften. WO 2004/110433 beschreibt Pyrrolidin-1,2-dicarbonsäure-1- [(4-ethinyl-phenyl)-amid]-2-[(phenyl)-amid] Derivaten als inhibitoren der Koagulationsfaktoren Xa und VIIa zur Behandlung von Thrombosen. WO 2004/087695 beschreibt Verfahren zur Herstellung von Pyrrolidin-1,2- dicarbonsäure-1-[(4-ethinyl-phenyl)-amid]-2-[(phenyl)-amid] Derivaten und 1- (Phenylcarbamoyl)-pyrrolidin-2-carbonsäure Derivate als Zwischenprodukte. WO 2005/092849 beschreibt Prolinylderivate zur Behandlung von Thrombose. WO 2005/058817 beschreibt Prolinylarylacetamide als inhibitoren des Koagulationsfaktors Xa. Gegenstand der vorliegenden Erfindung sind neue substituierte Prolinamide der allgemeinen Formel (I) 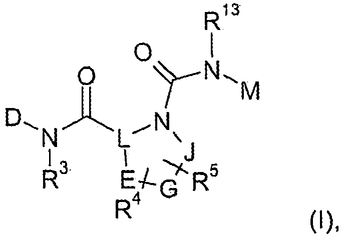 deren Tautomere, deren Enantiomere, deren Diastereomere, deren Gemische und deren Salze, insbesondere deren physiologisch verträgliche Salze mit anorganischen oder organischen Säuren oder Basen, welche wertvolle Eigenschaften aufweisen.

Die Verbindungen der obigen allgemeinen Formel (I) sowie deren Tautomere, deren Enantiomere, deren Diastereomere, deren Gemische und deren Salze, insbesondere deren physiologisch verträgliche Salze mit anorganischen oder organischen Säuren oder Basen, und deren Stereoisomere weisen wertvolle pharmakologische Eigenschaften auf, insbesondere eine antithrombotische Wirkung und eine Faktor Xa-inhibierende Wirkung.

Gegenstand der vorliegenden Anmeldung sind neue Verbindungen der obigen allgemeinen Formel (I), deren Herstellung, die die pharmakologisch wirksamen Verbindungen enthaltenden Arzneimittel, deren Herstellung und Verwendung.

Eine 1. Ausführungsform der vorliegenden Erfindung umfasst diejenigen Verbindungen der allgemeinen Formel (I), in denen
D ein substituiertes bicyclisches Ringsystem der Formel 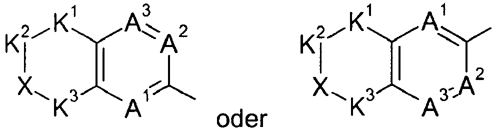 darstellt, in dem
K¹ eine Bindung, oder eine -CH₂-, -CHR^{7a}, -CR^{7b}R^{7c}- oder -C(O)-Gruppe bedeutet, und wobei
R^{7a}/R^{7b}/R^{7c}
jeweils unabhängig voneinander ein Fluoratom, eine Hydroxy-, C₁₋₅-Alkyloxy-, Amino-, C₁₋₅-Alkylamino-, Di-(C₁₋₅-alkyl)-amino-, C₃₋₅-Cycloalkylenimino-, C₁₋₅-Alkylcarbonylaminogruppe, eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkylgruppe bedeutet,
wobei nicht gleichzeitig beide Reste R^{7b}/R^{7c} über ein Heteroatom an das Ringkohlenstoffatom gebunden sein können, außer -C(R^{7b}R^{7c})- entspricht einer ―CF₂-Gruppe, oder
R^{7a} eine durch Fluor-, Chlor- , Brom-, Methyl-, Methoxy-, Amino- oder Nitro-substituierte Phenyl- oder monocyclische Heteroarylgruppe bedeutet, oder
zwei Reste R^{7b}/R^{7c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, 1,3-Dioxolan-, 1,4-Dioxan-, Hexahydropyridazin-, Piperazin-, Thiomorpholin-, Morpholin-, 2-Imidazolidinon-, 2-Oxazolidinon-, Tetrahydro-2(1H)-pyrimidinonoder [1,3]Oxazinan-2-on-ring bilden können,
wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder bei dem eine ―CH₂-Gruppe neben einem N-Atom durch eine ―CO-Gruppe ersetzt sein kann, und/oder dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid oder Sulfongruppe oxidiert sein kann,
K² und K³ jeweils unabhängig voneinander eine -CH₂-, -CHR^{8a}-, -CR^{8b}R^{8c}-oder eine -C(O)-Gruppe bedeuten, wobei
R^{8a}/R^{8b}/R^{8c}
jeweils unabhängig voneinander eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
oder zwei Reste R^{8b}/R^{8c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, Hexahydropyridazin-, Tetrahydro-2(1H)-pyrimidinon-, [1,3]Oxazinan-2-on-ring bilden können,
wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder bei dem eine ―CH₂-Gruppe neben einem Stickstoffatom durch eine ―CO-Gruppe ersetzt sein kann, und/oder dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid- oder Sulfongruppe oxidiert sein kann,
mit der Massgabe, dass ein durch R^{8b} oder R^{8c} eingebrachtes Heteroatom nicht durch nur ein Kohlenstoffatom von X in Formel (I) entfernt sein darf, und
insgesamt maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine CF₂-, Sulfen-, Sulfonoder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂-, C₃₋₆-Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkylsulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅-Alkyloxycarbonyl-, C₄₋₇-Cycloalkyleniminocarbonyl-gruppe bedeutet,
wobei die in den voranstehend genannten Gruppen befindlichen Methylen- und Methylgruppen zusätzlich durch eine C₁₋₃Alkyl-, Carboxy-, C₁₋₅-Alkoxycarbonylgruppe substituiert sein können,
oder durch eine Hydroxy-, C₁₋₅-Alkyloxy-, Amino-, C₁₋₅-Alkylamino-, C₁₋₅-Dialkylamino- oder C₄₋₇-Cycloalkyleniminogruppe substituiert sein können, sofern die Methylen- oder Methylgruppen nicht direkt an ein Heteroatom aus der Gruppe O, N oder S gebunden sind, und/oder ein bis drei
Wasserstoffatome durch Fluoratome ersetzt sein können, sofern die Methylen- oder Methylgruppen nicht direkt an ein Heteroatom aus der Gruppe O, N oder S gebunden sind,
und in dem
A¹ entweder N oder CR¹⁰ bedeutet,
A² entweder N oder CR¹¹ bedeutet,
A³ entweder N oder CR¹² bedeutet,
wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, C₂₋₅ -Alkenyl-, C₂₋₅-Alkinyl-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten, und
-L-E-G-J- eine ―C-C-C-C- oder ―C-C=C-C-Gruppe bedeutet, die durch R⁴ und R⁵ substituiert sein kann, und
R³ ein Wasserstoffatom oder eine C₁₋₃-Alkylgruppe bedeutet, und
R⁴ ein Wasserstoffatom oder
eine geradkettige oder verzweigte C₁₋₆-Alkyl-, C₂₋₆-Alkenyl- oder C₂₋₆-Alkinylgruppe bedeutet,
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls jeweils unabhängig voneinander durch ein bis zwei Substituenten ausgewählt aus einer C₃₋₅-Cycloalkylgruppe, einer Nitril-, Hydroxy-oder C₁₋₅-Alkyloxygruppe, wobei die Wasserstoffatome der C₁₋₅-Alkyloxygruppe gegebenenfalls ganz oder teilweise durch Fluoratome ersetzt sein können, einer Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Aminosulfonyl-, C₁₋₅-Alkylaminosulfonyl-, Di-(C₁₋₅-alkyl)-aminosulfonyl-, C₄₋₇-Cycloalkyleniminosulfonyl-, Amino-, C₁₋₅-Alkylamino-, Di-(C₁₋₅-alkyl)-amino-, C₁₋₅-Alkylcarbonylamino-, C₁₋₅-Alkylsulfonylamino-, *N*-(C₁₋₅-Alkylsulfonyl)-C₁₋₅-alkylamino-, C₃₋₆-Cycloalkylcarbonylaminogruppe, oder einer Morpholinyl-, Thiomorpholinyl-, Pyrrolidinyl-, Piperidinyl-, Piperazinyl-, Tetrahydrofuranyl-, Tetrahydropyranyl-gruppe substituiert sein können, wobei die vorgenannten Carbo- und Hetero-cyclen im Ring jeweils durch 1-4 C₁₋₃-Alkyl- oder C₁₋₃-Alkylcarbonylgruppen oder jeweils durch 1-2 Oxogruppen substituiert sein können, und/oder
wobei die Wasserstoffatome der sp²-hybridisierten Kohlenstoffatome der geradkettigen oder verzweigten C₂₋₆-Alkenyl-gruppe gegebenenfalls ganz oder teilweise durch Fluoratome ersetzt sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, oder
eine Phenyl-, mono- oder bicyclische Heteroaryl-, Phenyl-C₁₋₅-alkyl- oder mono- oder bicyclische Heteroaryl-C₁₋₅-alkylgruppe, die im Phenyl- oder Heteroarylteil gegebenenfalls ein- bis dreifach durch gleiche oder unterschiedliche Substituenten ausgewählt aus der Gruppe bestehend aus Fluor-, Chlor-, Brom-und Iodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein kann,
bedeutet, und
wenn -L-E-G-J- eine ―C-C-C-C-Gruppe bedeutet, kann R⁴ an E oder G auch ein Fluoratom oder eine Hydroxy-, Methoxy-, C₃₋₅-Alkenyl-oxy-, C₃₋₅-Alkinyl-oxy-, C₂₋₅-Alkyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₃-alkyloxy-, Heteroaryl-C₀₋₃-alkyloxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen,
wobei die in den vorgenannten Alkyl- oder Cycloalkylresten vorhandenen Methyl oder Methylengruppen jeweils unabhängig voneinander durch einen Substituenten ausgewählt aus der Gruppe Morpholinyl-, Thiorriorpholinyl-, Pyrrolidinyl-, Piperidinyl-, Piperazinyl-, Tetrahydrofuranyl-, Tetrahydropyranyl, Dimethylaminocarbonyl-, C₁₋₅Alkyloxycarbonyl-, Carboxy-, Methyl-, Hydroxy-, Methoxy- oder Amino- substituiert sein können, und
die vorgenannten Phenyl- oder Heteroarylreste gegebenenfalls ein- bis dreifach durch gleiche oder unterschiedliche Substituenten ausgewählt aus der Gruppe bestehend aus Fluor-, Chlor-, Brom-und Iodatomen, und C₁₋₅-Alkyl-, Trifluormethyl-, Amino-, C₁₋₅-alkylamino-, Di-(C₁₋₅-alkyl)-amino-, Hydroxy-, C₁₋₅-Alkyloxy-, Mono-, Di-oder Trifluormethoxy-, Carboxy- und C₁₋₅-Alkyloxycarbonylgruppe, substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine ―O-O- oder ―S-O-Bindung bilden,
ausgeschlossen ist, und
R⁵ ein Wasserstoffatom, eine C₁₋₅Alkyl-, C₂₋₅Alkenyl- oder C₂₋₅Alkinyl- oder eine Phenyl-C₀₋₅Alkylgruppe bedeutet, wobei die Alkylgruppe durch eine Hydroxy-, Methoxy-, Hydroxycarbonyl- oder C₁₋₅Alkoxycarbonyl-gruppe substituiert sein kann, oder sofern R⁵ mit E oder G verknüpft ist auch eine Hydroxy- oder Methoxy-gruppe darstellen kann, oder
R⁴ und R⁵ sofern sie an das selbe Kohlenstoffatom gebunden sind, zusammen mit dem Kohlenstoffatom eine -C=O-Gruppe, oder eine - CF₂- Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-7-gliedrigen Carbocyclus oder einen einfach ungesättigten 5-7 gliedrigen Carbocyclus bilden können,
wobei eines der Kohlenstoffkettenglieder dieses Cyclus durch ein Sauerstoff- oder Schwefelatom oder eine -NH-, -N(C₁₋₅-Alkyl)-, - N(C₁₋₄-Alkylcarbonyl)- oder eine Carbonyl-, Sulfinyl- oder Sulfonylgruppe ersetzt sein kann, und/oder
wobei zwei direkt benachbarte Kohlenstoffkettenglieder dieser C₄₋₇-Carbocyclen zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei vier direkt benachbarte Kohlenstoffkettenglieder dieser C₅₋₇-Carbocyclen zusammen durch eine -O-CH₂-CH₂-O-Gruppe ersetzt sein können, und/oder
wobei 1 bis 3 Kohlenstoffatome dieser 3-7-gliedrigen Cyclen gegebenenfalls unabhängig voneinander durch jeweils ein oder zwei Fluoratome oder eine oder zwei C₁₋₅-Alkyl-gruppen oder eine Hydroxy-, Formyloxy-, C₁₋₅-Alkyloxy-, C₁₋₅-Alkylcarbonyloxy-, Amino-, C₁₋₅-Alkylamino-, Di-(C₁₋₅-alkyl)-amino-, C₄₋₇-Cycloalkylenimino-, C₁₋₅-Alkylcarbonylamino-, C₃₋₆-Cycloalkylcarbonylamino-, Nitril-, Carboxy-C₁₋₅-alkyl-, C₁₋₅-Alkyloxycarbonyl-C₁₋₅-alkyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder C₄₋₇-Cycloalkyleniminocarbonylgruppe substituiert sein können,
mit der Maßgabe, dass ein solcher, zusammen aus R⁴ und R⁵ gebildeter Cyclus,
in dem zwei Stickstoffatome oder ein Stickstoff und ein Sauerstoffatom im Cyclus durch genau eine gegebenenfalls substituierte -CH₂-Gruppe voneinander getrennt sind, und/oder
in der zwei Atome im Ring eine ―O-O- oder ―S-O-Bindung bilden,
ausgeschlossen ist,
oder das Fragment 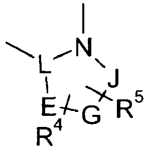 die Gruppe 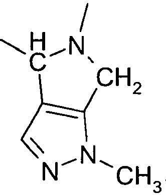 bedeutet,
R¹³ ein Wasserstoffatom oder eine C₁₋₅ Alkylgruppe bedeutet,
M einen gegebenenfalls durch R² und R⁶ substituierten Phenyl-, Thienyl-oder Pyridylring bedeutet, in dem
R² ein Fluor-, Chlor-, Brom- oder Jod-atom oder eine Methyl-, Ethyl-, Vinyl-, Methoxy-, Ethinyl-, Cyano- oder ―C(O)NH₂-Gruppe darstellt, und
R⁶ ein Wasserstoff-, Fluor-, Chlor-, Brom- oder Jod-atom oder eine Hydroxy-, Methoxy-, Trifluormethoxy-, eine gegebenenfalls durch Fluoratome substituierte C₁₋₃-Alkyl-, Cyano-, Amino-, oder NH₂C(O)-Gruppe darstellt,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome, und
die 5-gliedrige Heteroarylgruppe eine gegebenenfalls durch eine C₁₋₃-Alkylgruppe substituierte Iminogruppe, ein Sauerstoff- oder Schwefelatom, oder eine gegebenenfalls durch eine C₁₋₃-Alkylgruppe substituierte Iminogruppe oder ein Sauerstoff- oder Schwefelatom und zusätzlich ein oder zwei Stickstoffatome, oder
eine gegebenenfalls durch eine C₁₋₃-Alkylgruppe substituierte Iminogruppe und drei Stickstoffatome,
enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann,
und die Bindung über ein Stickstoffatom oder über ein Kohlenstoffatom des heterocyclischen Teils oder eines ankondensierten Phenylrings erfolgt,
und wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
und wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

Beispiele für monocyclische Heteroarylgruppen sind die Pyridyl-, N-Oxy-pyridyl-, Pyrazolyl-, Pyridazinyl-, Pyrimidinyl-, Pyrazinyl-, [1,2,3]Triazinyl-, [1,3,5]Triazinyl-, [1,2,4]Triazinyl-, Pyrrolyl-, Imidazolyl-, [1,2,4]Triazolyl-, [1,2,3]Triazolyl-, Tetrazolyl-, Furanyl-, Isoxazolyl-, Oxazolyl-, [1,2,3]Oxadiazolyl-, [1,2,4]Oxadiazolyl-, Furazanyl-, Thienyl-, Thiazolyl-, Isothiazolyl-, [1,2,3]Thiadiazolyl-, [1,2,4]Thiadiazolyl- oder [1,2,5]Thiadiazolyl-Gruppe.

Beispiele für bicyclische Heteroarylgruppen sind die Benzimidazolyl, Benzofuranyl-, Benzo[c]furanyl-, Benzothiophenyl-, Benzo[c]thiophenyl-, Benzothiazolyl-, Benzo[*c*]isothiazolyl-, Benzo[d]isothiazolyl-, Benzooxazolyl-, Benzo[c]isoxazolyl-, Benzo[*d*]isoxazolyl-, Benzo[1,2,5]oxadiazolyl-, Benzo[1,2,5]thiadiazolyl-, Benzo[1,2,3]thiadiazolyl-, Benzo[*d*][1,2,3]triazinyl-, Benzo[1,2,4]triazinyl-, Benzotriazolyl-, Cinnolinyl-, Chinolinyl-, N-Oxy-chinolinyl-, Isochinolinyl-, Chinazolinyl-, N-Oxy-chinazolinyl-, Chinoxalinyl-, Phthalazinyl-, Indolyl-, Isoindolyl- oder 1-Oxa-2,3-diaza-indenyl-Gruppe.

Beispiele für die voranstehend in den Definitionen erwähnten C₁₋₆-Alkylgruppen sind die Methyl-, Ethyl-, 1-Propyl-, 2-Propyl-, *n*-Butyl-, *sec*-Butyl-, *tert*-Butyl-, 1-Pentyl-, 2-Pentyl-, 3-Pentyl-, *neo*-Pentyl-, 3-Methyl-2-butyl-, 1-Hexyl-, 2-Hexyl-, 3-Hexyl, 3-Methyl-2-pentyl-, 4-Methyl-2-pentyl-, 3-Methyl-3-pentyl-, 2-Methyl-3-pentyl-, 2,2-Dimethyl-3-butyl- oder 2,3-Dimethyl-2-butyl-Gruppe.

Beispiele für die voranstehend in den Definitionen erwähnten C₁₋₅-Alkyloxygruppen sind die Methyloxy-, Ethyloxy-, 1-Propyloxy-, 2-Propyloxy-, *n*-Butyloxy-, *sec*-Butyloxy-, *tert*-Butyloxy-, 1-Pentyloxy-, 2-Pentyloxy-, 3-Pentyloxy- oder *neo*-Pentyloxy-Gruppe.

Beispiele für die voranstehend in den Definitionen erwähnten C₂₋₅-Alkenylgruppen sind die Ethenyl-, 1-Propen-1-yl-, 2-Propen-1-yl-, 1-Buten-1-yl-, 2-Buten-1-yl-, 3-Buten-1-yl-, 1-Penten-1-yl-, 2-Penten-1-yl-, 3-Penten-1-yl-, 4-Penten-1-yl-, 1-Hexen-1-yl-, 2-Hexen-1-yl-, 3-Hexen-1-yl-, 4-Hexen-1-yl-, 5-Hexen-1-yl-, But-1-en-2-yl-, But-2-en-2-yl-, But-1-en-3-yl-, 2-Methyl-prop-2-en-1-yl-, Pent-1-en-2-yl-, Pent-2-en-2-yl-, Pent-3-en-2-yl-, Pent-4-en-2-yl-, Pent-1-en-3-yl-, Pent-2-en-3-yl-, 2-Methyl-but-1-en-1-yl-, 2-Methyl-but-2-en-1-yl-, 2-Methyl-but-3-en-1-yl- oder 2-Ethyl-prop-2-en-1-yl -Gruppe,

Beispiele für die voranstehend in den Definitionen erwähnten C₂₋₅-Alkinylgruppen sind die Ethinyl-, 1-Propinyl-, 2-Propinyl-, 1-Butin-1-yl-, 1-Butin-3-yl-, 2-Butin-1-yl-, 3-Butin-1-yl-, 1-Pentin-1-yl-, 1-Pentin-3-yl-, 1-Pentin-4-yl-, 2-Pentin-1-yl-, 2-Pentin-3-yl-, 3-Pentin-1-yl-, 4-Pentin-1-yl-, 2-Methyl-1-butin-4-yl-, 3-Methyl-1-butin-1-yl- oder 3-Methyl-1-butin-3-yl-Gruppe.

Eine 2. Ausführungsform der vorliegenden Erfindung umfasst diejenigen Verbindungen der allgemeinen Formel (I), in denen E, G, J, L, M, R³-R⁵ und R¹³ wie in Ausführungsform 1 beschrieben definiert sind und in denen
D ein substituiertes bicyclisches Ringsystem der Formel 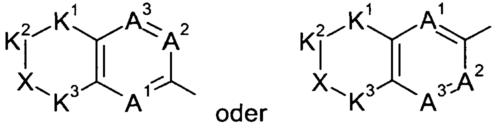 darstellt, in dem
K¹ eine -CH₂-, -CHR^{7a}-, -CR^{7b}R^{7c}- oder eine -C(O)-Gruppe bedeutet, und wobei
R^{7a}/R^{7b}/R^{7c} jeweils unabhängig voneinander ein Fluoratom, eine Hydroxy-, C₁₋₅-Alkyloxy-, eine C₁₋₅-Alkylgruppe bedeutet,
wobei nicht gleichzeitig beide Reste R^{7b}/R^{7c} über ein Heteroatom an das Ringkohlenstoffatom gebunden sein können, außer - C(R^{7b}R^{7c})- entspricht einer ―CF₂-Gruppe, oder
zwei Reste R^{7b}/R^{7c} zusammen mit dem Ringkohlenstoffatom einen 3-gliedrigen Carbocyclus bilden können und
K² und K³ jeweils unabhängig voneinander eine -CH₂-, -CHR^{8a}-, -CR^{8b}R^{8c}-oder eine -C(O)- Gruppe bedeuten, wobei
R^{8a}/R^{8b}/R^{8c}
jeweils unabhängig voneinander eine C₁₋₅-Alkylgruppe bedeutet, und/oder
zwei Reste R^{8b}/R^{8c} zusammen mit dem Ringkohlenstoffatom einen 3-gliedrigen gesättigten Carbocyclus bilden können
und
insgesamt maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine Sulfen-, Sulfon-, -CF₂-oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂- oder eine C₃₋₆-Cycloalkylgruppe bedeutet,
und in dem
A¹ entweder N oder CR¹⁰ bedeutet,
A² entweder N oder CR¹¹ bedeutet,
A³ entweder N oder CR¹² bedeutet,
wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten.

Eine 3. Ausführungsform der vorliegenden Erfindung umfasst diejenigen Verbindungen der Ausführungsformen 1 oder 2, in denen E, G, J, L, M, R³-R⁵, R¹³ , D, K¹, K² und K³ wie in der ersten oder zweiten Ausführungsform beschrieben definiert sind, und in denen
X eine NR¹-Gruppe bedeutet, in der
R¹ ein Wasserstoffatom oder eine C₁₋₅-Alkyl-, Allyl- oder Cyclopropylgruppe bedeutet, und
A¹ CR¹⁰ bedeutet,
A² CR¹¹ bedeutet,
A³ entweder N oder CR¹² bedeutet,
wobei R¹⁰, R¹¹ und R¹² jeweils unabhängig voneinander
ein Wasserstoff-, Fluor- oder Chloratom, oder eine Methyl-, CF₃-, Cyano-, Carboxy-, C₁₋₅-Alkyloxycarbonyl-, Hydroxy-, Methoxy-, CF₃O- CHF₂O-, CH₂FO-Gruppe bedeuten.

Eine 4. Ausführungsform der vorliegenden Erfindung umfasst diejenigen Verbindungen der allgemeinen Formel (I) in denen D, E, G, J, L, M, R³ und R¹³ wie in Ausführungsform 1, 2 oder 3 beschrieben definiert sind, und in denen
R⁴ ein Wasserstoffatom oder
eine geradkettige oder verzweigte C₁₋₆-Alkylgruppe bedeutet, wobei die Wasserstoffatome der Methylen- und/oder Methylfragmente der geradkettigen oder verzweigten C₁₋₆-Alkylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkylgruppe gegebenenfalls jeweils unabhängig voneinander durch einen Substituenten ausgewählt aus einer Hydroxy-, C₁₋₅-Alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Amino-, C₁₋₅-Alkylamino-, Di-(C₁₋₅-alkyl)-amino-, C₁₋₅-Alkylcarbonylamino-, C₁₋₅-Alkyl-sulfonylamino-, *N*-(C₁₋₅-Alkylsulfonyl)-C₁₋₅-alkylamino-, C₃₋₆-Cycloalkylcarbonylaminogruppe substituiert sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, bedeutet, und
wenn -L-E-G-J- eine ―C-C-C-C-Gruppe bedeutet, kann R⁴ an E oder G auch ein Fluoratom oder eine Hydroxy-, Methoxy-, C₃₋₅-Alkenyl-oxy-, C₃₋₅-Alkinyl-oxy-, C₂₋₅-Alkyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₂-alkyloxygruppe darstellen, die im Phenylring durch 1-2 Fluoratome oder Methoxygruppen substituiert sein kann, eine Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen,
wobei die in den vorgenannten Alkyl- oder Cycloalkylresten vorhandenen Methyl oder Methylengruppen jeweils unabhängig voneinander durch einen Substituenten ausgewählt aus der Gruppe Dimethylaminocarbonyl-, C₁₋₅Alkyloxycarbonyl-, Carboxy-, Methyl-, Hydroxy-, Methoxy- oder Amino- substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine ―O-O- oder ―S-O-Bindung bilden,
ausgeschlossen ist, und
R⁵ ein Wasserstoffatom oder eine C₁₋₅Alkyl-, Allyl-, Propargyl- oder Benzylgruppe bedeutet, oder sofern R⁵ mit E oder G verknüpft ist, auch eine Hydroxy- oder Methoxy-gruppe darstellen kann oder
R⁴ und R⁵ sofern sie an das selbe Kohlenstoffatom gebunden sind, zusammen mit dem Kohlenstoffatom eine -C=O-Gruppe, oder eine - CF₂- Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-7-gliedrigen Carbocyclus bilden können,
wobei eines der Kohlenstoffkettenglieder dieses Cyclus durch ein Sauerstoff- oder Schwefelatom oder eine -NH-, -N(C₁₋₅-Alkyl)-, - N(C₁₋₄-Alkylcarbonyl)- oder eine Carbonyl-, Sulfinyl- oder Sulfonylgruppe ersetzt sein kann, und/oder
wobei zwei direkt benachbarte Kohlenstoffkettenglieder dieser C₄₋₇-Carbocyclen zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei vier direkt benachbarte Kohlenstoffkettenglieder dieser C₅₋₇-Carbocyclen zusammen durch eine -O-CH₂-CH₂O-Gruppe ersetzt sein können,
mit der Maßgabe, dass ein solcher, zusammen aus R⁴ und R⁵ gebildeter Cyclus,
in dem zwei Stickstoffatome oder ein Stickstoff und ein Sauerstoffatom im Cyclus durch genau eine gegebenenfalls substituierte -CH₂-Gruppe voneinander getrennt sind, und/oder
in der zwei Atome im Ring eine ―O-O- oder ―S-O-Bindung bilden,
ausgeschlossen ist.

Eine 5. Ausführungsform der vorliegenden Erfindung umfasst diejenigen Verbindungen der Ausführungsformen 1, 2, 3 oder 4, in denen D, M, R³ und R¹³ wie in Ausführungsform 1, 2, 3 oder 4 beschrieben definiert sind, und in denen
-L-E-G-J- eine ―C-C-C-C-Gruppe bedeutet, die durch R⁴ und R⁵, die wie oben in den Ausführungsformen 1, 2, 3 oder 4 definiert sind, substituiert sein kann.

Eine 6. Ausführungsform der vorliegenden Erfindung umfasst diejenigen Verbindungen der Ausführungsformen 1, 2, 3, 4 oder 5, in denen
D ein substituiertes bicyclisches Ringsystem der allgemeinen Formel 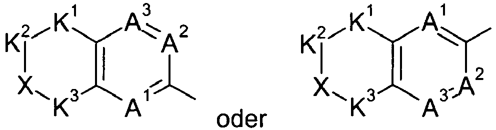 darstellt, in dem
K¹ eine -CH₂-, -CHR^{7a}-, -CR^{7b}R^{7c}- oder eine -C(O)-Gruppe bedeutet, wobei
R^{7a} eine C₁₋₂-Alkylgruppe bedeutet und
R^{7b}/R^{7c} jeweils unabhängig voneinander eine Hydroxy-, Methoxy-oder eine C₁₋₃-Alkylgruppe bedeutet,
wobei nicht gleichzeitig beide Reste R^{7b}/R^{7c} über ein Sauerstoffatom an das Ringkohlenstoffatom gebunden sein können,oder
zwei Reste R^{7b}/R^{7c} zusammen mit dem Ringkohlenstoffatom einen 3-gliedrigen Carbocyclus bilden können,
und
K² und K³ jeweils unabhängig voneinenader
eine -CH₂-, -CHR^{8a}- oder eine -CR^{8b}R^{8c}-Gruppe bedeuten, wobei
R^{8a}/R^{8b}/R^{8c}
jeweils unabhängig voneinander eine C₁₋₃-Alkylgruppe bedeutet, und/oder
zwei Reste R^{8b}/R^{8c} zusammen mit dem Ringkohlenstoffatom einen 3-gliedrigen gesättigten Carbocyclus bilden können
und
insgesamt maximal vier Reste ausgewählt aus R^{7a}, R^{7b}, R^{7c}, R^{8a}, R^{8b} und R^{8c} vorhanden sein dürfen, und
X eine NR¹-Gruppe bedeutet, in der
R¹ ein Wasserstoffatom oder eine C₁₋₃-Alkyl-, Allyl- oder Cyclopropylgruppe bedeutet, und
A¹ CR¹⁰ bedeutet,
A² CR¹¹ bedeutet,
A³ CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O- CHF₂O-, CH₂FO-Gruppe bedeuten, und
-L-E-G-J- eine ―C-C-C-C-Gruppe bedeutet, die durch R⁴ und R⁵ substituiert sein kann, und
R³ ein Wasserstoffatom bedeutet, und
R⁴ ein Wasserstoffatom oder
eine geradkettige oder verzweigte C₁₋₃-Alkylgruppe bedeutet, wobei die Wasserstoffatome der Methylen- und/oder Methylfragmente der geradkettigen oder verzweigten C₁₋₆-Alkylgruppe gegebenenfalls unabhängig voneinander durch einen Substituenten ausgewählt aus einer Hydroxy-, C₁₋₅-Alkyloxy-, Carboxy-, C₁₋₅-Alkyloxycarbonyl-gruppe substituiert sein können, oder
wenn R⁴ an E oder G angebunden ist auch ein Fluoratom oder eine Hydroxy-, Methoxy-, C₃₋₅-Alkenyl-oxy-, C₂₋₅-Alkyl-oxy-, C₃₋₆-Cycloalkyloxy-, C₁₋₅-Alkylaminocarbonyloxy-, Di(C₁₋₅-alkyl)aminocarbonyloxy- oder C₄₋₇-Cycloalkyleniminocarbonyloxy-gruppe darstellen kann,
mit der Maßgabe,
dass zwei Heteroatome aus der Gruppe Sauerstoff und Stickstoff durch genau eine gegebenenfalls substituierte -CH₂-Gruppe voneinander getrennt sind,
ausgeschlossen ist, und
R⁵ ein Wasserstoffatom oder eine C₁₋₅Alkyl-, Allyl- oder Benzylgruppe bedeutet, oder sofern R⁵ mit E oder G verknüpft ist auch eine Hydroxy-oder Methoxy-gruppe darstellen kann, oder
R⁴ und R⁵ sofern sie an das selbe Kohlenstoffatom gebunden sind, zusammen mit dem Kohlenstoffatom eine -C=O-Gruppe, oder eine - CF₂- Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-6-gliedrigen Carbocyclus bilden können,
wobei vier direkt benachbarte Kohlenstoffkettenglieder dieser C₅₋₆-Carbocyclen zusammen durch eine -O-CH₂-CH₂O-Gruppe ersetzt sein können,
R¹³ ein Wasserstoffatom bedeutet,
M einen durch R² in 4-Position substituierten Phenyl- oder durch R² in 5-Position substituierten Pyridylring bedeutet, in dem
R² ein Fluor-, Chlor-, Bromatom, eine Methoxy- oder Ethinyl-Gruppe darstellt, und
R⁶ ein Wasserstoff- oder Fluoratom darstellt.

Eine 7. Ausführungsform der vorliegenden Erfindung umfasst diejenigen Verbindungen der Ausführungsformen 1, 2, 3, 4, 5 oder 6 in denen D, R3, R13 und M wie oben definiert sind und in denen der zentrale Ring 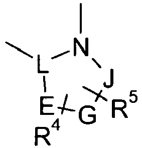 entweder 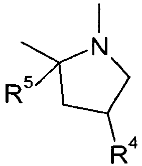 oder 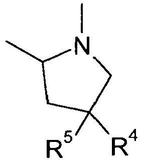 bedeutet.

Eine 8. Ausführungsform der vorliegenden Erfindung umfasst diejenigen Verbindungen der Ausführungsformen 1, 2, 3, 4, 5, 6 oder 7 in denen
D ein substituiertes bicyclisches Ringsystem der allgemeinen Formel 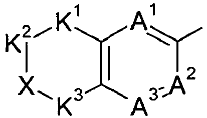 darstellt.

Eine 9. Ausführungsform der vorliegenden Erfindung umfasst diejenigen Verbindungen der Ausführungsformen 1, 2, 3, 4, 5, 6, 7 oder 8 die an den Kettengliedern G und L des 5-gliedrigen zentralen Rings R- konfiguriert sind.

Erfindungsgemäß erhält man die Verbindungen der allgemeinen Formel (I) nach an sich bekannten Verfahren, beispielsweise nach folgenden Verfahren:
(a) Die Herstellung einer Verbindung der allgemeinen Formel (IIa) oder (IIb) 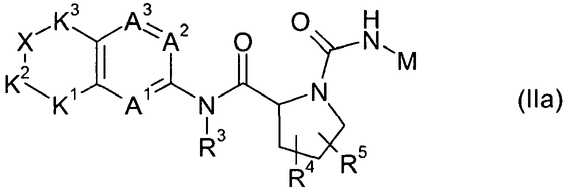 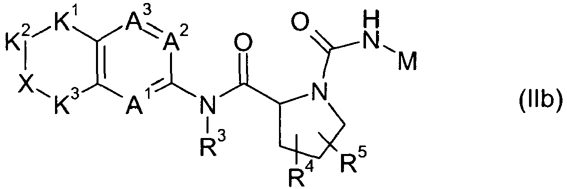 in der A¹ bis A³, K¹ bis K³, M und R¹ bis R⁶ wie in Ausführungsform 1 erwähnt definiert sind,
   und die gegebenenfalls an vorhandenen Amino-, Hydroxy-, Carboxy- oder Thiolgruppen durch gängige Schutzgruppen wie beispielsweise denen in T.W. Greene, P.G.M. Wuts in "Protective Groups in Organic Synthesis", Wiley, 1991 und 1999, beschriebenen geschützt sein kann, und deren Schutzgruppen in
   Literatur nach bekannter Methode abgespalten werden können, wird in den Ausführungsbeispielen beschrieben oder kann beispielsweise nach einem der folgenden Formelschemata 1 und 2 oder in Analogie zu den Syntheseverfahren die in WO2004/87695, WO2004/87646 oder in WO2003/45912 beschrieben sind durchgeführt werden. 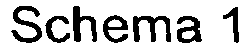 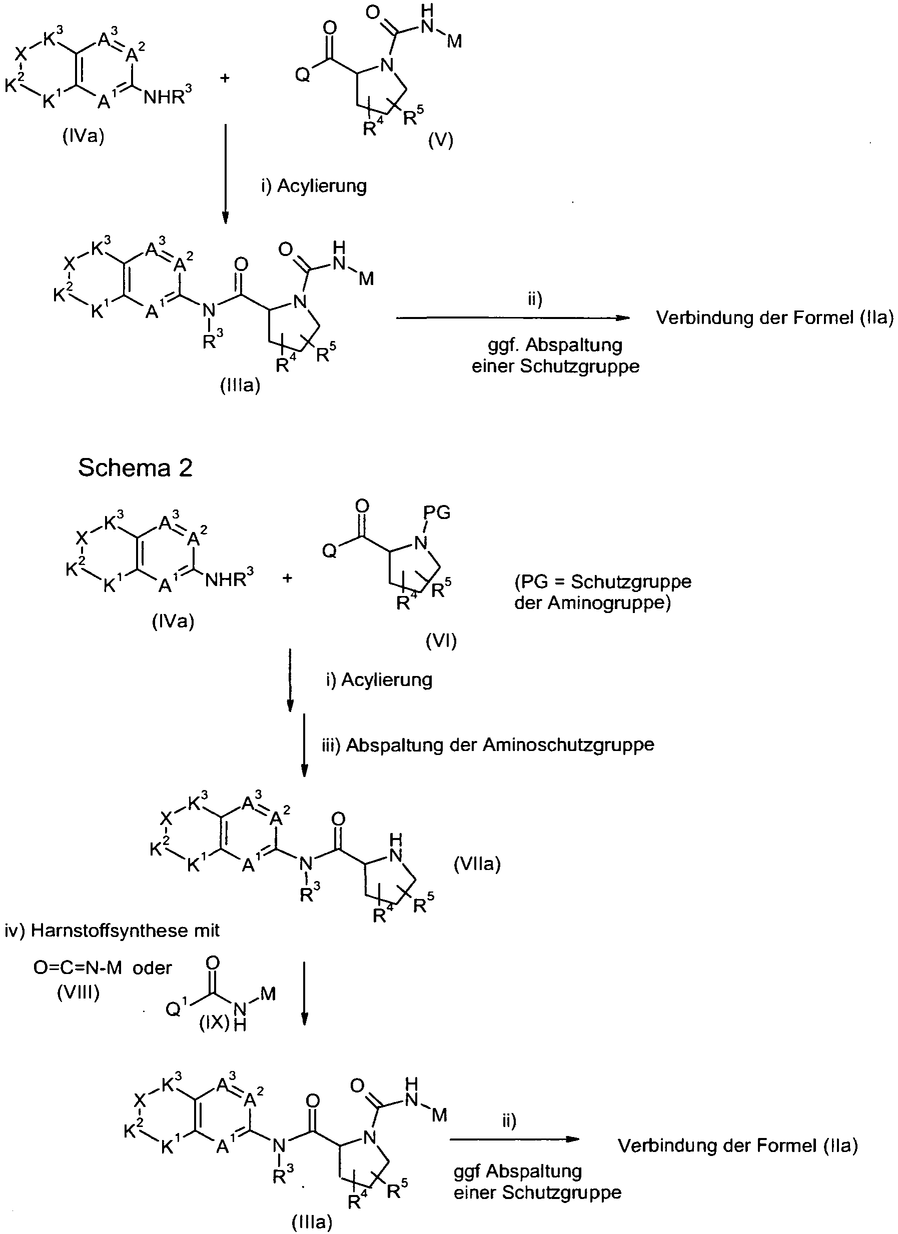 wobei
   Q/Q¹ eine Austrittsgruppe oder eine *in-situ* in eine Austrittsgruppe überführbare Gruppe wie beispielsweise ein Halogenatom, eine Hydroxy-, C₁₋₄-Alkyloxy-, Alkyloxycarbonyloxy-, 4-Nitrophenyloxy-, eine Trichlormethyl- oder Acyloxy-gruppe darstellt, und PG eine in Literatur bekannte Schutzgruppe der Aminofunktion wie beispielsweise eine tert.-Butoxycarbonyl-, Benzyloxycarbonyl- oder eine Trifluoracetyl-gruppe darstellt.

Die in Schema 1 und 2 beschriebenen Reaktionsstufen i) ―iv) können auf die in den Beispielen beschriebene Weise oder nach in Literatur bekannter Bedingungen beispielsweise wie folgt durchgeführt werden:
i) Acylierung eines Amins (IVa) mit einer gegebenenfalls aktivierten Carbonsäure (V) oder (VI) :
   Die Acylierung wird zweckmäßigerweise mit einem entsprechenden Halogenid oder Anhydrid in einem Lösungsmittel wie Methylenchlorid, Chloroform, Tetrachlorkohlenstoff, Ether, Tetrahydrofuran, Dioxan, Benzol, Toluol, Acetonitril, Dimethylformamid, Dimethylsulfoxid, Natronlauge oder Sulfolan gegebenenfalls in Gegenwart einer anorganischen oder organischen Base bei Temperaturen zwischen -20 und 200°C, vorzugsweise jedoch bei Temperaturen zwischen -10 und 100°C, durchgeführt.
   Die Acylierung kann jedoch auch mit der freien Säure gegebenenfalls in Gegenwart eines die Säure aktivierenden Mittels oder eines wasserentziehenden Mittels, beispielsweise in Gegenwart von Ethyl-1-ethoxy-1,2-dihydrochinolin-1-carboxylat, Chlorameisensäureisobutylester, Thionylchlorid, Trimethylchlorsilan, Chlorwasserstoff, Schwefelsäure, Methansulfonsäure, *p*-Toluolsulfonsäure, Phosphortrichlorid, Phosphorpentoxid. Propanphosphonsäurecycloanhydrid, *N*,*N*'-Dicyclohexylcarbodiimid, *N*,*N*'-Dicyclohexylcarbodiimid/Camphersulfonsäure, *N*,*N*'-Dicyclohexylcarbodiimid/*N*-Hydroxysuccinimid oder 1-Hydroxy-benztriazol, *N*,*N*'-Carbonyldiimidazol, *O*-(Benzotriazol-1-yl)-*N*,*N*,*N*',*N*'-tetramethyluroniumtetrafluorborat/*N*-Methylmorpholin, *O*-(Benzotriazol-1-yl)-*N*,*N*,*N*',*N*'-tetramethyl-uroniumtetrafluorborat/*N-*Ethyldiisopropylamin, *O*-(7-Azabenzotriazol-1-yl)-*N*,*N*,*N*',*N*'-tetramethyluronium-hexafluorphosphat/*N*-Methylmorpholin, *O*-Pentafluorophenyl-*N*,*N*,*N'*,N*'*-tetramethyluronium-hexafluorophosphat/Triethylamin, *N*,*N*'-Thionyldiimidazol oder Triphenylphosphin/Tetrachlorkohlenstoff, gegebenenfalls unter Zusatz einer Hilfsbase wie Natronlauge, Cäsium-, Kalium- oder Natrium-carbonat oder ―hydrogencarbonat oder einer Aminbase wie Pyridin, Triethylamin, N-Methylmorpholin oder Diisopropylethylamin bei Temperaturen zwischen -20 und 200°C, vorzugsweise jedoch bei Temperaturen zwischen -10 und 160°C, durchgeführt werden.
   Die Acylierung kann auch mit einem Carbonsäureester (V) oder (VI) und dem Amin (IVa) durch Aktivierung mit Trimethylaluminium durchgeführt werden.
   Weitere Verfahren zur Amidkupplung sind beispielsweise in P.D. Bailey, I.D. Collier, K.M. Morgan in "Comprehensive Functional Group Interconversions", Vol. 5, Seite 257ff., Pergamon 1995, oder auch im Houben-Weyl Ergänzungsband 22, Thieme Verlag, 2003 und der dort zitierten Literatur beschrieben.
ii) bzw. iii) Abspaltung einer Schutzgruppe
   Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0 und 100°C, vorzugsweise bei Temperaturen zwischen 10 und 50°C.
   Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Tetrahydrofuran, Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.
   Die Abspaltung ener Schutzgruppe kann aber auch nach den in T.W. Greene, P.G.M. Wuts in "Protective Groups in Organic Synthesis", Wiley, 1991 und 1999, beschriebenen Verfahren durchgeführt werden.
iv) Synthese eines Harnstoffs
   Die Umsetzung eines Derivates (VIIa) mit einem Isocyanat (VIII) oder einer gegebenenfalls aktivierten Carbaminsäure IX ― wie zum Beispiel einem 4-Nitrophenylcarbaminsäureester - erfolgt in einem Lösungsmittel wie beispielsweise Wasser, Methylenchlorid, Chloroform, Tetrachlorkohlenstoff, Ether, Tetrahydrofuran, Dioxan, Benzol, Toluol, Acetonitril, Dimethylformamid, Dimethylsulfoxid oder Sulfolan oder einem Gemisch der genannten Lösungsmittel gegebenenfalls unter Zusatz einer Hilfsbase wie Natronlauge, Cäsium-, Kalium- oder Natrium-carbonat oder ―hydrogencarbonat oder einer Aminbase wie Pyridin, Triethylamin, N-Methylmorpholin oder Diisopropylethylamin bei Temperaturen zwischen -20 und 200°C, vorzugsweise jedoch bei Temperaturen zwischen -10 und 160°C, durchgeführt werden.

Die Synthese von Verbindungen der allgemeinen Formel (IIb) können analog der Schemata 1 und 2 ausgehend von dem Baustein (IVb) hergestellt werden.
(b) Die Bausteine der allgemeinen Formel (IVa) und (IVb) 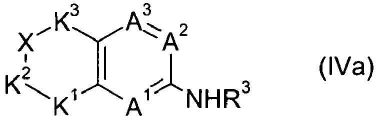 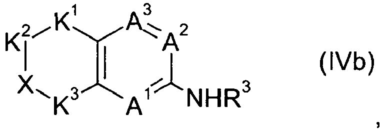 in denen A¹, A², A³, K¹, K², K³, X und R³ wie in Ausführungsform 1 erwähnt definiert sind, und
   die gegebenenfalls an vorhandenen Amino-, Hydroxy-, Carboxy-oder Thiol-gruppen durch gängige Schutzgruppen, wie beispielsweise denen in T.W. Greene, P.G.M. Wuts in "Protective Groups in Organic Synthesis", Wiley, 1991 und 1999, beschriebenen geschützt sein können, und deren Schutzgruppen in Literatur nach bekannter Methode im Verlauf der Synthesesequenz zu Verbindungen der Formel (I) abgespalten werden können,
   sind aus der Literatur bekannt, oder deren Synthese wird in den Ausführungsbeispielen beschrieben, oder sie können beispielsweise nach in Literatur bekannter Syntheseverfahren oder in Analogie zu in Literatur bekannten Syntheseverfahren wie beispielsweise in WO2006/108709; S. Durand-Henchoz et al. Bull. Soc. Chim. France 1966, 11, 3413; J. P. Deer et al. Synth. Commun. 2002, 32, 2555; G. J. Quallich et al., J. Org. Chem. 1998, 63, 4116 oder in J. D. Harling et al. Synth. Commun. 2001, 31, 787 beschrieben, hergestellt werden.

Beispielsweise können Verbindungen der allgemeinen Formel (IVa) und (IVb), in der R³ ein Wasserstoffatom bedeutet und A¹, A², A³, K¹, K², K³ und X wie in Ausführungsform 1 erwähnt definiert sind durch Reduktion der Nitrogruppe von Verbindungen der allgemeinen Formel (Xa) und (Xb) 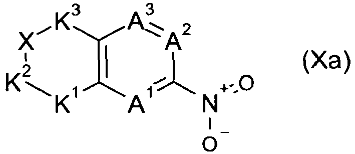 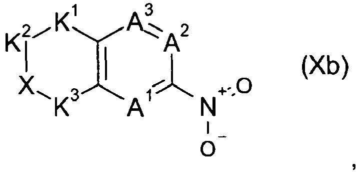 in der A¹, A², A³, K¹, K², K³ und X wie in Ausführungsform 1 erwähnt definiert sind, wie folgt hergestellt werden.

Die Reduktion der Nitrogruppe wird beispielsweise zweckmäßigerweise in einem Lösungsmittel oder Lösungsmittelgemisch wie Wasser, wäßriger Ammoniumchlorid-Lösung, Salzsäure, Schwefelsäure, Phosphorsäure, Ameisensäure, Essigsäure, Acetanhydrid mit Metallen wie Eisen, Zink, Zinn oder Schwefelverbindungen wie Ammoniumsulfid, Natriumsulfid oder Natriumdithionit oder durch katalytische Hydrierung mit Wasserstoff, beispielsweise unter einem Druck zwischen 0.5 und 100 bar, vorzugsweise jedoch zwischen 1 und 50 bar, oder mit Hydrazin als Reduktionsmittel, zweckmäßigerweise in Gegenwart eines Katalysators wie beispielsweise Raney-Nickel, Palladiumkohle, Platinoxid, Platin auf Mineralfaser oder Rhodium, oder mit komplexen Hydriden wie Lithiumaluminiumhydrid, Natriumborhydrid, Natriumcyanborhydrid, Diisobutylaluminiumhydrid, zweckmäßigerweise in einem Lösungsmittel oder Lösungsmittelgemisch wie Wasser, Methanol, Ethanol, Isopropanol, Pentan, Hexan, Cyclohexan, Heptan, Benzol, Toluol, Xylol, Ethylacetat, Methylpropionat, Glykol, Glykoldimethylether, Diethylenglykoldimethylether, Dioxan, Tetrahydrofuran, N-Methylpyrrolidinon, oder aber *N*-Ethyl-diisopropylamin, *N*-C₁₋₅-Alkylmorpholin, *N*-C₁₋₅-Alkylpiperidin, *N*-C₁₋₅-Alkylpyrrolidin, Triethylamin, Pyridin, beispielsweise bei Temperaturen zwischen -30 und 250°C, vorzugsweise jedoch zwischen 0 und 150°C, durchgeführt.
(c) Die Bausteine der allgemeinen Formel 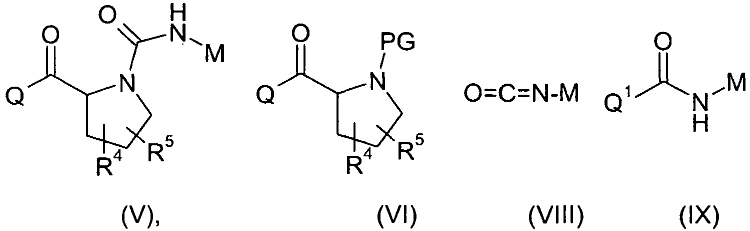 in denen R⁴, R⁵, R⁶ und R² wie in Ausführungsform 1 erwähnt definiert sind, und wobei
   Q/Q¹ beispielsweise eine Hydroxy- oder C₁₋₄-Alkyloxygruppe, ein Halogenatom, eine Aikyloxycarbonyloxy- oder Acyloxygruppe darstellt die gegebenenfalls an vorhandenen Amino-, Hydroxy-, Carboxy-oder Thiolgruppen durch gängige Schutzgruppen wie beispielsweise denen in T.W. Greene, P.G.M. Wuts in "Protective Groups in Organic Synthesis", Wiley, 1991 und 1999, beschriebenen geschützt sein können und deren Schutzgruppen in literaturbekannterweise im Verlauf der Synthesesequenz zu Verbindungen der Formel (I) abgespalten werden können,
   sind in der Literatur bekannt, oder deren Synthese wird in den Ausführungsbeispielen beschrieben, oder sie können beispielsweise nach in der Literatur bekannten Syntheseverfahren oder in Analogie zu in der Literatur bekannten Syntheseverfahren wie beispielsweise in WO2004/87646, WO2003/45912, WO06/32342 oder US2007/0015812 beschrieben, hergestellt werden.

Bei den vorstehend beschriebenen Umsetzungen können gegebenenfalls vorhandene reaktive Gruppen wie Hydroxy-, Carboxy-, Amino-, Alkylamino-oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Methoxy-, Benzyloxy-, Trimethylsilyl-, Acetyl-, Benzoyl-, *tert*.-Butyl-, Trityl-, Benzyl- oder Tetrahydropyranyl-gruppe in Betracht.

Beispielsweise kommt als Schutzrest für eine Carboxylgruppe die Trimethylsilyl-, Methyl-, Ethyl-, *tert*.-Butyl-, Benzyl- oder Tetrahydropyranyl-gruppe in Betracht.

Beispielsweise kommt als Schutzrest für eine Amino-, Alkylamino- oder Iminogruppe die Acetyl-, Trifluoracetyl-, Benzoyl-, Ethoxycarbonyl-, *tert*.-Butoxycarbonyl-, Benzyloxycarbonyl-, Benzyl-, Methoxybenzyl- oder 2,4-Dimethoxybenzyl-gruppe, und für die Aminogruppe zusätzlich die Phthalylgruppe in Betracht.

Beispielsweise kommt als Schutzrest für eine Ethinylgruppe die Trimethylsilyl-, Diphenylmethylsilyl-, tert.Butyldimethylsilyl- oder eine 1-Hydroxy-1-methyl-ethylgruppe in Betracht.

Weitere Schutzgruppen die eingesetzt werden können und deren Abspaltung sind in T.W. Greene, P.G.M. Wuts, "Protective Groups in Organic Synthesis", Wiley, 1991 und 1999 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von Iodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung einer Methoxygruppe erfolgt zweckmäßigerweise in Gegenwart Bortribromid in einem Lösungsmittel wie Methylenchlorid bei Temperaturen zwischen -35 und -25°C.

Die Abspaltung eines 2,4-Dimethoxybenzylrestes erfolgt jedoch vorzugsweise in Trifluoressigsäure in Gegenwart von Anisol.

Die Abspaltung eines *tert*.-Butyl- oder *tert*.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(0) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20 und 70°C.

Ferner können die erhaltenen Verbindungen der allgemeinen Formel (I) in ihre Enantiomeren und/oder Diastereomeren aufgetrennt werden.

So lassen sich beispielsweise die erhaltenen Verbindungen der allgemeinen Formel (I), welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel (I) mit mindestes zwei asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch chromatographische Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel (I) in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel (I), falls diese eine Carboxygruppe enthalten, gegebenenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die Verbindungen der allgemeinen Formel (I) sowie deren Tautomere, deren Enantiomere, deren Diastereomere und deren physiologisch verträglichen Salze wertvolle pharmakologische Eigenschaften auf, insbesondere eine antithrombotische Wirkung, welche vorzugsweise auf einer Thrombin oder Faktor Xa beeinflussenden Wirkung beruht, beispielsweise auf einer thrombinhemmenden oder Faktor Xahemmenden Wirkung, auf einer die aPTT-Zeit verlängernden Wirkung und auf einer Hemmwirkung auf verwandte Serinproteasen wie z. B. Urokinase, Faktor Vlla, Faktor IX, Faktor XI und Faktor XII.

Die im Experimentellen Teil angeführten Verbindungen können auf ihre Wirkung auf die Hemmung des Faktors Xa wie folgt untersucht werden.

### Methodik

Enzymkinetische Messung mit chromogenem Substrat. Die durch humanen Faktor Xa aus dem farblosen chromogenen Substrat freigesetzte Menge an p-Nitroanilin (pNA) wird photometrisch bei 405 nm bestimmt. Sie ist proportional der Aktivität des eingesetzten Enzyms. Die Hemmung der Enzymaktivität durch die Testsubstanz (bezogen auf die Lösungsmittelkontrolle) wird bei verschiedenen Testsubstanz-Konzentrationen ermittelt und hieraus die IC₅₀ berechnet als diejenige Konzentration, die den eingesetzten Faktor Xa um 50 % hemmt.

### Material

Tris(hydroxymethyl)-aminomethan-Puffer (100 mMol) und Natriumchlorid (150 mMol), pH 8.0 plus 1 mg/ml Human Aibumin Fraction V, Proteasefrei.

Faktor Xa (Calbiochem), Spez. Aktivität: 217 IU/mg, Endkonzentration: 7 IU/ml pro Reaktionsansatz.

Substrat S 2765 (Chromogenix), Endkonzentration: 0.3 mM/l (1 KM) pro Reaktionsansatz.

Testsubstanz: Endkonzentration 100, 30, 10, 3, 1, 0.3, 0.1, 0.03, 0.01, 0.003, 0.001 µmol/l.

### Durchführung

10 µl einer 23.5-fach konzentrierteren Ausgangslösung der Testsubstanz bzw. Lösungsmittel (Kontrolle), 175 µl TRIS/HSA-Puffer und 25 µl Faktor Xa-Gebrauchslösung von 65.8 U/L werden 10 Minuten bei 37°C inkubiert. Nach Zugabe von 25 µl S 2765-Gebrauchslösung (2.82 mMol/L) wird die Probe im Photometer (SpectraMax 250) bei 405 nm für 600 Sekunden bei 37°C gemessen.

### Auswertung

1. Ermittlung der maximalen Zunahme (deltaOD/Minuten) über 21 Messpunkte.
2. Ermittlung der %-Hemmung bezogen auf die Lösungsmittelkontrolle.
3. Erstellen einer Dosiswirkungskurve (%-Hemmung vs Substanzkonzentration).
4. Ermittlung der IC₅₀ durch Interpolation des X-Wertes (Substanzkonzentration) der Dosiswirkungskurve bei Y = 50 % Hemmung.

Alle getesteten Verbindungen zeigen IC₅₀-Werte, die kleiner als 100 µmol/L sind.

Die erfindungsgemäß hergestellten Verbindungen sind im allgemeinen gut verträglich.

Aufgrund ihrer pharmakologischen Eigenschaften eignen sich die neuen Verbindungen und deren physiologisch verträgliche Salze zur Vorbeugung und Behandlung venöser und arterieller thrombotischer Erkrankungen, wie zum Beispiel der Vorbeugung und Behandlung von tiefen Beinvenen-Thrombosen, Thrombophlebitis, der Verhinderung von Reokklusionen nach Bypass-Operationen oder Angioplastie (PT(C)A), sowie der Okklusion bei peripheren arteriellen Erkrankungen, sowie Vorbeugung und Behandlung von Lungenembolie, der disseminierten intravaskulären Gerinnung und der schweren Sepsis, der Verhinderung und Prophylaxe der DVT in Patienten mit Exacerbation der COPD, der Behandlung der ulzerativen Colitis, der Prophylaxe und Behandlung der Koronarthrombose, der Prophylaxe des Schlaganfalls und der Verhinderung der Okklusion von Shunts.

Zusätzlich sind die erfindungsgemäßen Verbindungen zur antithrombotischen Unterstützung bei einer thrombolytischen Behandlung, wie zum Beispiel mit Alteplase, Reteplase, Tenecteplase, Staphylokinase oder Streptokinase, zur Verhinderung der Langzeitrestenose nach PT(C)A, zur Prophylaxe und Behandlung von ischämischen Vorfällen in Patienten mit allen Formen der koronaren Herzerkrankung, zur Verhinderung der Metastasierung und des Wachstums von Tumoren und von Entzündungsprozessen, z.B. bei der Behandlung der pulmonalen Fibrose, zur Prophylaxe und Behandlung der rheumatoiden Arthritis, zur Verhütung oder Verhinderung von fibrin-abhängigen Gewebsadhäsionen und/oder Narbengewebebildung sowie zur Förderung von Wundheilungsprozessen geeignet.

Die genannten Verbindungen können auch als Antikoagulantien in Zusammenhang mit der Herstellung, Lagerung, Fraktionierung oder Verwendung von Vollblut oder bei invasiven Therapieverfahren, zum Beispiel zum Beschichten von Prothesen, künstlichen Herzklappen und Kathetern zur Reduktion des Thromboserisikos eingesetzt werden.

Aufgrund ihrer pharmakologischen Eigenschaften eignen sich außerdem die neuen Verbindungen und deren physiologisch verträgliche Salze zur Behandlung der Alzheimer- und Parkinson'schen Krankheit. Eine Rationale dafür ergibt sich zum Beispiel aus folgende Befunden, aus denen man schließen kann, dass Thrombinhemmer bzw. Faktor Xa Hemmer, durch Hemmung der Thrombinbildung bzw. -aktivität, wertvolle Medikamente in der Behandlung der Alzheimer- und Parkinson'schen Krankheit darstellen könnten. Klinische und experimentelle Studien legen nahe, dass neurotoxische Mechanismen, beispielsweise die mit der Aktivierung von Proteasen der Gerinnungskaskade einhergehende Entzündung, beteiligt ist am Absterben von Neuronen infolge von Hirntraumata. Verschiedene Studien deuten auf eine Beteiligung von Thrombin bei neurodegenerativen Prozessen hin, beispielsweise infolge eines Schlaganfalls, wiederholter Bypassoperation oder traumatischen Hirnverletzungen. Eine erhöhte Thrombinaktivität konnte beispielsweise noch Tage nach peripherer Nervenverletzung nachgewiesen werden. Es konnte weiterhin gezeigt werden, dass Thrombin eine Neuritenretraktion, sowie Glia-Proliferation, und Apoptose in Primärkulturen von Neuronen und Neuroblastomzellen hervorruft (zur Übersicht siehe: Neurobiol. Aging, 2004, 25(6), 783-793). Darüberhinaus deuten verschiedene in vitro Studien an Gehirnen von Patienten mit Alzheimer-Krankheit darauf hin, dass Thrombin in der Pathogenese dieser Krankheit eine Rolle spielt (Neurosci. Lett., 1992, 146, 152-54). Eine Anreicherung immunreaktiven Thrombins konnte in Neuriten-Plaques in Gehirnen von Alzheimer-Patienten nachgewiesen werden. In vitro wurde gezeigt, dass Thrombin ebenfalls eine Rolle bei der Regulation und Stimulation der Produktion des "Amyloid Precursor Proteins" (APP) spielt sowie bei der Spaltung des APP in Fragmente, welche in den Amyloid-Plaques im Gehirn von Alzheimer-Patienten nachgewiesen werden können. Weiterhin konnte gezeigt werden, dass die thrombin-induzierte mikrogliale Aktivierung in vivo zur Degeneration von nigralen dopaminergen Neuronen führt. Diese Befunde lassen den Schluss zu, dass mikrogliale Aktivierung -ausgelöst durch endogene Substanz(en) wie beispielsweise Thrombin- beteiligt sind am neuropathologischen Prozess des Zelltodes dopaminerger Neurone, wie er bei Patienten mit Parkinson'scher Krankheit vorkommt (J. Neurosci., 2003, 23, 5877-86).

Die neuen Verbindungen und ihre physiologisch verträglichen Salze sind auch für die Prophylaxe und Behandlug von arteriellen vaskulären Erkrankungen in der Kombinationstherapie mit lipidsenkenden Wirkstoffen wie HMG-CoA Reduktase Inhibitoren und Vasodilatatoren, insbesondere ACE-Inhibitoren, Angiotensin II-Antagonisten, Renin-Inhibitoren, ß-Rezeptor-Antagonisten, α-Rezeptor-Antagonisten, Diuretika, Ca-Kanalblockern, oder Stimulatoren der löslichen Guanylatzyklase einsetzbar.

Durch Erhöhung der antithrombotischen Wirkung sind die neuen Verbindungen und ihre physiologisch verträglichen Salze auch in der Kombinationstherapie mit anderen Antikoagulantien wie beispielsweise unfraktioniertem Heparin, niedermolekularem Heparin, Fondaparinux oder direkten Thrombinhemmern, zum Beispiel rekombinantem Hirudin oder "active-site"-Thrombinhemmern, einsetzbar

Die neuen Verbindungen und deren physiologisch verträgliche Salze können therapeutisch in Kombination mit Azetylsalizylsäure, mit Inhibitoren der Plättchen-Aggregation wie Fibrinogen-Rezeptorantagonisten (z.B. Abciximab, Eptifibatide, Tirofiban, Roxifiban), mit physiologischen Aktivatoren und Inhibitoren des Gerinnungssystems und deren rekombinanter Analoga (z.B. Protein C, TFPI, Antithrombin), mit Inhibitoren der ADP-induzierten Aggregation (z.B. Clopidogrel, Prasugrel, Ticlopidin), mit P₂T-Rezeptorantagonisten (z.B. Cangrelor) oder mit kombinierten Thromboxan Rezeptorantagonisten/Synthetaseinhibitoren (z.B. Terbogrel) eingesetzt werden.

Die zur Erzielung einer entsprechenden Wirkung erforderliche Dosierung beträgt zweckmäßigerweise bei intravenöser Gabe 0.01 bis 3 mg/kg, vorzugsweise 0.03 bis 1.0 mg/kg, und bei oraler Gabe 0.03 bis 30 mg/kg, vorzugsweise 0.1 bis 10 mg/kg, jeweils 1 bis 4 x täglich.

Hierzu lassen sich die erfindungsgemäß hergestellten Verbindungen der Formel (I), gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen oder Zäpfchen einarbeiten.

Die neuen Verbindungen und deren physiologisch verträgliche Salze können therapeutisch in Kombination mit Acetylsalicylsäure, mit Inhibitoren der Plättchen-Aggregation wie Fibrinogen-Rezeptorantagonisten (z.B. Abciximab, Eptifibatide, Tirofiban, Roxifiban), mit physiologischen Aktivatoren und Inhibitoren des Gerinnungssystems und deren rekombinanter Analoga (z.B. Protein C, TFPI, Antithrombin), mit Inhibitoren der ADP-induzierten Aggregation (z.B. Clopidogrel, Ticlopidin), mit P₂T-Rezeptorantagonisten (z.B. Cangrelor) oder mit kombinierten Thromboxan Rezeptorantagonisten/Synthetaseinhibitoren (z.B. Terbogrel) eingesetzt werden.

### Experimenteller Teil

Die nachfolgenden Beispiele sollen die Erfindung näher erläutern, ohne diese jedoch in ihrem Umfang zu beschränken.

Für die hergestellten Verbindungen liegen in der Regel Schmelzpunkte und/oder IR-, UV-, ¹H-NMR und/oder Massenspektren vor. Wenn nicht anders angegeben, wurden R_{f}-Werte unter Verwendung von DC-Fertigplatten Kieselgel 60 F₂₅₄ (E. Merck, Darmstadt, Artikel-Nr. 1.05714) ohne Kammersättigung bestimmt. Die unter der Bezeichnung Alox ermittelten R_{f}-Werte wurden unter Verwendung von DC-Fertigplatten Aluminiumoxid 60 F₂₅₄ (E. Merck, Darmstadt, Artikel-Nr. 1.05713) ohne Kammersättigung bestimmt. Die unter der Bezeichnung Reversed-Phase-8 ermittelten R_{f}-Werte wurden unter Verwendung von DC-Fertigplatten RP-8 F₂₅₄ₛ (E. Merck, Darmstadt, Artikel-Nr. 1.15684) ohne Kammersättigung bestimmt. Die bei den Fliessmitteln angegebenen Verhältnisse beziehen sich auf Volumeneinheiten der jeweiligen Lösungsmittel. Zu chromatographischen Reinigungen wurde Kieselgel der Firma Millipore (MATREX*™*, 35-70 µm) verwendet. Falls nähere Angaben zur Konfiguration fehlen, bleibt offen, ob es sich um reine Stereoisomere oder um Enantiomeren-/Diastereomerengemische handelt.

Die HPLC-MS Daten wurden unter den folgenden Bedingungen erzeugt:

### Methode A:

Waters Alliance 2690, Waters ZQ2000 Massen Spektrometer mit Diodenarraydetektor 996.
Als mobile Phase wurde eingesetzt:
A: Wasser mit 0.10% TFA
B: Acetonitril mit 0.8% TFA

| Zeit in min | %A | %B | Flussrate in ml/min |
|---|---|---|---|
| 0.00 | 95 | 5 | 1.00 |
| 0.10 | 95 | 5 | 1.00 |
| 3.10 | 2 | 98 | 1.00 |

Als stationäre Phase diente eine Säule X-Terra MS C18, 2.5 µm, 4.6 mm x 30 mm.

Die Diodenarraydetektion erfolgte im Wellenlängenbereich 210-500 nm.

### Methode B:

Waters Alliance 2695, PDA Detektor 2996.
Als mobile Phase wurde eingesetzt:
A: Wasser mit 0.13% TFA
B: Acetonitril

| Zeit in min | %A | %B | Flussrate in ml/min |
|---|---|---|---|
| 0.00 | 95 | 5 | 3.50 |
| 0.18 | 95 | 5 | 3.50 |
| 2.00 | 2 | 98 | 3.50 |
| 2.20 | 2 | 98 | 3.50 |

Als stationäre Phase diente eine Säule Varian Microsorb 100 C18, 3 µm, 4.6 mm x 30 mm.
Die Diodenarraydetektion erfolgte im Wellenlängenbereich 210-380 nm.

### Methode C:

Waters Alliance 2695, PDA Detektor 2996.
Als mobile Phase wurde eingesetzt:
A: Wasser mit 0.1 % HCOOH
B: Acetonitril mit 0.1% HCOOH

| Zeit in min | %A | %B | Flussrate in ml/min |
|---|---|---|---|
| 0.00 | 95 | 5 | 1.60 |
| 4.50 | 10 | 90 | 1.60 |

Als stationäre Phase diente eine Säule YMC-Pack ODS-AQ, 3 µm, 4.6 mm x 75 mm.

### Methode D:

Waters Alliance 2695, PDA Detektor 2996.
Als mobile Phase wurde eingesetzt:
A: Wasser mit 0.1 % HCOOH
B: Acetonitril mit 0.1% HCOOH

| Zeit in min | %A | %B | Flussrate in ml/min |
|---|---|---|---|
| 0.00 | 95 | 5 | 1.60 |
| 4.50 | 10 | 90 | 1.60 |

Als stationäre Phase diente eine Säule Zorbax StableBond C18, 3 µm, 4.6 mm x 75 mm.

In den Versuchsbeschreibungen werden die folgenden Abkürzungen verwendet.
- DCM: Dichlormethan
- DIPEA: *N*-Ethyl-diisopropylamin
- DMF: *N*,*N*-Dimethylformamid
- EtOH: Ethanol
- ges.: gesättigt
- h: Stunde(n)
- HATU: *O*-(7-Azabenzotriazol-1-yl)-*N*,*N*,*N'*,*N'*-tetramethyluroniumhexafluorphosphat
- i. Vak.: im Vakuum
- konz.: konzentriert
- min: Minute(n)
- NMM: N-Methyl-morpholin
- R_{f}: Retentionsfaktor
- Rₜ: Retentionszeit
- TBTU: *O*-(Benzotriazol-1-yl)-*N*,*N*,*N*',*N*'-tetramethyluroniumtetrafluorborat
- TEA: Triethylamin
- TFA: Trifluoressigsäure
- THF: Tetrahydrofuran

### Beispiel 1

(2R,4R)-4-Methoxy-pyrrolidin-1,2-dicarbonsäure-1-[(4-chlor-phenyl)-amid]-2-(2 methyl-1,2,3,4-tetrahydro-isochinolin-7-yl)-amid 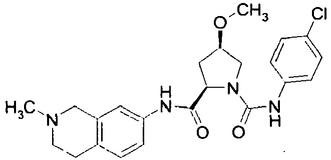
(a) (2R,4R)-4-Methoxy-pyrrolidin-2-carbonsäure (als Hydrochlorid)
   10.3 g (48.9 mmol) (2R,4R)-4-Methoxy-pyrrolidin-1,2-dicarbonsäure-1-tert.butoxy-ester werden in 50 ml THF gelöst, mit 50 ml 6N Salzsäure (300 mmol) versetzt und drei Stunden gerührt. Das Reaktionsgemisch wird i.Vak. zur Trockene eingeengt.
   Ausbeute: 7.89 g (quantitativ)
   C₆H₁₁NO₃ (145.16) x HCl
   Massenspektrum: (M+H)⁺ = 146
(b) (2R,4R)-1-(4-Chlor-phenylcarbamoyl)-4-methoxy-pyrrolidin-2-carbonsäure
   Zu einer Mischung aus 7.89 g (43.9 mmol) (2R,4R)-4-Methoxy-pyrrolidin-2-carbonsäure-hydrochlorid in 288 ml 5%iger wässriger Natriumhydrogencarbonat-Lösung werden 13.5 g (87.8 mmol) 4-Chlor-phenylisocyanat gegeben und für 3h bei 80°C gerührt. Man gibt weitere 0.6 g des Isocyanats zu und rührt eine weitere Stunde. Anschließend wird das Reaktionsgemisch abgekühlt und von Feststoff abfiltriert. Der Feststoff wird mit Wasser gewaschen. Die wässrigen Phasen werden vereint und mit 6N wässriger Salzsäure angesäuert. Anschließend extrahiert man dreimal mit Dichlormethan. Die vereinten organischen Phasen werden über Natriumsulfat getrocknet, filtriert und i. Vak. zur Trockene eingeengt.
   Ausbeute: 10.0 g (76%)
   R_{f}-Wert: 0.47 (RP-8; Methanol / 5% Natriumchlorid-Lösung 6:4) C₁₃H₁₅ClN₂O₄ (298.72)
   Massenspektrum: (M+H)⁺ = 299/301 (Chlorisotope)
(c) (2R,4R)-4-Methoxy-pyrrolidin-1,2-dicarbonsäure-1-[(4-chlor-phenyl)-amid]-2-(2-methyl-1,2,3,4-tetrahydro-isochinolin-7-yl)-amid
   Zu einer Lösung aus 299 mg (1 mmol) (2R,4R)-1-(4-Chlor-phenylcarbamoyl)-4-methoxy-pyrrolidin-2-carbonsäure in 2 ml THF werden 248 mg (0.266 mmol) 2-Ethoxy-1-ethoxycarbonyl-1,2-dihydrochinolin zugegeben und 30 min. gerührt. Dann werden 162 mg (1 mmol) 2-Methyl-1,2,3,4-tetrahydroisochinolin-7-ylamin zugegeben und unter Rückfluß für 18 Stunden gerührt.
   Das Reaktionsgemisch wird i.Vak. konzentriert und mittels Chromatographie an Silicagel (Fließmittel: DCM / (Ethanol/Ammoniak 95:5) 96:4 ― 94:6) gereinigt.
   Ausbeute: 15 mg (3%)
   R_{f}-Wert: 0.41 (Kieselgel; Dichlormethan/Ethanol/Ammoniak = 90:10:1) C₂₃H₂₇ClN₄O₃ (442.94)
   Massenspektrum: (M+H)⁺ = 443/445 (Chlorisotope)

Analog können die folgenden Verbindungen hergestellt werden:

| **Bsp.** | **Strukturformel** | **Ausbeute** | **Massenpeak(s)** | **DC/HPLC** |
|---|---|---|---|---|
| **2** | 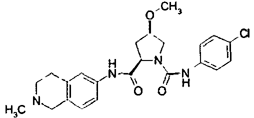 | 25% | (M-H)⁻ = 441/443 (Chlorisotope) | R_{f}-Wert: 0.37 (Kieselgel; DCM/EtOH/NH₃ = 90:10:1) |
| | (2R,4R)-4-Methoxy-pyrrolidin-1,2-dicarbonsäure-1-[(4-chlor-phenyl)-amid]-2-(2-methyl-1,2,3,4-tetrahydro-isochinolin-6-yl)-amid | | | |
| **3** | 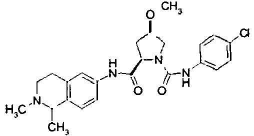 | 57% | (M+H)⁺ = 457/459 (Chlorisotope) | Rₜ = 2.80 min (Methode A) |
| | (2R,4R)-4-Methoxy-pyrrolidin-1,2-dicarbonsäure-1-[(4-chlor-phenyl)-amid]-2-(1,2-dirnethyl-1,2,3,4-tetrahydro-isochinolin-6-yl)-amid | | | |
| **8** | 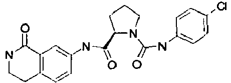 | 41% | (M+H)⁺ = 413/415 (Chlorisotope) | Rₜ = 1.18 min (Methode B) |
| | (R)-Pyrrolidin-1,2-dicarbonsäure-1-[(4-chlor-phenyl)-amid]-2-(1-oxo-1,2,3,4-tetrahydro-isochinolin-7-yl)-amid | | | |

### Beispiel 4

(2S,4R)-4-Methoxy-2-methyl-pyrrolidin-1,2-dicarbonsäure-1-[(4-chlor-phenyl)-amid]-2-[(2-methyl-1,2,3,4-tetrahydro-isochinolin-6-yl)-amid (als Trifluoracetat-Salz) 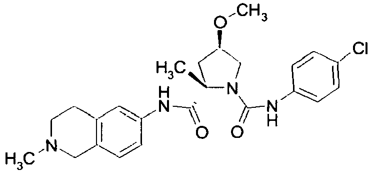
(a) (2S/R,4R)-4-Methoxy-2-methyl-pyrrolidin-1,2-dicarbonsäure-1-tert.-butylester-2-methyl-ester
   Zu einer Lösung von 4.8 ml (34.2 mmol) Diisopropylamin in 200 ml THF werden bei ~5°C 21.4 ml (1.6 M in n-Hexan, 34.2 mmol) n-Butyllithium-Lösung zugetropft und 10 min gerührt. Dann wird die Mischung auf -35°C abgekühlt und mit einer Lösung von 5.8 g (22.4 mmol) (2S,4R)-4-Methoxy-2-methyl-pyrrolidin-1,2-dicarbonsäure-1-tert.-butyl-ester-2-methyl-ester in 200 ml THF versetzt. Die Mischung wird innerhalb einer Stunde auf 0°C erwärmt und dann auf -78°C abgekühlt. Es werden 2.1 ml (33.7 mmol) Methyliodid zugetropft und für 4 Stunden bei -78°C gerührt. Dann werden 3 ml ges. Ammoniumchlorid-Lösung zugetropft und auf RT erwärmt. Anschließend wird mit Wasser versetzt und dreimal mit Ethylacetat extrahiert. Die vereinigten organischen Phasen werden über Natriumsulfat getrocknet, filtriert und i. Vak. eingeengt. Der Rückstand wird säulenchromatographisch an Silicagel (DCM / MeOH 4:1) gereinigt.
   Ausbeute: 4.2 g (69%)
   R_{f}-Wert: 0.38 (Kieselgel; Dichlormethan/Methanol = 80:20)
   C₁₃H₂₃NO₅ (273.33)
   Massenspektrum: (M+H)⁺ = 274
(b) (2S/R,4R)-4-Methoxy-2-methyl-pyrrolidin-2-carbonsäure-methylester (Trifluoracetat-Salze)
   800 mg (293 µmol) (2S/R,4R)-4-Methoxy-2-methyl-pyrrolidin-1,2-dicarbonsäure-1-tert.-butyl-ester-2-methyl-ester werden in 2.5 ml DCM gelöst, mit 2.5 ml TFA versetzt und 16 Stunden bei RT gerührt. Das Reaktionsgemisch wird i. Vak. zur Trockene eingeengt.
   Ausbeute: quantitativ
   Rₜ-Wert: 0.42 min (Methode B)
   C₈H₁₅NO₃ (173.21)
   Massenspektrum: (M+H)⁺ = 174
(c) (2S,4R)-4-Methoxy-2-methyl-pyrrolidin-1,2-dicarbonsäure-1-benzylester-2-methylester
   419 mg (146 µmol) (2S/R,4R)-4-Methoxy-2-methyl-pyrrolidin-2-carbonsäuremethylester (als Trifluoracetat-Salze) werden in 4.5 ml DCM gelöst, bei 0°C mit 0.5 ml (292 µmol) DIPEA versetzt und anschließend mit 0.3 ml (175 µmol) Chlorameisensäurebenzylester versetzt. Es wird 10 min bei 0°C und dann 16 Stunden bei RT gerührt. Anschließend wird das Reaktionsgemisch i. Vak. eingeengt und mittel RP-HPLC gereinigt.
   Ausbeute: 114 mg (50%)
   Rₜ-Wert: 1.37 min (Methode B)
   C₁₆H₂₁NO₅ (307.34)
   Massenspektrum: (M+H)⁺ = 308 Zusätzlich wird erhalten:
   (2R,4R)-4-Methoxy-2-methyl-pyrrolidin-1,2-dicarbonsäure-1-benzylester-2-methylester
   Ausbeute: 114 mg (50%)
   Rₜ-Wert: 1.41 min (Methode B)
   C₁₆H₂₁NO₅ (307.34)
   Massenspektrum: (M+H)⁺ = 308
(d) (2S,4R)-4-Methoxy-2-methyl-pyrrolidin-1,2-dicarbonsäure-1-benzylester
   114 mg (370 µmol) (2S,4R)-4-Methoxy-2-methyl-pyrrolidin-1,2-dicarbonsäure-1-benzylester-2-methylester werden in 1 ml Methanol gelöst und mit 2.4 ml (605 µmol) 8%iger wässriger Lithiumhydroxid-Lösung versetzt. Das Reaktionsgemisch wird drei Tage bei RT gerührt und dann i.Vak. eingeengt. Der Rückstand wird mit 1 N HCl angesäuert und dreimal mit Ethylacetat ausgeschüttelt. Die vereinigten organischen Phasen werden über Natriumsulfat getrocknet, filtriert und i. Vak. eingeengt.
   Ausbeute: 100 mg (92%)
   Rₜ-Wert: 1.25 min (Methode B)
   C₁₅H₁₉NO₅ (437.54)
   Massenspektrum: (M+H)⁺ = 294
(e) (2S,4R)-4-Methoxy-2-methyl-2-(2-methyl-1,2,3,4-tetrahydro-isochinolin-6-ylcarbamoyl)-pyrrolidin-1-carbonsäure-benzylester (als Trifluoracetat-Salz)
   56 mg (345 µmol) (2S,4R)-4-Methoxy-2-methyl-pyrrolidin-1,2-dicarbonsäure-1-benzylester werden in 0.5 ml DMF gelöst und mit 134 mg (352 µmol) HATU und mit 160 µl NMM versetzt. Die Mischung wird 15 min bei RT gerührt und dann mit 100 mg (341 µmol) 2-Methyl-1,2,3,4-tetrahydroisochinolin-6-ylamin versetzt. Die Reaktionsmischung wird drei Stunden bei RT gerührt und dann mit TFA angesäuert. Das Produkt wird aus dieser Mischung mittels RP-HPLC isoliert.
   Ausbeute: 104 mg (54%)
   Rₜ-Wert: 1.16 min (Methode B)
   C₂₅H₃₁N₃O₄ (437.54) x CF₃CO₂H
   Massenspektrum: (M+H)⁺ = 438
(f) (2S,4R)-4-Methoxy-2-methyl-pyrrolidin-2-carbonsäure-(2-methyl-1,2,3,4-tetrahydro-isochinolin-6-yl)-amid (als Trifluoracetat-Salz)
   104 mg (345 µmol) (2S,4R)-4-Methoxy-2-methyl-2-(2-methyl-1,2,3,4-tetrahydroisochinolin-6-ylcarbamoyl)-pyrrolidin-1-carbonsäure-benzylester (als Trifluoracetat-Salz) werden in einem Gemisch aus 6 ml THF und 6 ml Methanol gelöst, mit 30 mg Palladium/Kohle (10%ig) versetzt und 2,5 Stunden mit 3 bar Wasserstoff hydriert. Anschließend wird die Mischung filtriert und i. Vak. eingeengt.
   Ausbeute: 75 mg (96%)
   Rₜ-Wert: 0.32 min (Methode B)
   C₁₇H₂₅N₃O₂ (303.41) x CF₃CO₂H
   Massenspektrum: (M+H)⁺ = 304
(g) (2S,4R)-4-Methoxy-2-methyl-pyrrolidin-1,2-dicarbonsäure-1-[(4-chlorphenyl)-amid]-2-[(2-methyl-1,2,3,4-tetrahydro-isochinolin-6-yl)-amid (als Trifluoracetat-Salz)
   75 mg (180 µmol) (2S,4R)-4-Methoxy-2-methyl-pyrrolidin-2-carbonsäure-(2-methyl-1,2,3,4-tetrahydro-isochinolin-6-yl)-amid (als Trifluoracetat-Salz) werden in 1.5 ml DMF gelöst und mit 122 µl ( 796 µmol) NMM versetzt. Dann werden 30 mg (195 µmol) 4-Chlor-phenylisocyanat zugegeben, und es wird drei Tage bei RT gerührt. Anschließend wird mit TFA sauer gestellt. Das Produkt wird aus dieser Mischung mittels RP-HPLC isoliert.
   Ausbeute: 62 mg (60%)
   Rₜ-Wert: 1.17 min (Methode B)
   C₂₄H₂₉ClN₄O₃ (456.98) x CF₃CO₂H
   Massenspektrum: (M+H)⁺ = 457/459 (Chlorisotope)

Analog kann die folgende Verbindung hergestellt werden:

| **Bsp.** | **Strukturformel** | **Ausbeute** | **Massenpeak(s)** | **DC/HPLC** |
|---|---|---|---|---|
| **5** | 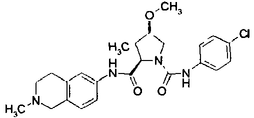 | 11.4 % | (M+H)⁺ = 457/459 (Chlorisotope) | Rt= 1.15 min (Methode B) |
| | (2R,4R)-4-Methoxy-2-methyl-pyrrolidin-1,2-dicarbonsäure-1-[(4-chlor-phenyl)-amid]-2-[(2-methyl-1,2,3,4-tetrahydro-isochinolin-6-yl)-amid (als Trifluoracetat-Salz) | | | |

### Beispiel 6

(2R,4R)-4-Methoxy-pyrrolidin-1,2-dicarbonsäure-1-[(5-chlor-pyridin-2-yl)-amid]-2-(2-methyl-1,2,3,4-tetrahydro-isochinolin-6-yl)-amid (als Trifluoracetat-Salz) 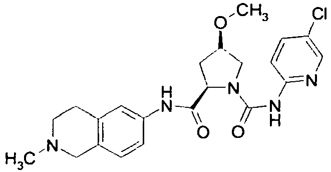
(a) (2R,4R)-4-Methoxy-2-(2-methyl-1,2,3,4-tetrahydro-isochinolin-6-ylcarbamoyl)-pyrrolidin-1-carbonsäure-benzylester (als Trifluoracetat-Salz)
   100 mg (616 µmol) 2-Methyl-1,2,3,4-tetrahydro-isochinolin-6-ylamin werden in 1.5 ml DCM gelöst und bei RT mit 0.61 ml (1.22 mmol) Trimethylaluminium-Lösung (2M in Toluol) versetzt und 15 min gerührt. Dieses Reaktionsgemisch wird zu 180 mg (614 µmol) (2R,4R)-4-Methoxy-pyrrolidin-1,2-dicarbonsäure-1-benzylester-2-methylester gegeben und mit 0.5 ml DCM nachgespült. Das Reaktionsgemisch wird drei Stunden bei RT gerührt und dann auf 2N Natronlauge gegeben. Mit extrahiert die wässrige Phase dreimal mit Ethylacetat. Die vereinigten organischen Phasen werden über Natriumsulfat getrocknet, filtriert und i.Vak. zur Trockene eingeengt. Der Rückstand wird mittels RP-HPLC gereinigt.
   Ausbeute: 169 mg (51%)
   Rₜ-Wert: 1.05 min (Methode B)
   C₂₄H₂₉N₃O₄ (423.52) x CF₃CO₂H
   Massenspektrum: (M+H)⁺ = 424
(b) (2R,4R)-4-Methoxy-pyrrolidin-2-carbonsäure-(2-methyl-1,2,3,4-tetrahydro-isochinolin-6-yl)-amid (als Trifluoracetat-Salz)
   168 mg (313 µmol) (2R,4R)-4-Methoxy-2-(2-methyl-1,2,3,4-tetrahydroisochinolin-6-ylcarbamoyl)-pyrrolidin-1-carbonsäure-benzylester (als Trifluoracetat-Salz) werden analog Beispiel 4 f hydriert.
   Ausbeute: 120 mg (95%)
   Rt-Wert: 0.31 min (Methode B)
   C₁₆H₂₃N₃O₂ (289.38) x CF₃CO₂H
   Massenspektrum: (M+H)⁺ = 290
(c) (2R,4R)-4-Methoxy-pyrrolidin-1,2-dicarbonsäure-1-[(5-chlor-pyridin-2-yl)-amid]-2-(2-methyl-1,2,3,4-tetrahydro-isochinolin-6-yl)-amid (als Trifluoracetat-Salz)
   Zu einer Lösung aus 100 mg (778 µmol) 2-Amino-5-chlor-pyridin in 2 ml DCM und 70 µl (867 µmol) Pyridin werden 170 mg (843 µmol) Chlorameisensäure-4-nitrophenylester zugegeben und 3,5 Stunden gerührt. Anschließend wird die Reaktionsmischung zur Trockene eingeengt und als Rohprodukt zu einer Lösung von 120 mg (297 µmol) (2R,4R)-4-Methoxy-pyrrolidin-2-carbonsäure-(2-methyl-1,2,3,4-tetrahydro-isochinolin-6-yl)-amid (als Trifluoracetat-Salz) und 125 µl (900 µmol) TEA in 2.5 ml DMF gegeben. Die Reaktionsmischung wird drei Tage bei RT gerührt und dann mit ges. Natriumhydrogencarbonat-Lösung versetzt. Die wässrige Phase wird dreimal mit Ethylacetat extrahiert.
   Die vereinigten organischen Phasen werden über Natriumsulfat getrocknet, filtriert und i.Vak. zur Trockene eingeengt. Der Rückstand wird mittels RP-HPLC gereinigt.
   Ausbeute: 54 mg (32%)
   Rₜ-Wert: 0.94 min (Methode B)
   C₂₂H₂₆ClN₅O₃ (443.94) x CF₃CO₂H
   Massenspektrum: (M+H)⁺ = 444/446 (Chlorisotope)

Analog können die folgenden Verbindungen hergestellt werden:

| **Bsp.** | **Strukturformel** | **Ausbeute** | **Massenpeak(s)** | **DC/HPLC** |
|---|---|---|---|---|
| **9** | 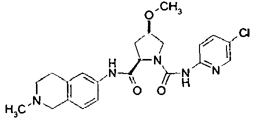 | 35% | (M+H)⁺ = 458/460 (Chlorisotope) | Rₜ-Wert: 1.04 min Methode B |
| | (2R,4R)-4-Methoxy-pyrrolidin-2-methyl-1,2-dicarbonsäure-1-[(5-chlor-pyridin-2-yl)-amid]-2-(2-methyl-1,2,3,4-tetrahydro-isochinolin-6-yl)-amid | | | |
| **10** | 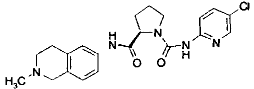 | 15% | (M+H)⁺ = 428/430 (Chlorisotope) | Rₜ-Wert: 0.98 min Methode B |
| | (2R)-Pyrrolidin-1,2-dicarbonsäure-1-[(5-chlor-pyridin-2-yl)-amid]-2-(2-methyl-1,2,3,4-tetrahydro-isochinolin-6-yl)-amid | | | |

### Beispiel 11

### (2R,4R)-4-Methoxy-pyrrolidin-1,2-dicarbonsäure-1-[(4-brom-phenyl)-amid]-2-[(2,3-dimethyl-1,2,3,4-tetrahydro-isochinolin-6-yl)-amid (Mischung von Diastereomeren)

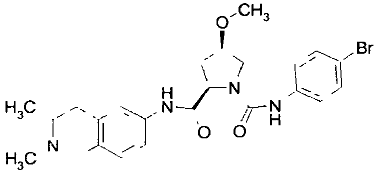

(a) Essigsäure-(3-methyl-1,2,3,4-tetrahydro-isochinolin-6-yl)-amid
   Eine Mischung aus 2.2 ml (31 mmol) Dioxolan,1.2 g (9.6 mmol) Piperidin Hydrochlorid, 1.78 g (7.8 mmol) Essigsäure-[3-(2-aminopropyl)phenyl]amid und 5 µl Konz. HCl werden für 7.5 h auf 90°C erwärmt. Nach dem Abkühlen wird mit Wasser und Essigester versetzt, die Wasserphase wird mit 2 N NaOH basisch gestellt und 3 x mit Essigester extrahiert. Nach dem Trocknen der organischen Phasen mit Na₂SO₄ wird das Gemisch konzentriert und durch Säulenchromatographie gereinigt (Kieselgel; CH₂Cl₂/EtOH : NH₄OH 95:5 110/0->4/1).
   R_{f}-Wert: 0.15 (Kieselgel; Dichlormethan/Ethanol/NH₄OH = 80:20:2)
   C₁₂H₁₆N₂O (204.27)
   Massenspektrum: (M+H)⁺ = 205.
(b) Essigsäure-(2,3-dimethyl-1,2,3,4-tetrahydro-isochinolin-6-yl)-amid Formiat
   0.71 g (3.5 mmol) Essigsäure-(3-methyl-1,2,3,4-tetrahydro-isochinolin-6-yl)-amid werden in 2.0 ml Ameisensäure unter Rühren bei Raumtemperatur mit 0.31 ml 37% Formalin-Lösung in Wasser versetzt und bei 70°C 4.5 h gerührt. Die Reaktionsmischung wird konzentriert, mehrmals mit Ethanol versetzt und wieder konzentriert.
   R_{f}-Wert: 0.23 (Kieselgel; Dichlormethan/Ethanol/NH₄OH = 80:20:2)
   C₁₂H₁₆N₂O X CH₂O₂ (264.32)
   Massenspektrum: (M+H)⁺ = 219.
(c) 6-Amino-2,3-dimethyl-1,2,3,4-tetrahydro-isochinolin Dihydrochlorid
   0.98 g (3.7 mmol) Essigsäure-(2,3-dimethyl-1,2,3,4-tetrahydro-isochinolin-6-yl)-amid werden im mehrstündigen Abstand mit insgesamt 15 ml 6N HCl versetzt und insgesamt 16 h gerührt. Anschließend wird das Reaktionsgemisch im Vakuum konzentriert.
   R_{f}-Wert: 0.84 (RP-8; Methanol/5%-NaCl-Lösung = 6:4)
   C₁₁H₁₆N₂ X 2HCl (249.18)
   Massenspektrum: (M+H)⁺ = 177.
(d) (2R,4R)-4-Methoxy-pyrrolidin-1,2-dicarbonsäure-1-[(4-brom-phenyl)-amid]-2-[(2,3-dimethyl-1,2,3,4-tetrahydro-isochinolin-6-yl)-amid (Mischung von Diastereomeren)
   Die Titelverbindungen werden aus 6-Amino-2,3-dimethyl-1,2,3,4-tetrahydroisochinolin Dihydrochlorid und (2R,4R)-4-Methoxy-2-methyl-pyrrolidin-1,2-dicarbonsäure-1-benzylester und 4-Bromphenylisocyanat gemäß der Reaktionssequenz 4e, 4f, 4g hergestellt.
   R_{f}-Wert: 0.42 (RP-8; Methanol/5%-NaCl-Lösung = 6:4)
   C₂₄H₂₉BrN₄O₃ (501.42)
   Massenspektrum: (M+H)⁺ = 501/503 (Bromisotope).

### Beispiel 12 und 13

### (2R,4R)-4-Methoxy-pyrrolidin-1,2-dicarbonsäure-1-[(4-brom-phenyl)-amid]-2-[(3S)-(2,3-dimethyl-1,2,3,4-tetrahydro-isochinolin-6-yl)-amid und (2R,4R)-4-Methoxy-pyrrolidin-1,2-dicarbonsäure-1-[(4-brom-phenyl)-amid]-2-[(3R)-(2,3-dimethyl-1,2,3,4-tetrahydro-isochinolin-6-yl)-amid

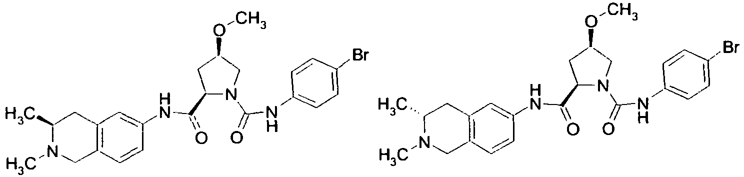

Analog Beispiel 11 können die beiden reinen Stereoisomeren hergestellt werden. Dazu wird Essigsäure-(3-methyl-1,2,3,4-tetrahydro-isochinolin-6-yl)-amid durch präparative Säulenchromatographie mit chiraler stationärer Phase in die Enantiomeren aufgetrennt (SupercriticlaFluidChromatographie: Säule DAICEL-ADH, 250 mm x 20 mm; Fluß 70 ml/min; Eluens: superkritisches CO2/Methanol + 0.2% Diemethylamin 87/13, Enantiomer 1 Rₜ-Wert: 5.8 min; Enantiomer 2 Rₜ-Wert: 6.7 min) und anschließend werden die einzelnen Enantiomere entsprechend der in Beispiel 11 beschriebenen Reaktionssequenz zu den Titelverbindungen umgesetzt.
Diastereomer 1
R_{f}-Wert: 0.39 (RP-8; Methanol/5%-NaCl-Lösung = 6:4)
C₂₄H₂₉BrN₄O₃ (501.42)
Massenspektrum: (M+H)⁺ = 501/503 (Bromisotope).
Diastereomer 2
R_{f}-Wert: 0.39 (RP-8; Methanol/5%-NaCl-Lösung = 6:4)
C₂₄H₂₉BrN₄O₃ (501.42)
Massenspektrum: (M+H)⁺ = 501/503 (Bromisotope).

### Beispiel 14

### (2R,4R)-4-Methoxy-pyrrolidin-1,2-dicarbonsäure-1-[(4-chlor-phenyl)-amid]-2-[(1,2-dimethyl-1,2,3,4-tetrahydro-isochinolin-6-yl)-amid (Mischung von Diastereomeren)

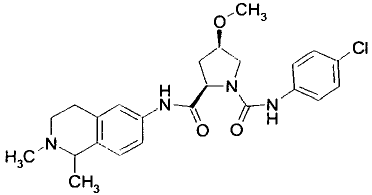

(a) (1-Methyl-3,4-dihydro-isoquinolin-6-yl)-carbaminsäuremethylester
   Eine Mischung aus 4.13 g (17.5 mmol) N-[3-(2-Methylcarbonylamino-ethyl)phenyl]carbaminsäuremethylester und 35 ml Chloroform wird langsam mit 8.00 g (38.4 mmol) Phosphorpentachlorid versetzt und 16 h gerührt. Anschließend wird das Gemisch vorsichtig in Wasser gegossen, 45 min gerührt. Es wird 3 x mit Methylenchlorid extrahiert und anschließend wird die Wasserphase mit 4N NaOH basisch gestellt. Die ausfallenden Kristalle werden abgesaugt und getrocknet
   Ausbeute: 2.6 g (68%)
   Massenspektrum: (M+H)⁺ = 219
(b) (1,2-Dimethyl-1,2,3,4-tetrahydro-isoquinolin-6-yl)-carbaminsäuremethylester
   Eine Mischung aus 1.00 g (4.5 mmol) (1-Methyl-3,4-dihydro-isoquinolin-6-yl)-carbaminsäuremethylester, 2.3 ml (37 mmol) Methyliodid und 25 ml EtOAc wird 72 h gerührt. Die ausgefallenen Kristalle werden abfiltriert in 10 ml Methanol aufgenommen und portionsweise mit 140 mg (3.6 mmol) Natriumborhydrid versetzt. Nach 2 h wird das Gemisch konzentriert und chromatographisch gereinigt (Kieselgel; Dichlormethan/Methanol 90:10)..
   Ausbeute: 93%
   Massenspektrum: (M+H)⁺ = 236
   Rₜ-Wert: 0.78 min Methode B
(c) 6-Amino-1,2-dimethyl-1,2,3,4-tetrahydro-isoquinolin Hydrobromid
   Eine Mischung aus 110 mg (0.47 mmol) (1,2-Dimethyl-1,2,3,4-tetrahydro-isoquinolin-6-yl)-carbaminsäuremethylester, 2.0 MI 33% HBr in Eisessig und 2.0 ml Eisessig werden 1.5 h zum Sieden erhitzt. Anschließend wird das Gemisch im Vakuum konzentriert, mit Wasser versetzt und die Wasserphase abgetrennt und gefriergetrocknet.
   Massenspektrum: (M+H)⁺ = 177
   Rₜ-Wert: 0.28 min Methode B
(d) (2R,4R)-4-Methoxy-pyrrolidin-1,2-dicarbonsäure-1-[(4-chlor-phenyl)-amid]-2-[(1,2-dimethyl-1,2,3,4-tetrahydro-isochinolin-6-yl)-amid x CF3COOH (Mischung von Diastereomeren)
   Eine Mischung aus 100 mg 6-Amino-1,2-dimethyl-1,2,3,4-tetrahydro-isoquinolin Hydrobromid, 90 mg (0.30 mmol) (2R,4R)-1-(4-Chlor-phenylcarbamoyl)-4-methoxy-pyrrolidin-2-carbonsäure und 0.25 ml Triethylamin in 5 ml THF wird langsam mit 0.71 ml 50% Propanphosphonsäurecycloanhydrid in Essigester versetzt und 3 h auf 75°C erhitzt. Anschließend wird das Reaktionsgemisch im Vakuum konzentriert, mit TFA sauer gestellt und chromatographisch gereinigt.
   Rₜ-Wert: 1.13 min (Methode B)
   C₂₄H₂₉ClN₄O₃ (456.97) x CF₃CO₂H
   Massenspektrum: (M+H)⁺ = 457/459 (Chlorisotope)

### Beispiel 15 und 16

### (2R,4R)-4-Methoxy-pyrrolidin-1,2-dicarbonsäure-1-[(4-brom-phenyl)-amid]-2-[(R)-(1,2-dimethyl-1,2,3,4-tetrahydro-isochinolin-6-yl)-amid und (2R,4R)-4-Methoxy-pyrrolidin-1,2-dicarbonsäure-1-[(4-brom-phenyl)-amid]-2-[(S)-(1,2-dimethyl-1,2,3,4-tetrahydro-isochinolin-6-yl)-amid

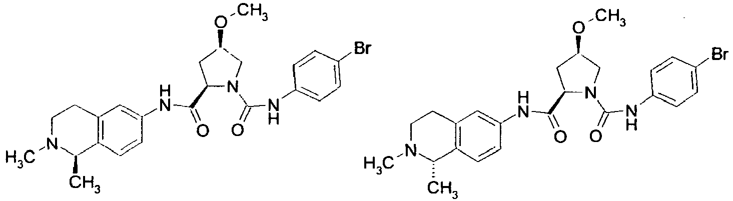

Analog Beispiel 14 können die beiden reinen Stereoisomeren hergestellt werden. Dazu wird 6-Amino-1,2-dimethyl-1,2,3,4-tetrahydro-isoquinolin Hydrobromid durch präparative Säulenchromatographie mit chiraler stationärer Phase in die Enantiomeren aufgetrennt (DAICEL OJ-H, 250 mm x 20 mm, 5 µM, Hexan + 0.2% Cyclohexylamin/Isopropanol 60/40, Flußrate 15 ml/min, Enantiomer 1 Rₜ-Wert: 9.2 min; Enantiomer 2 Rₜ-Wert: 14.1 min) und anschließend werden die einzelnen Enantiomere entsprechend der in Beispiel 14 beschriebenen Reaktionssequenz zu den Titelverbindungen umgesetzt.
Diastereomer 1
Rₜ-Wert: 1.20 min (Methode B)
C₂₄H₂₉BrN₄O₃ (501.42) x CF₃CO₂H
Massenspektrum: (M+H)⁺ = 501/503 (Bromisotope)
Diastereomer 2
Rₜ-Wert: 1.19 min (Methode B)
C₂₄H₂₉BrN₄O₃ (501.42) x CF₃CO₂H
Massenspektrum: (M+H)⁺ = 501/503 (Bromisotope)

### Beispiel 52

### (2R,4R)-4-Methoxy-pyrrolidin-1,2-dicarbonsäure-1-[(4-chlor-phenyl)-amid]-2-[(2-methyl-1,2,3,4-tetrahydro-8-aza-isochinolin-6-yl)-amid

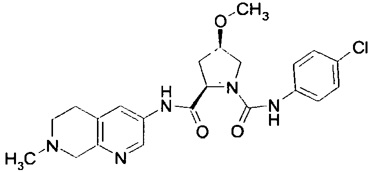

(a) 2-Methyl-6-nitro-1,2,3,4-tetrahydro-8-aza-isochinolin
   Eine Mischung aus 400 mg (2.0 mmol) 1-Methyl-3,5-dinitro-pyridon, 300 mg 1-Methyl-piperidin-3-on und 15 ml 2M Ammoniak in Methanol werden 20 h auf 60°C erwärmt. Es wird konzentriert und durch Chromatographie gereinigt (Kieselgel, CH₂Cl₂/MeOH 98/2).
   Ausbeute: 15%
   Rₜ-Wert: 0.25 min (Methode B)
   Massenspektrum: (M+H)⁺ = 194
(b) 2-Methyl-6-amino-1,2,3,4-tetrahydro-8-aza-isochinolin
   Eine Mischung aus 75 mg (0.311 mmol) 2-Methyl-6-nitro-1,2,3,4-tetrahydro-8-aza-isochinolin, 50 mg Raney-Nickel und 10 ml Methanol wird bei 3 bar Wasserstoffdruck 4 h reduziert. Anschließend wird filtriert und eingeengt. Ausbeute: quantitativ
   R_{f}-Wert: 0.1 (Kieselgel; CH₂Cl₂/Methanol 9/1)
   Massenspektrum: (M+H)⁺ = 164
(c) (2R,4R)-4-Methoxy-pyrrolidin-1,2-dicarbonsäure-1-[(4-chlor-phenyl)-amid]-2-[(2-methyl-1,2,3,4-tetrahydro-8-aza-isochinolin-6-yl)-amid x CF₃COOH
   Man erhält die Titelverbindung aus 2-Methyl-6-amino-1,2,3,4-tetrahydro-8-aza-isochinolin analog Beispiel 14 d.
   Rₜ-Wert: 1..03 min (Methode B)
   C₂₂H₂₆ClN₅O₃ (443.93) x CF₃CO₂H
   Massenspektrum: (M+H)⁺ = 444/446 (Chlorisotope)

### Beispiel 53

### (2R,4R)-4-Methoxy-2-methyl-pyrrolidin-1,2-dicarbonsäure-1-[(5-chlor-pyridin-2-yl)-amid]-2-[(2-methyl-1,2,3,4-tetrahydro-8-aza-isochinolin-6-yl)-amid

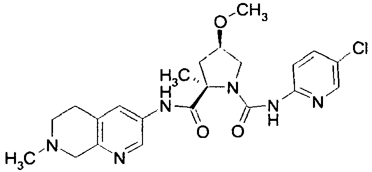

Man erhält die Titelverbindung aus 2-Methyl-6-amino-1,2,3,4-tetrahydro-8-aza-isochinolin analog Beispiel 6 a, 6b, 6c.
Rₜ-Wert: 0.96 min (Methode B)
C₂₂H₂₇ClN₆O₃ (458.94) x CF₃CO₂H
Massenspektrum: (M+H)⁺ = 459/461 (Chlorisotope)

### Beispiel 54

### (2R,4R)-4-Methoxy-pyrrolidin-1,2-dicarbonsäure-1-[(4-chlor-phenyl)-amid]-2-[(2-methyl-1,2,3,4-tetrahydro-5-aza-isochinolin-7-yl)-amid x CF3COOH

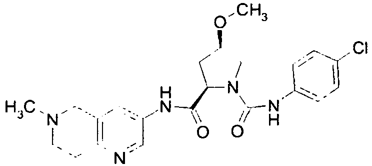

2-Methyl-7-amino-1,2,3,4-tetrahydro-5-aza-isochinolin wurde analog Beispiel 52 a und 52b aus 1-Methyl-3,5-dinitro-pyridon und 1-Methyl-piperidin-4-on hergestellt und gemäß Beispiel 14 d zur Titelverbindung umgesetzt.
Rₜ-Wert: 0.99 min (Methode B)
C₂₂H₂₆ClN₅O₃ (458.94) x CF₃CO₂H
Massenspektrum: (M+H)⁺ = 444/446 (Chlorisotope)

### Beispiel 55

### (2R,4R)-4-Methoxy-pyrrolidin-1,2-dicarbonsäure-1-[(4-chlor-phenyl)-amid]-2-[(1,2-dimethyl-1,2,3,4-tetrahydro-8-aza-isochinolin-6-yl)-amid x CF3COOH

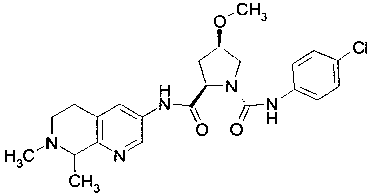

1,2-Dimethyl-6-amino-1,2,3,4-tetrahydro-8-aza-isochinolin wurde analog Beispiel 52 a aus 1-Methyl-3,5-dinitro-pyridon und 1-Butoxycarbonyl-piperidin-2-methyl-3-on, anschließender Butoxycarbonylabspaltung mit TFA, Leukart-Wallach-Reaktion gemäß Beispiel 11b und Reduktion der Nitrogruppe analog Beispiel 52b hergestellt und gemäß Beispiel 14 d zur Titelverbindung umgesetzt.
Rₜ-Wert: 1.07 min (Methode B)
C₂₂H₂₈ClN₅O₃ (457.96) x CF₃CO₂H
Massenspektrum: (M+H)⁺ = 458/460 (Chlorisotope)

### Beispiel 56

### (2R,4R)-4-Methoxy-pyrrolidin-1,2-dicarbonsäure-1-[(4-chlor-phenyl)-amid]-2-[(2-methyl-1,2,3,4-tetrahydro-8-methoxycarbonyl-isochinolin-6-yl)-amid

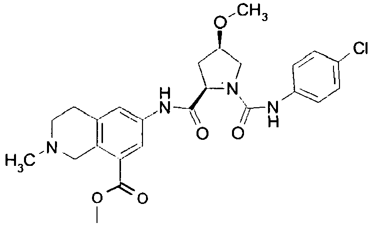

(a) 2-Methyl-1,2,3,4-tetrahydro-8-methoxycarbonyl-isochinolin
   2-Butoxycarbonyl-1,2,3,4-tetrahydro-8-methoxycarbonyl-isochinolin wird mit methanolischer HCl entschützt und anschließend gemäß Beispiel 11 b methyliert. Rₜ-Wert: 0.84 min (Methode B) Massenspektrum: (M+H)⁺ = 206
(b) 2-Methyl-6-nitro-1,2,3,4-tetrahydro-8-methoxycarbonyl-isochinolin und Regioisomere
   Eine Mischung aus 1.04 g (5.0 mmol) 2-Methyl-1,2,3,4-tetrahydro-8-methoxycarbonyl-isochinolin und Schwefelsäure wird langsam bei -7°C mit 0.57 g-Kaliumnitrat versetzt, 15 min bei -7°C und 1 h bei Raumtemperatur gerührt. Anschließend wird langsam auf Eiswasser gegossen, mit NaOH alkalisch gestellt. Die ausgefallenen Kristalle werden abfiltriert und getrocknet. Man erhält ein Gemisch von Regioisomeren.
   Rₜ-Wert: 0.87 min (Methode B)
   Massenspektrum: (M+H)⁺ = 251
(c) (2R,4R)-4-Methoxy-pyrrolidin-1,2-dicarbonsäure-1-[(4-chlor-phenyl)-amid]-2-[(2-methyl-1,2,3,4-tetrahydro-8-methoxycarbonyl-isochinolin-6-yl)-amid
   Man erhält die Titelverbindung aus 2-Methyl-6-nitro-1,2,3,4-tetrahydro-8-methoxycarbonyl-isochinolin (im Gemisch mit Regioisomeren) durch Reduktion der Niotrogruppe mit Pd/Kohle und anschließende Amidkupplung von 2-Methyl-6-amino-1,2,3,4-tetrahydro-8-methoxycarbonyl-isochinolin gemäß Beispiel 14 d.
   Rₜ-Wert: 1.18 min (Methode B)
   C₂₅H₂₉ClN₄O₅ (500.98) x CF₃CO₂H
   Massenspektrum: (M+H)⁺ = 501/503 (Chlorisotope)

### Beispiel 57

### (2R,4R)-4-Methoxy-pyrrolidin-1,2-dicarbonsäure-1-[(4-chlor-phenyl)-amid]-2-[(2-methyl-4-methoxy-1,2,3,4-tetrahydro-isochinolin-6-yl)-amid (Isomerengemisch)

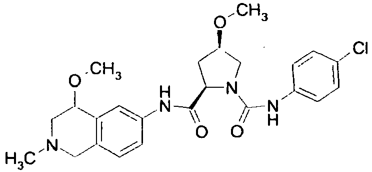

(a) N-Methyl-N-(4-nitrophenylmethyl)-2,2-dimethoxy-ethylamin
   Eine Mischung aus 1.56 g N-Methyl-N-(4nitrobenzyl)amin in 40 ml THF wird mit 2.85 ml einer 45% Lösung von 2,2-Dimethoxyacetaldehyd in tert.-Butylmethylether versetzt. Dann werden 24 mg p-TsOHx H2O und 1.12 ml Eisessig zugegeben und 2 h gerührt. Anschließend gibt man portionsweise 1.81 g Natriumcyanborhydrid zu und rührt weitere 2 h nach. Zur Mischung werden 5ml Wasser gegeben, dann wird auf ca. 30% des Volumens eingeengt, der Rückstand wird mit wasser versetzt und 3 x mit EtOAc extrahiert. Die vereinigten organischen Phasen werden mit Na₂SO₄ getrocknet, konzentriert und das rohprodukt wird durch Chromatographie gereinigt (Alox; Petrolether/EtOAc 8/2 -> 7/3).
   Rₜ-Wert: 2.2 min (Methode D)
   Massenspektrum: (M+H)⁺ = 255
(b) 6-Nitro-4-methoxy-2-methyl-1,2,3,4-tetrahydro-isochinolin
   Eine Mischung aus 0.83 g N-Methyl-N-(4-nitrophenylmethyl)-2,2-dimethoxyethylamin und 3.0 ml Trifluormethansulfonsäure wird im Trockeneis/Ethanolkühlbad hergestellt, langsam auf Raumtemperatur gebracht und 18 h gerührt. Dann wird auf Eiswasser gegossen, mit 2N NaOH alkalische gestellt, 3x mit EtOAc extrahiert, die organischen Phasen werden mit Na₂SO₄ getrocknet konzentriert und durch mehrmalige Chrtomatographie gereinigt.
   Rₜ-Wert: 1.7 min (Methode D)
   Massenspektrum: (M+H)⁺ = 223
(c) 6-Amino-4-methoxy-2-methyl-1,2,3,4-tetrahydro-isochinolin
   Eine Mischung aus 70 mg 6-Nitro-4-methoxy-2-methyl-1,2,3,4-tetrahydroisochinolin, 25 mg Pd/Kohle und 5.0 ml MeOH wird 9 h bei 3 bar Wasserstoffdruck hydriert. Anschließend wird filtriert und eingeengt.
   Ausbeute : quantitativ
   R_{f}-Wert: 0.75 (RP-8; Methanol/5%-NaCl-Lösung = 6:4)
   Massenspektrum: (M+H)⁺ = 193
(d) (2R,4R)-4-Methoxy-pyrrolidin-1,2-dicarbonsäure-1-[(4-chlor-phenyl)-amid]-2-[(2-methyl-4-methoxy-1,2,3,4-tetrahydro-isochinolin-6-yl)-amid (Isomerengemisch)
   Die Titelverbindung wird aus 6-Amino-4-methoxy-2-methyl-1,2,3,4-tetrahydroisochinolin und (2R,4R)-1-(4-Chlor-phenylcarbamoyl)-4-methoxy-pyrrolidin-2-carbonsäure gemäß Beispiel 4 e hergestellt.
   Rₜ-Wert: 2.8 min (Methode D)
   C₂₄H₂₉ClN₄O₄ (472.96) x HCO₂H
   Massenspektrum: (M+H)⁺ = 473/475 (Chlorisotope)

### Beispiel 58

### (2R,4R)-4-Methoxy-pyrrolidin-1,2-dicarbonsäure-1-[(4-brom-phenyl)-amid]-2-[(2-methyl-5,8-difluor-1,2,3,4-tetrahydro-isochinolin-7-yl)-amid x HCOOH

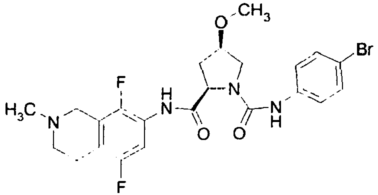

(a) 2-Methyl-5,8-difluor-1,2,3,4-tetrahydro-isochinolin
   Die Titelverbindung wird aus 5,8-Difluor-1,2,3,4-tetrahydro-isochinolin gemäß Beispiel 11 b hergestellt.
   Rₜ-Wert: 1.6 min (Methode D)
   Massenspektrum: (M+H)⁺ = 184
(b) 2-Methyl-5,8-difluor-7-nitro-1,2,3,4-tetrahydro-isochinolin
   Eine eisgekühlte Mischung aus 0.94 g (5.1 mmol) 2-Methyl-5,8-difluor-1,2,3,4-tetrahydro-isochinolin und 2.8 ml konz. H₂SO₄ wird langsam mit 0.36 ml 65% Salpetersäure versetzt. Dann wird 2.5 h unter Eiskühlung gerührt und das Gemisch dann auf Eiswasser gegossen. Es wird mit NaOH alkalisch gestellt, 3 x mit EtOAc extrahiert, die organischen Phasen werden mit Na₂SO₄ getrocknet , filtzriert und konzentriert.
   Massenspektrum: (M+H)⁺ = 229
(c) (2R,4R)-4-Methoxy-pyrrolidin-1,2-dicarbonsäure-1-[(4-brom-phenyl)-amid]-2-[(2-methyl-5,8-difluor-1,2,3,4-tetrahydro-isochinolin-7-yl)-amid x HCOOH
   Die Titelverbindung wird aus 2-Methyl-5,8-difluor-7-nitro-1,2,3,4-tetrahydroisochinolin analog der Synthesesequenz 52b, 14d zur Titelverbindung umgesetzt.
   R_{f}-Wert: 0.25 (Kieselgel; Dichlormethan/Ethanol/Ammoniak = 95:5:0.5)
   C₂₃H₂₅BrF₂N₄O₃ (523.36) x HCO₂H
   Massenspektrum: (M+H)⁺ = 523/525 (Bromisotope)

Analog zu den obigen Syntheseschritten bzw. in Analogie zu Literatursynthesewegen können die folgenden Verbindungen aus literaturbekannten oder in Analogie zu literaturbekannten Synthesewegen herstellbaren Anilinen und Prolinderivaten hergestellt werden:

| **Bsp.** | **Strukturformel** | **Ausbeute letzte Stufe** | **Massenpeak(s)** | **DC/HPLC** |
|---|---|---|---|---|
| **17** | 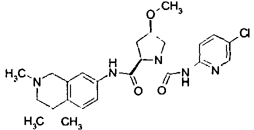 | 11% | (M-H)⁻ = 470/472 (Chlorisotope) | Rₜ-Wert: 2.65 min Methode C |
| | (2R,4R)-4-Methoxy-pyrrolidin-1,2-dicarbonsäure-1-[(5-chlor-pyridin-2-yl)-amid]-2-(2,4,4-trimethyl-1,2,3,4-tetrahydro-isochinolin-7-yl)-amid | | | |
| **18** | 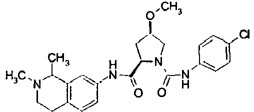 | 68% | (M-H)⁻ = 457/459 (Chlorisotope) | Rₜ-Wert: 1.12 min Methode B |
| | (2R,4R)-4-Methoxy-pyrrolidin-1,2-dicarbonsäure-1-[(4-chlor-phenyl)-amid]-2-(1,2-dimethyl-1,2,3,4-tetrahydro-isochinolin-7-yl)-amid x CF₃COOH, Stereoisomerenmischung | | | |
| **19** | 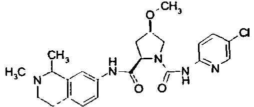 | 33% | (M+H)⁺ = 458/460 (Chlorisotope) | Rₜ-Wert: 0.99 min Methode B |
| | (2R,4R)-4-Methoxy-pyrrolidin-1,2-dicarbonsäure-1-[(5-chlor-pyridin-2-yl)-amid]-2-(1,2-dimethyl-1,2,3,4-tetrahydro-isochinolin-7-yl)-amid x CF₃COOH, Stereoisomerenmischung | | | |
| **20** | 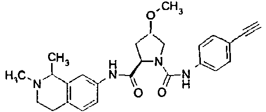 | 18% | (M+H)⁺ = 447 | Rₜ-Wert: 1.11 min Methode B |
| | (2R,4R)-4-Methoxy-pyrrolidin-1,2-dicarbonsäure-1-[(4-ethinyl-phenyl)-amid]-2-(1,2-dimethyl-1,2,3,4-tetrahydro-isochinolin-7-yl)-amid x CF₃COOH, Stereoisomerenmischung | | | |
| **21** | 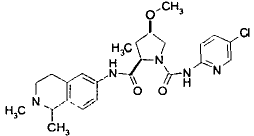 | 5% | (M+H)⁺ = 472/474 (Chlorisotope) | Rₜ-Wert: 1.11 min Methode B |
| | (2R,4R)-4-Methoxy-2-methyl-pyrrolidin-1,2-dicarbonsäure-1-[(5-chlor-pyridin-2-yl)-amid]-2-(1,2-dimethyl-1,2,3,4-tetrahydro-isochinolin-6-yl)-amid x CF₃COOH, Stereoisomerenmischung | | | |
| **22** | 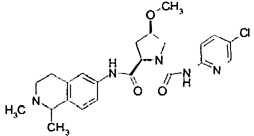 | 4% | (M+H)⁺ = 458/460 (Chlorisotope) | |
| | (2R,4R)-4-Methoxy-pyrrolidin-1,2-dicarbonsäure-1-[(5-chlor-pyridin-2-yl)-amid]-2-(1,2-dimethyl-1,2,3,4-tetrahydro-isochinolin-6-yl)-amid x CF₃COOH, Stereoisomerenmischung | | | |
| **23** | 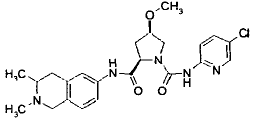 | 8% | (M+H)⁺ = 458/460 (Chlorisotope) | R_{F}-Wert: 0.72; Kieselgel:C H₂Cl₂/EtOH /NH₄OH = 80/20/2 |
| | (2R,4R)-4-Methoxy-pyrrolidin-1,2-dicarbonsäure-1-[(5-chlor-pyridin-2-yl)-amid]-2-(2,3-dimethyl-1,2,3,4-tetrahydro-isochinolin-6-yl)-amid, Stereoisomer 1 | | | |
| **24** | 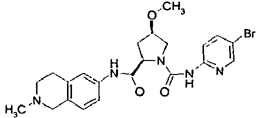 | 18% | (M+H)⁺ = 488/490 (Bromisotope) | Rₜ-Wert: 0.96 min Methode B |
| | (2R,4R)-4-Methoxy-pyrrolidin-1,2-dicarbonsäure-1-[(5-brom-pyridin-2-yl)-amid]-2-(2-methyl-1,2,3,4-tetrahydro-isochinolin-6-yl)-amid | | | |
| **25** | 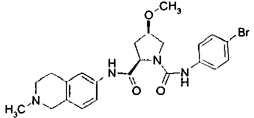 | 70% | (M+H)⁺ = 487/489 (Bromisotope) | Rₜ-Wert: 1.13 min Methode B |
| | (2R,4R)-4-Methoxy-pyrrolidin-1,2-dicarbonsäure-1-[(4-brom-phenyl)-amid]-2-(2-methyl-1,2,3,4-tetrahydro-isochinolin-6-yl)-amid | | | |
| **26** | 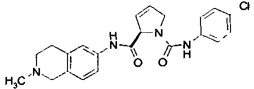 | 54 % | (M+H)⁺ = 411/413 (Chlorisotope) | Rₜ-Wert: 1.12 min Methode B |
| | (2R)-3,4-Dehydropyrrolidin-1,2-dicarbonsäure-1-[(4-chlor-phenyl)-amid]-2-(2-methyl-1,2,3,4-tetrahydro-isochinolin-6-yl)-amid x CF₃COOH | | | |
| **27** | 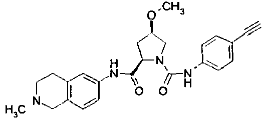 | 16% | (M+H)⁺ = 433 | Rt-Wert: 1.09 min Methode B |
| | (2R,4R)-4-Methoxy-pyrrolidin-1,2-dicarbonsäure-1-[(4-ethinyl-phenyl)-amid]-2-(2-methyl-1,2,3,4-tetrahydro-isochinolin-6-yl)-amid x CF₃COOH | | | |
| **28** | 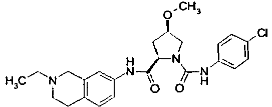 | 25 % | (M+H)⁺ = 457/459 (Chlorisotope) | |
| | (2R,4R)-4-Methoxy-pyrrolidin-1,2-dicarbonsäure-1-[(4-chlor-phenyl)-amid]-2-(2-ethyl-1,2,3,4-tetrahydro-isochinolin-7-yl)-amid | | | |
| **29** | 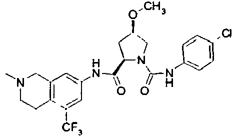 | 5% | (M+H)⁺ = 511/513 (Chlorisotope) | R_{F}-Wert: 0.75; Kieselgel:C H₂Cl₂/EtOH /NH₄OH = 80/20/2 |
| | (2R,4R)-4-Methoxy-pyrrolidin-1,2-dicarbonsäure-1-[(4-chlor-phenyl)-amid]-2-(2-methyl-5-trifluormethyl-1,2,3,4-tetrahydro-isochinolin-7-yl)-amid x HCOOH | | | |
| **30** | 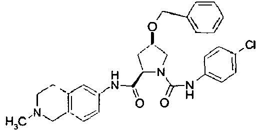 | 31% | (M+H)⁺ = 519/521 (Chlorisotope) | Rₜ-Wert: 1.36 min Methode B |
| | (2R,4R)-4-Benzyloxy-pyrrolidin-1,2-dicarbonsäure-1-[(4-chlor-phenyl)-amid]-2-(2-methyl-1,2,3,4-tetrahydro-isochinolin-6-yl)-amid | | | |
| **31** | 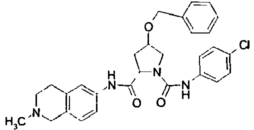 | 33% | (M+H)⁺ = 519/521 (Chlorisotope) | Rₜ-Wert: 1.44 min Methode B |
| | 4-Benzyloxy-pyrrolidin-1,2-dicarbonsäure-1-[(4-chlor-phenyl)-amid]-2-(2-methyl-1,2,3,4-tetrahydro-isochinolin-6-yl)-amid, Stereoisomer von Bsp. 30 | | | |
| **32** | 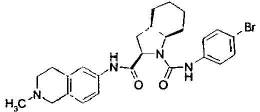 | 7% | (M+H)⁺ = 511/513 (Bromisotope) | Rₜ-Wert: 1.40 min Methode B |
| | (2R,3aR, 8aR)-Octahydro-cyclohepta[*b*]pyrrol-1,2-dicarbonsäure-1-[(4-brom-phenyl)-amid]-2-(2-methyl-1,2,3,4-tetrahydro-isochinolin-6-yl)-amid | | | |
| **33** | 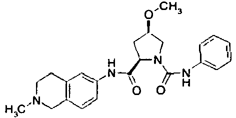 | 42% | (M+H)⁺ = 409 | Rₜ-Wert: 1.01 min Methode B |
| | (2R,4R)-4-Methoxy-pyrrolidin-1,2-dicarbonsäure-1-phenylamid-2-(2-methyl-1,2,3,4-tetrahydro-isochinolin-7-yl)-amid x CF₃COOH | | | |
| **34** | 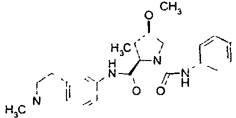 | 31 % | (M+H)⁺ = 447 | Rₜ-Wert: 1.01 min Methode B |
| | (2R,4R)-4-Methoxy-2-methyl-pyrrolidin-1,2-dicarbonsäure-1-[(4-ethinyl-phenyl)-amid]-2-(2-methyl-1,2,3,4-tetrahydro-isochinolin-6-yl)-amid | | | |
| **35** | 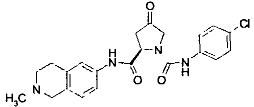 | 9% | (M+H)⁺ = 427/429 (Chlorisotope) | Rₜ-Wert: 1.09 min Methode B |
| | (2R)-4-Oxo-pyrrolidin-1,2-dicarbonsäure-1-[(4-chlor-phenyl)-amid]-2-(2-methyl-1,2,3,4-tetrahydro-isochinolin-6-yl)-amid | | | |
| **36** | 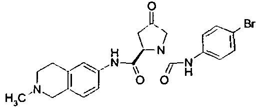 | 9% | (M+H)⁺ = 471/473 (Bromisotope) | Rₜ-Wert: 1.12 min Methode B |
| | (2R)-4-Oxo-pyrrolidin-1,2-dicarbonsäure-1-[(4-brom-phenyl)-amid]-2-(2-methyl-1,2,3,4-tetrahydro-isochinolin-6-yl)-amid | | | |
| **37** | 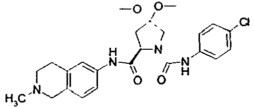 | 3% | (M+H)⁺ = 473/475 (Chlorisotope) | Rₜ-Wert: 1.17 min Methode B |
| | (2R)-4,4-Dimethyloxy-pyrrolidin-1,2-dicarbonsäure-1-[(4-chlor-phenyl)-amid]-2-(2-methyl-1,2,3,4-tetrahydro-isochinolin-6-yl)-amid x CF₃COOH | | | |
| **38** | 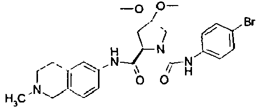 | 5% | (M+H)⁺ = 517/519 (Bromisotope) | Rₜ-Wert: 1.21 min Methode B |
| | (2R)-4,4-Dimethyloxy-pyrrolidin-1,2-dicarbonsäure-1-[(4-brom-phenyl)-amid]-2-(2-methyl-1,2,3,4-tetrahydro-isochinolin-6-yl)-amid x CF₃COOH | | | |
| **39** | 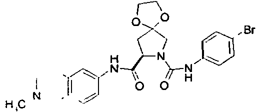 | 31% | (M+H)⁺ = 515/517 (Bromisotope) | R_{F}-Wert: 0.17; Kieselgel:C H₂Cl₂/EtOH /NH₄OH = 90/10/1 |
| | (8R)-1,4-Dioxa-7-aza-spiro[4.4]nonane-7,8-dicarbonsäure-7-[(4-brom-phenyl)-amid]-8-(2-methyl-1,2,3,4-tetrahydro-isochinolin-6-yl)-amid x HCOOH | | | |
| **40** | 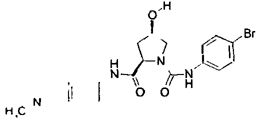 | 64 % | (M-H)⁻ = 471/473 (Bromisotope) | R_{f}-Wert: 0.49 (RP-8; Methanol/5 %-NaCl-Lösung = 6:4) |
| | (2R,4R)-4-Hydroxy-pyrrolidin-1,2-dicarbonsäure-1-(4-bromphenyl)-amid-2-(2-methyl-1,2,3,4-tetrahydro-isochinolin-6-yl)-amid | | | |
| **41** | 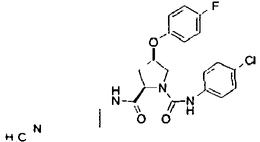 | 52 % | (M+H)⁺ = 523/525 (Chlorisotope) | Rₜ-Wert: 1.27 min Methode B |
| | (2R,4R)-4-(4-Fluorphenyloxy)-pyrrolidin-1,2-dicarbonsäure-1-(4-chlorphenyl)-amid-2-(2-methyl-1,2,3,4-tetrahydro-isochinolin-6-yl)-amid x CF₃COOH | | | |
| **42** | 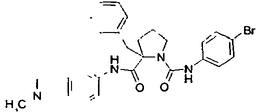 | 32 % | (M+H)⁺ = 545/547 (Bromisotope) | |
| | (rac)-2-Benzyl-pyrrolidin-1,2-dicarbonsäure-1-(4-bromphenyl)-amid-2-(2-methyl-1,2,3,4-tetrahydro-isochinolin-6-yl)-amid x HCOOH | | | |
| **43** | 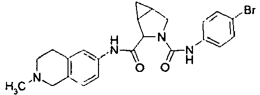 | 39% | (M+H)⁺ = 469/471 (Bromisotope) | R_{f}-Wert: 0.12 (RP-8; Methanol/5 %-NaCl-Lösung = 6:4) |
| | 3-Aza-bicyclo[3.1.0]hexane-2,3-dicarbonsäure-3-(4-bromphenyl)amid-2-(2-methyl-1,2,3,4-tetrahydro-isochinolin-6-yl)amid x HCOOH (Stereoisomerengemisch) | | | |
| **44** | 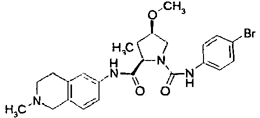 | 31 % | (M+H)⁺ = 501/503 (Bromisotope) | Rₜ-Wert: 3.0 min Methode D |
| | (2R,4R)-4-Methoxy-2-methyl-pyrrolidin-1,2-dicarbonsäure-1-[(4-brom-phenyl)-amid]-2-(2-methyl-1,2,3,4-tetrahydro-isochinolin-6-yl)-amid x HCOOH | | | |
| **45** | 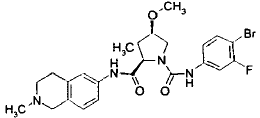 | 30% | (M+H)⁺ = 519/521 (Bromisotope) | Rₜ-Wert: 3.1 min Methode D |
| | (2R,4R)-4-Methoxy-2-methyl-pyrrolidin-1,2-dicarbonsäure-1-[(3-fluor-4-brom-phenyl)-amid]-2-(2-methyl-1,2,3,4-tetrahydro-isochinolin-6-yl)-amid x HCOOH | | | |
| **46** | 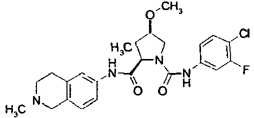 | 25 % | (M+H)⁺ = 475/477 (Chlorisotope) | Rₜ-Wert: 3.1 min Methode D |
| | (2R,4R)-4-Methoxy-2-methyl-pyrrolidin-1,2-dicarbonsäure-1-[(3-fluor-4-chlor-phenyl)-amid]-2-(2-methyl-1,2,3,4-tetrahydro-isochinolin-6-yl)-amid x HCOOH | | | |
| **47** | 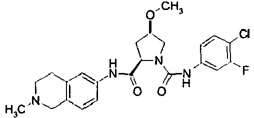 | 42 % | (M+H)⁺ = 461/463 (Chlorisotope) | R_{F}-Wert: 0.60; Kieselgel:C H₂Cl₂/EtOH /NH₄OH = 80/20/2 |
| | (2R,4R)-4-Methoxy-pyrrolidin-1,2-dicarbonsäure-1-[(3-fluor-4-chlor-phenyl)-amid]-2-(2-methyl-1,2,3,4-tetrahydro-isochinolin-6-yl)-amid x HCOOH | | | |
| **48** | 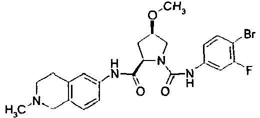 | 50% | (M-H)⁻ = 503/505 (Bromisotope) | R_{F}-Wert: 0.62; Kieselgel:C H₂Cl₂/EtOH /NH₄OH = 80/20/2 |
| | (2R,4R)-4-Methoxy-pyrrolidin-1,2-dicarbonsäure-1-[(3-fluor-4-brom-phenyl)-amid]-2-(2-methyl-1,2,3,4-tetrahydro-isochinolin-6-yl)-amid x HCOOH | | | |
| **49** | 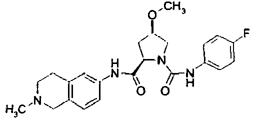 | 61 % | (M+H)⁺ = 427 | R_{f}-Wert: 0.52 (RP-8; Methanol/5 %-NaCl-Lösung = 6:4) |
| | (2R,4R)-4-Methoxy-pyrrolidin-1,2-dicarbonsäure-1-[(4-fluor-phenyl)-amid]-2-(2-methyl-1,2,3,4-tetrahydro-isochinolin-6-yl)-amid x HCOOH | | | |
| **50** | 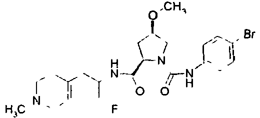 | 9% | (M+H)⁺ = 505/507 (Bromisotupe) | Rₜ-Wert: 3.1 min Methode D |
| | (2R,4R)-4-Methoxy-pyrrolidin-1,2-dicarbonsäure-1-[(4-brom-phenyl)-amid]-2-(2-methyl-7-fluor-1,2,3,4-tetrahydro-isochinolin-6-yl)-amid x HCOOH | | | |
| **51** | 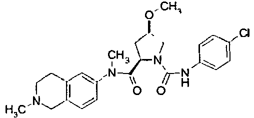 | 23 % | (M-H)⁻ = 457/459 (Chlorisotope) | Rₜ-Wert: 2.67 min Methode D |
| | (2R,4R)-4-Methoxy-pyrrolidin-1,2-dicarbonsäure-1-(4-chlorphenyl)-amid-2-methyl-2-(2-methyl-1,2,3,4-tetrahydro-isochinolin-6-yl)-amid x HCOOH | | | |
| **59** | 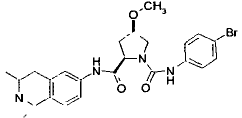 | 32% | (M+H)⁺ = 515/517 (Bromisotope) | R_{f}-Wert: 0.42 (RP-8; Methanol/5 %-NaCl-Lösung = 6:4) |
| | (2R,4R)-4-Methoxy-pyrrolidin-1,2-dicarbonsäure-1-[(4-brom-phenyl)-amid]-2-(1,1,3-trimethyl-1,2,3,4-tetrahydro-isochinolin-6-yl)-amid x HCOOH | | | |
| **60** | 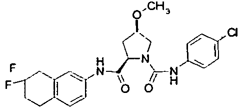 | 27 % | (M+H)⁺ = 464/466 (Chlorisotope) | Rₜ-Wert: 1.64 min Methode B |
| | (2R,4R)-4-Methoxy-pyrrolidin-1,2-dicarbonsäure-1-[(4-chlor-phenyl)-amid]-2-(2,2-difluor-1,2,3,4-tetrahydro-naphthalin-7-yl)-amid | | | |
| **61** | 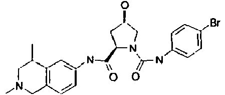 | | | |
| | (2R,4R)-4-Methoxy-pyrrolidin-1,2-dicarbonsäure-1-[(4-brom-phenyl)-amid]-2-(2,4-dimethyl-1,2,3,4-tetrahydro-isochinolin-6-yl)-amid | | | |
| **62** | 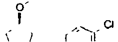 | | | |
| | (2R,4R)-4-Methoxy-pyrrolidin-1,2-dicarbonsäure-1-[(5-chlor-pyridin-2-yl)-amid]-2-(2,4-dimethyl-1,2,3,4-tetrahydro-isochinolin-6-yl)-amid | | | |
| **63** | 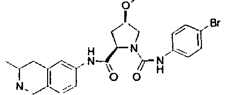 | | | |
| | (2R,4R)-4-Methoxy-pyrrolidin-1,2-dicarbonsäure-1-[(4-brom-phenyl)-amid]-2-(3-methyl-1,2,3,4-tetrahydro-isochinolin-6-yl)-amid | | | |
| **64** | 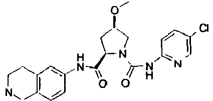 | | | |
| | (2R,4R)-4-Methoxy-pyrrolidin-1,2-dicarbonsäure-1-[(5-chlor-pyridin-2-yl)-amid]-2-(1,2,3,4-tetrahydro-isochinolin-6-yl)-amid | | | |
| **65** | 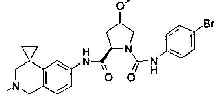 | | | |
| **66** | 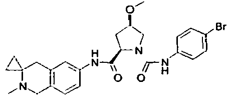 | | | |
| **67** | 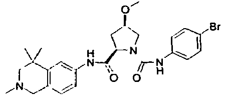 | | | |
| **68** | 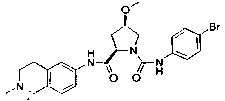 | | | |
| **69** | 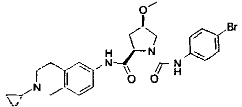 | | | |
| **70** | 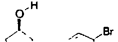 | | | |
| **71** | 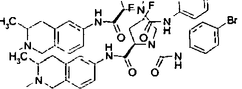 | | | |
| **72** | 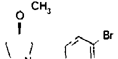 | | | |
| **73** | 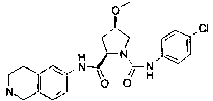 | | | |
| **74** | 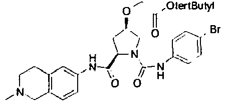 | | (M+H)⁺ = 587/589 (Bromisotope) | |
| **75** | 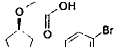 | | | |
| **76** | 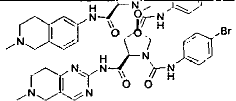 | | | |
| | | | | |

Die nachfolgenden Beispiele beschreiben die Herstellung pharmazeutischer Anwendungsformen, die als Wirkstoff eine beliebige Verbindung der allgemeinen Formel I enthalten.

### Beispiel A

### Trockenampulle mit 75 mg Wirkstoff pro 10 ml

| Zusammensetzung: | |
|---|---|
| Wirkstoff | 75.0 mg |
| Mannitol | 50.0 mg |
| Wasser für Injektionszwecke ad | 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel B

Trockenampulle mit 35 mg Wirkstoff pro 2 ml

| Zusammensetzung: | |
|---|---|
| Wirkstoff | 35.0 mg |
| Mannitol | 100.0 mg |
| Wasser für Injektionszwecke ad | 2.0 ml |

### Herstellung:

Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

### Beispiel C

**Tablette mit 50 mg Wirkstoff**

| Zusammensetzung: | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.

Durchmesser der Tabletten: 9 mm.

### Beispiel D

**Tablette mit 350 mg Wirkstoff**

| Zusammensetzung: | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.

Durchmesser der Tabletten: 12 mm.

### Beispiel E

### Kapseln mit 50 mg Wirkstoff

| Zusammensetzung: | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 3 abgefüllt.

### Beispiel F

### Kapseln mit 350 mg Wirkstoff

| Zusammensetzung: | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 0 abgefüllt.

### Beispiel G

### Suppositorien mit 100 mg Wirkstoff

| 1 Zäpfchen enthält: | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

### Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

1. Verbindungen der allgemeinen Formel (I) 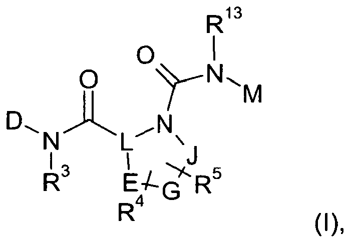 in denen
D ein substituiertes bicyclisches Ringsystem der Formel 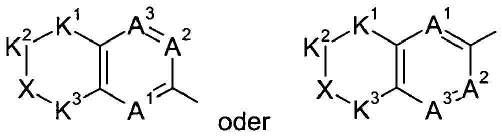 darstellt, in dem
K¹
eine Bindung, eine -CH₂-, -CHR^{7a}-, -CR^{7b}R^{7c}- oder -C(O)-Gruppe bedeutet, und wobei
R^{7a}/R^{7b}/R^{7c}
jeweils unabhängig voneinander ein Fluoratom, eine Hydroxy-, C₁₋₅-Alkyloxy-, Amino-, C₁₋₅-Alkylamino-, Di-(C₁₋₅-alkyl)-amino-, C₃₋₅-Cycloalkylenimino-, C₁₋₅-Alkylcarbonylaminogruppe,
eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C_{1- 5}-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkylgruppe bedeutet,
wobei nicht gleichzeitig beide Reste R^{7b}/R^{7c} über ein Heteroatom an das Ringkohlenstoffatom gebunden sein können, außer -C(R^{7b}R^{7c})- entspricht einer ―CF₂-Gruppe, oder
R^{7a} eine durch Fluor-, Chlor- , Brom-, Methyl-, Methoxy-, Amino-oder Nitro-substituierte Phenyl- oder monocyclische Heteroarylgruppe bedeutet, oder
zwei Reste R^{7b}/R^{7c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, 1,3-Dioxolan-, 1,4-Dioxan-, Hexahydropyridazin-, Piperazin-, Thiomorpholin-, Morpholin-, 2-Imidazolidinon-, 2-Oxazolidinon-, Tetrahydro-2(1H)-pyrimidinon-oder [1,3]Oxazinan-2-on-ring bilden können,
wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder
bei dem eine ―CH₂-Gruppe neben einem N-Atom durch eine ―CO-Gruppe ersetzt sein kann, und/oder dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid oder Sulfongruppe oxidiert sein kann,
K² und K³
jeweils unabhängig voneinander eine -CH₂-, -CHR^{8a}-, -CR^{8b}R^{8c}-oder eine -C(O)-Gruppe bedeuten, wobei
R^{8a}/R^{8b}/R^{8c}
jeweils unabhängig voneinander eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
oder zwei Reste R^{8b}/R^{8c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, Hexahydropyridazin-, Tetrahydro-2(1H)-pyrimidinon-, [1,3]Oxazinan-2-on-ring bilden können,
wobei dessen Methylengruppen durch 1-2 C₁₋₃-Alkyl- oder CF₃-gruppen substituiert sein können, und/oder dessen Methylengruppen, sofern sie nicht an ein Heteroatom gebunden sind, durch 1-2 Fluoratome substituiert sein können, und/oder bei dem eine ―CH₂-Gruppe neben einem Stickstoffatom durch eine ―CO-Gruppe ersetzt sein kann, und/oder dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid- oder Sulfongruppe oxidiert sein kann,
mit der Massgabe, dass ein durch R^{8b} oder R^{8c} eingebrachtes Heteroatom nicht durch nur ein Kohlenstoffatom von X in Formel (I) entfernt sein darf, und
insgesamt maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine CF₂-, Sulfen-, Sulfon-oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂-, C₃₋₆-Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkylsulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅-Alkyloxycarbonyl-, C₄₋₇-Cycloalkyleniminocarbonyl-gruppe bedeutet,
wobei die in den voranstehend genannten Gruppen befindlichen Methylen- und Methylgruppen zusätzlich durch eine C₁₋₃Alkyl-, Carboxy-, C₁₋₅ -Alkoxycarbonylgruppe substituiert sein können,
oder durch eine Hydroxy-, C₁₋₅-Alkyloxy-, Amino-, C₁₋₅-Alkylamino-, C₁₋₅-Dialkylamino- oder C₄₋₇-Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind,
und in dem
A¹ entweder N oder CR¹⁰ bedeutet,
A² entweder N oder CR¹¹ bedeutet,
A³ entweder N oder CR¹² bedeutet,
wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, C₂₋₅ -Alkenyl-, C₂₋₅-Alkinyl-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten, und -L-E-G-J- eine ―C-C-C-C- oder ―C-C=C-C-Gruppe bedeutet, die durch R⁴
und R⁵ substituiert sein kann, und
R³ ein Wasserstoffatom oder eine C₁₋₃-Alkylgruppe bedeutet, und
R⁴ ein Wasserstoffatom oder
eine geradkettige oder verzweigte C₁₋₆-Alkyl-, C₂₋₆-Alkenyl- oder C₂₋₆-Alkinylgruppe bedeutet,
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls ganz oder teilweise durch Fluoratome ersetzt sein können, und/oder wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls jeweils unabhängig voneinander durch ein bis zwei Substituenten ausgewählt aus einer C₃₋₅-Cycloalkylgruppe, einer Nitril-, Hydroxy-oder C₁₋₅-Alkyloxygruppe, wobei die Wasserstoffatome der C₁₋₅-Alkyloxygruppe gegebenenfalls ganz oder teilweise durch Fluoratome ersetzt sein können, einer Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Aminosulfonyl-, C₁₋₅-Alkylaminosulfonyl-, Di-(C₁₋₅-alkyl)-aminosulfonyl-, C₄₋₇-Cycloalkyleniminosulfonyl-, Amino-, C₁₋₅-Alkylamino-, Di-(C₁₋₅-alkyl)-amino-, C₁₋₅-Alkylcarbonylamino-, C₁₋₅-Alkylsulfonylamino-, *N*-(C₁₋₅-Alkylsulfonyl)-C₁₋₅-alkylamino-, C₃₋₆-Cycloalkylcarbonylaminogruppe, oder einer Morpholinyl-, Thiomorpholinyl-, Pyrrolidinyl-, Piperidinyl-, Piperazinyl-, Tetrahydrofuranyl-, Tetrahydropyranyl-gruppe substituiert sein können, wobei die vorgenannten Carbo- und Hetero-cyclen im Ring jeweils durch 1-4 C₁₋₃-Alkyl- oder C₁₋₃-Alkylcarbonylgruppen oder jeweils durch 1-2 Oxogruppen substituiert sein können, und/oder
wobei die Wasserstoffatome der sp²-hybridisierten Kohlenstoffatome der geradkettigen oder verzweigten C₂₋₆-Alkenyl-gruppe gegebenenfalls ganz oder teilweise durch Fluoratome ersetzt sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, oder
eine Phenyl-, mono- oder bicyclische Heteroaryl-, Phenyl-C₁₋₅-alkyl- oder mono- oder bicyclische Heteroaryl-C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Fluor-, Chlor-, Brom-und Iodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein kann,
bedeutet, und
wenn -L-E-G-J- eine ―C-C-C-C-Gruppe bedeutet, kann R⁴ an E oder G auch ein Fluoratom oder eine Hydroxy-, Methoxy-, C₃₋₅-Alkenyl-oxy-, C₃₋₅-Alkinyl-oxy-, C₂₋₅-Alkyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₃-alkyloxy-, Heteroaryl-C₀₋₃-alkyloxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen,
wobei die in den vorgenannten Alkyl- oder Cycloalkylresten vorhandenen Methyl oder Methylengruppen jeweils unabhängig voneinander durch einen Substituenten ausgewählt aus der Gruppe Morpholinyl-, Thiomorpholinyl-, Pyrrolidinyl-, Piperidinyl-, Piperazinyl-, Tetrahydrofuranyl-, Tetrahydropyranyl, Dimethylaminocarbonyl-, C₁₋₅Alkyloxycarbonyl-, Carboxy-, Methyl-, Hydroxy-, Methoxy- oder Amino- substituiert sein können,
und
die vorgenannten Phenyl- oder Heteroarylreste gegebenenfalls ein- bis dreifach durch gleiche oder unterschiedliche Substituenten ausgewählt aus der Gruppe bestehend aus Fluor-, Chlor-, Brom-und Iodatomen, und C₁₋₅-Alkyl-, Trifluormethyl-, Amino-, C₁₋₅-alkylamino-, Di-(C₁₋₅-alkyl)-amino-, Hydroxy-, C₁₋₅-Alkyloxy-, Mono-, Di-oder Trifluormethoxy-, Carboxy- und C₁₋₅-Alkyloxycarbonylgruppe, substituiert sein können,
mit der Maßgabe,
dass zwei Heteroatome aus der Gruppe Sauerstoff und Stickstoff durch genau eine gegebenenfalls substituierte -CH₂-Gruppe voneinander getrennt sind, und/oder
dass zwei Atome eine ―O-O- oder ―S-O-Bindung bilden,
ausgeschlossen ist, und
R⁵ ein Wasserstoffatom, eine C₁₋₅ Alkyl-, C₂₋₅ Alkenyl- oder C₂₋₅ Alkinyl-oder eine Phenyl-C₀₋₅ Alkylgruppe bedeutet, wobei die Alkylgruppe durch eine Hydroxy-, Methoxy-, Hydroxycarbonyl- oder C₁₋₅Alkoxycarbonylgruppe substituiert sein kann,
oder sofern R⁵ mit E oder G verknüpft ist auch eine Hydroxy- oder Methoxy-gruppe darstellen kann, oder
R⁴ und R⁵ sofern sie an das selbe Kohlenstoffatom gebunden sind,
zusammen mit dem Kohlenstoffatom eine -C=O-Gruppe, oder eine - CF₂- Gruppe bilden können, oder
R⁴ und R⁵ sofern sie an das selbe Kohlenstoffatom oder an zwei
benachbarte Kohlenstoffatome gebunden sind, zusammen mit dem oder den Kohlenstoffatomen einen 3-7-gliedrigen Carbocyclus oder einen einfach ungesättigten 5-7 gliedrigen Carbocyclus bilden können,
wobei eines der Kohlenstoffkettenglieder dieses Cyclus durch ein Sauerstoff- oder Schwefelatom oder eine -NH-, -N(C₁₋₅-Alkyl)-, - N(C₁₋₄-Alkylcarbonyl)- oder eine Carbonyl-, Sulfinyl- oder Sulfonylgruppe ersetzt sein kann, und/oder
wobei zwei direkt benachbarte Kohlenstoffkettenglieder dieser C₄₋₇-Carbocyclen zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei vier direkt benachbarte Kohlenstoffkettenglieder dieser C₅₋₇-Carbocyclen zusammen durch eine -O-CH₂-CH₂-O-Gruppe ersetzt sein können, und/oder
wobei 1 bis 3 Kohlenstoffatome dieser 3-7-gliedrigen Cyclen gegebenenfalls unabhängig voneinander durch jeweils ein oder zwei Fluoratome oder eine oder zwei C₁₋₅-Alkyl-gruppen oder eine Hydroxy-, Formyloxy-, C₁₋₅-Alkyloxy-, C₁₋₅-Alkylcarbonyloxy-, Amino-, C₁₋₅-Alkylamino-, Di-(C₁₋₅-alkyl)-amino-, C₄₋₇-Cycloalkylenimino-, C₁₋₅-Alkylcarbonylamino-, C₃₋₆-Cycloalkylcarbonylamino-, Nitril-, Carboxy-C₁₋₅-alkyl-, C₁₋₅-Alkyloxycarbonyl-C₁₋₅-alkyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder C₄₋₇-Cycloalkyleniminocarbonylgruppe substituiert sein können,
mit der Maßgabe, dass eine solcher, zusammen aus R⁴ und R⁵ gebildeter Cyclus,
in dem zwei Stickstoffatome oder ein Stickstoff und ein Sauerstoffatom im Cyclus durch genau eine gegebenenfalls substituierte -CH₂-Gruppe voneinander getrennt sind, und/oder in der zwei Atome im Ring eine ―O-O- oder ―S-O-Bindung bilden,
ausgeschlossen ist,
oder das Fragment 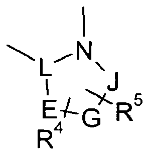 die Gruppe 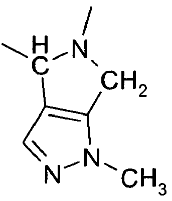 bedeutet, R¹³ ein Wasserstoffatom oder eine C₁₋₅ Alkylgruppe bedeutet,
M einen gegebenenfalls durch R² und R⁶ substituierten Phenyl-, Thienyl-oder Pyridylring bedeutet, in dem
R² ein Fluor-, Chlor-, Brom- oder Jod-atom oder eine Methyl-, Ethyl-, Vinyl-, Methoxy-, Ethinyl-, Cyano- oder ―C(O)NH₂-Gruppe darstellt, und
R⁶ ein Wasserstoff-, Fluor-, Chlor-, Brom- oder Jod-atom oder eine Hydroxy-, Methoxy-, Trifluormethoxy-, eine gegebenenfalls durch Fluoratome substituierte C₁₋₃-Alkyl-, Cyano-, Amino-, oder NH₂C(O)-Gruppe darstellt,
wobei, soweit nichts anderes erwähnt wurde, unter dem voranstehend in den Definitionen erwähnten Ausdruck "Heteroarylgruppe" eine monocyclische 5-oder 6-gliedrige Heteroarylgruppe zu verstehen ist, wobei
die 6-gliedrige Heteroarylgruppe ein, zwei oder drei Stickstoffatome, und die 5-gliedrige Heteroarylgruppe eine gegebenenfalls durch eine C₁₋₃-Alkylgruppe substituierte Iminogruppe, ein Sauerstoff- oder Schwefelatom, oder
eine gegebenenfalls durch eine C₁₋₃-Alkylgruppe substituierte Iminogruppe oder ein Sauerstoff- oder Schwefelatom und zusätzlich ein oder zwei Stickstoffatome, oder
eine gegebenenfalls durch eine C₁₋₃-Alkylgruppe substituierte Iminogruppe und drei Stickstoffatome,
enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann,
und die Bindung über ein Stickstoffatom oder über ein Kohlenstoffatom des heterocyclischen Teils oder eines ankondensierten Phenylrings erfolgt,
und wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
und wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

2. Verbindungen der allgemeinen Formel (I) nach Anspruch 1, in denen
D ein substituiertes bicyclisches Ringsystem der Formel 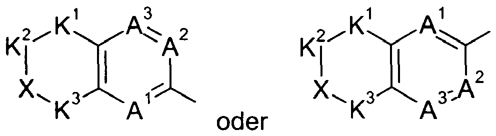 darstellt, in dem
K¹
eine -CH₂-, -CHR^{7a}-, -CR^{7b}R^{7c}- oder eine -C(O)-Gruppe bedeutet, und wobei
R^{7a}/R^{7b}/R^{7c} jeweils unabhängig voneinander ein Fluoratom, eine Hydroxy-, C₁₋₅-Alkyloxy-, eine C₁₋₅-Alkylgruppe bedeutet,
wobei nicht gleichzeitig beide Reste R^{7b}/R^{7c} über ein Heteroatom an das Ringkohlenstoffatom gebunden sein können, außer - C(R^{7b}R^{7c})- entspricht einer ―CF₂-Gruppe, oder
zwei Reste R^{7b}/R^{7c} zusammen mit dem Ringkohlenstoffatom einen 3-gliedrigen Carbocyclus bilden können und
K² und K³
jeweils unabhängig voneinander eine -CH₂-, -CHR^{8a}-, -CR^{8b}R^{8c}-oder eine -C(O)- Gruppe bedeuten, wobei
R^{8a}/R^{8b}/R^{8c}
jeweils unabhängig voneinander eine C₁₋₅-Alkylgruppe bedeutet, und/oder
zwei Reste R^{8b}/R^{8c} zusammen mit dem Ringkohlenstoffatom einen 3-gliedrigen gesättigten Carbocyclus bilden können
und
insgesamt maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine Sulfen-, Sulfon-, -CF₂-oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂- oder eine C₃₋₆-Cycloalkylgruppe bedeutet,
und in dem
A¹ entweder N oder CR¹⁰ bedeutet,
A² entweder N oder CR¹¹ bedeutet,
A³ entweder N oder CR¹² bedeutet,
wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten,
deren Tautomere, deren Enantiomere, deren Diastereomere, deren Gemische und deren Salze.

3. Verbindungen der allgemeinen Formel (I) nach Anspruch 1 oder 2, in denen
X eine NR¹-Gruppe bedeutet, in der
R¹ ein Wasserstoffatom oder eine C₁₋₅-Alkyl-, Allyl- oder Cyclopropylgruppe bedeutet, und
A¹ CR¹⁰ bedeutet,
A² CR¹¹ bedeutet,
A³ entweder N oder CR¹² bedeutet,
wobei R¹⁰, R¹¹ und R¹² jeweils unabhängig voneinander ein Wasserstoff-, Fluor- oder Chloratom, oder eine Methyl-, CF₃-, Cyano-, Carboxy-, C₁₋₅-Alkyloxycarbonyl-, Hydroxy-, Methoxy-, CF₃O-, CHF₂O-, CH₂FO-Gruppe bedeuten,
deren Tautomere, deren Enantiomere, deren Diastereomere, deren Gemische und deren Salze.

4. Verbindungen der allgemeinen Formel (I) nach einem der Ansprüche 1, 2 oder 3, in denen
R⁴ ein Wasserstoffatom oder
eine geradkettige oder verzweigte C₁₋₆-Alkylgruppe bedeutet, wobei die Wasserstoffatome der Methylen- und/oder Methylfragmente der geradkettigen oder verzweigten C₁₋₆-Alkylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkylgruppe gegebenenfalls jeweils unabhängig voneinander durch einen Substituenten ausgewählt aus einer Hydroxy-, C₁₋₅-Alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Amino-, C₁₋₅-Alkylamino-, Di-(C₁₋₅-alkyl)-amino-, C₁₋₅-Alkylcarbonylamino-, C₁₋₅-Alkylsulfonylamino-, *N*-(C₁₋₅-Alkylsulfonyl)-C₁₋₅-alkylamino-, C₃₋₆-Cycloalkylcarbonylaminogruppe substituiert sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, bedeutet, und
wenn -L-E-G-J- eine -C-C-C-C-Gruppe bedeutet, kann R⁴ an E oder G auch ein Fluoratom oder eine Hydroxy-, Methoxy-, C₃₋₅-Alkenyl-oxy-, C₃₋₅-Alkinyl-oxy-, C₂₋₅-Alkyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₂-alkyloxygruppe darstellen, die im Phenylring durch 1-2 Fluoratome oder Methoxygruppen substituiert sein kann, eine Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen,
wobei die in den vorgenannten Alkyl- oder Cycloalkylresten vorhandenen Methyl oder Methylengruppen jeweils unabhängig voneinander durch einen Substituenten ausgewählt aus der Gruppe Dimethylaminocarbonyl-, C₁₋₅Alkyloxycarbonyl-, Carboxy-, Methyl-, Hydroxy-, Methoxy- oder Amino- substituiert sein können,
mit der Maßgabe,
dass zwei Heteroatome aus der Gruppe Sauerstoff und Stickstoff durch genau eine gegebenenfalls substituierte -CH₂-Gruppe voneinander getrennt sind, und/oder
dass zwei Atome eine ―O-O- oder ―S-O-Bindung bilden,
ausgeschlossen ist, und
R⁵ ein Wasserstoffatom oder eine C₁₋₅Alkyl-, Allyl-, Propargyl- oder
Benzylgruppe bedeutet, oder sofern R⁵ mit E oder G verknüpft ist, auch eine Hydroxy- oder Methoxy-gruppe darstellen kann, oder
R⁴ und R⁵ sofern sie an das selbe Kohlenstoffatom gebunden sind, zusammen mit dem Kohlenstoffatom eine -C=O-Gruppe, oder eine - CF₂- Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-7-gliedrigen Carbocyclus bilden können,
wobei eines der Kohlenstoffkettenglieder dieses Cyclus durch ein Sauerstoff- oder Schwefelatom oder eine -NH-, -N(C₁₋₅-Alkyl)-, - N(C₁₋₄-Alkylcarbonyl)- oder eine Carbonyl-, Sulfinyl- oder Sulfonylgruppe ersetzt sein kann, und/oder
wobei zwei direkt benachbarte Kohlenstoffkettenglieder dieser C₄₋₇-Carbocyclen zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei vier direkt benachbarte Kohlenstoffkettenglieder dieser C₅₋₇-Carbocyclen zusammen durch eine -O-CH₂-CH₂O-Gruppe ersetzt sein können,
mit der Maßgabe, dass ein solcher, zusammen aus R⁴ und R⁵ gebildeter Cyclus,
in dem zwei Stickstoffatome oder ein Stickstoff und ein Sauerstoffatom im Cyclus durch genau eine gegebenenfalls substituierte -CH₂-Gruppe voneinander getrennt sind, und/oder
in der zwei Atome im Ring eine ―O-O- oder ―S-O-Bindung bilden,
ausgeschlossen ist,
deren Tautomere, deren Enantiomere, deren Diastereomere, deren Gemische und deren Salze.

5. Verbindungen der allgemeinen Formel (I) nach einem der Ansprüche 1, 2, 3 oder 4, in denen
-L-E-G-J- eine ―C-C-C-C-Gruppe bedeutet, die durch R⁴ und R⁵, die wie oben in den Ansprüchen 1, 2, 3 oder 4 definiert sind, substituiert sein kann,
deren Tautomere, deren Enantiomere, deren Diastereomere, deren Gemische und deren Salze.

6. Verbindungen der allgemeinen Formel (I) nach einem der Ansprüche 1, 2, 3, 4 oder 5, in denen
D ein substituiertes bicyclisches Ringsystem der allgemeinen Formel 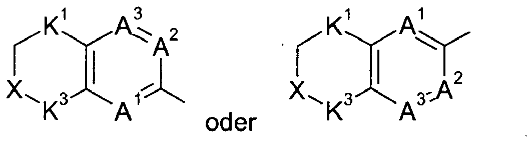 darstellt, in dem
K¹ eine -CH₂-, -CHR^{7a}-, -CR^{7b}R^{7c}- oder eine -C(O)-Gruppe bedeutet, wobei
R^{7a} eine C₁₋₂-Alkylgruppe bedeutet und
R^{7b}/R^{7c} jeweils unabhängig voneinander eine Hydroxy-, Methoxy-oder eine C₁₋₃-Alkylgruppe bedeutet, wobei nicht gleichzeitig beide Reste R^{7b}/R^{7c} über ein Sauerstoffatom an das Ringkohlenstoffatom gebunden sein können, oder
zwei Reste R^{7b}/R^{7c} zusammen mit dem Ringkohlenstoffatom einen 3-gliedrigen Carbocyclus bilden können,
und
K² und K³ jeweils unabhängig voneinenader
eine -CH₂-, -CHR^{8a}- oder eine -CR^{8b}R^{8c}-Gruppe bedeuten, wobei R^{8a}/R^{8b}/R^{8c}
jeweils unabhängig voneinander eine C₁₋₃-Alkylgruppe bedeutet, und/oder
zwei Reste R^{8b}/R^{8c} zusammen mit dem Ringkohlenstoffatom einen 3-gliedrigen gesättigten Carbocyclus bilden können
und
insgesamt maximal vier Reste ausgewählt aus R^{7a}, R^{7b}, R^{7c}, R^{8a}, R^{8b} und R^{8c} vorhanden sein dürfen,
und
X eine NR¹-Gruppe bedeutet, in der
R¹ ein Wasserstoffatom oder eine C₁₋₃-Alkyl-, Allyl- oder Cyclopropylgruppe bedeutet, und
A¹ CR¹⁰ bedeutet,
A² CR¹¹ bedeutet,
A³ CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten, und
-L-E-G-J- eine ―C-C-C-C-Gruppe bedeutet, die durch R⁴ und R⁵ substituiert
sein kann, und
R³ ein Wasserstoffatom bedeutet, und
R⁴ ein Wasserstoffatom oder
eine geradkettige oder verzweigte C₁₋₃-Alkylgruppe bedeutet,
wobei die Wasserstoffatome der Methylen- und/oder Methylfragmente der geradkettigen oder verzweigten C₁₋₆-Alkylgruppe gegebenenfalls unabhängig voneinander durch einen Substituenten ausgewählt aus einer Hydroxy-, C₁₋₅-Alkyloxy-, Carboxy-, C₁₋₅-Alkyloxycarbonyl-gruppe substituiert sein können, oder
wenn R⁴ an E oder G angebunden ist auch ein Fluoratom oder eine Hydroxy-, Methoxy-, C₃₋₅-Alkenyl-oxy-, C₂₋₅-Alkyl-oxy-, C₃₋₆-Cycloalkyloxy-, C₁₋₅-Alkylaminocarbonyloxy-, Di(C₁₋₅-alkyl)aminocarbonyloxy- oder C₄₋₇-Cycloalkyleniminocarbonyloxy-gruppe darstellen kann,
mit der Maßgabe,
dass zwei Heteroatome aus der Gruppe Sauerstoff und Stickstoff durch genau eine gegebenenfalls substituierte -CH₂-Gruppe voneinander getrennt sind,
ausgeschlossen ist, und
R⁵ ein Wasserstoffatom oder eine C₁₋₅Alkyl-, Allyl- oder Benzylgruppe bedeutet, oder sofern R⁵ mit E oder G verknüpft ist auch eine Hydroxy-oder Methoxy-gruppe darstellen kann, oder
R⁴ und R⁵ sofern sie an das selbe Kohlenstoffatom gebunden sind, zusammen mit dem Kohlenstoffatom eine -C=O-Gruppe, oder eine - CF₂- Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-6-gliedrigen Carbocyclus bilden können,
wobei vier direkt benachbarte Kohlenstoffkettenglieder dieser C₅₋₆-Carbocyclen zusammen durch eine -O-CH₂-CH₂O-Gruppe ersetzt sein können,
und
R¹³ ein Wasserstoffatom bedeutet,
M einen durch R² in 4-Position substituierten Phenyl- oder durch R² in 5-Position substituierten Pyridylring bedeutet, in dem
R² ein Fluor-, Chlor-, Bromatom, eine Methoxy- oder Ethinyl-Gruppe darstellt, und
R⁶ ein Wasserstoff- oder Fluoratom darstellt,
deren Tautomere, deren Enantiomere, deren Diastereomere, deren Gemische und deren Salze.

7. Verbindungen der allgemeinen Formel (I) nach einem der Ansprüche 1, 2, 3, 4, 5 oder 6, in denen der zentrale Ring
entweder 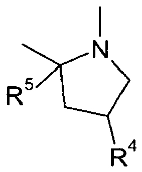 oder 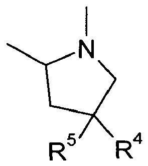 bedeutet,
deren Tautomere, deren Enantiomere, deren Diastereomere, deren Gemische und deren Salze.

8. Verbindungen der allgemeinen Formel (I) nach einem der Ansprüche 1, 2, 3, 4, 5, 6 oder 7, in denen
D ein substituiertes bicyclisches Ringsystem der allgemeinen Formel 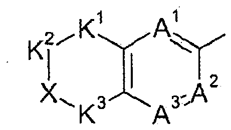 darstellt,
deren Tautomere, deren Enantiomere, deren Diastereomere, deren Gemische und deren Salze.

9. Verbindungen der allgemeinen Formel (I) nach einem der Ansprüche 1, 2, 3, 4, 5, 6, 7 oder 8, die an den Kettengliedern G und L des 5-gliedrigen zentralen Rings R- konfiguriert sind,
deren Tautomere, deren Enantiomere, deren Diastereomere, deren Gemische und deren Salze.

10. Verbindung gemäß Anspruch 1 ausgewählt aus der Gruppe bestehend aus: 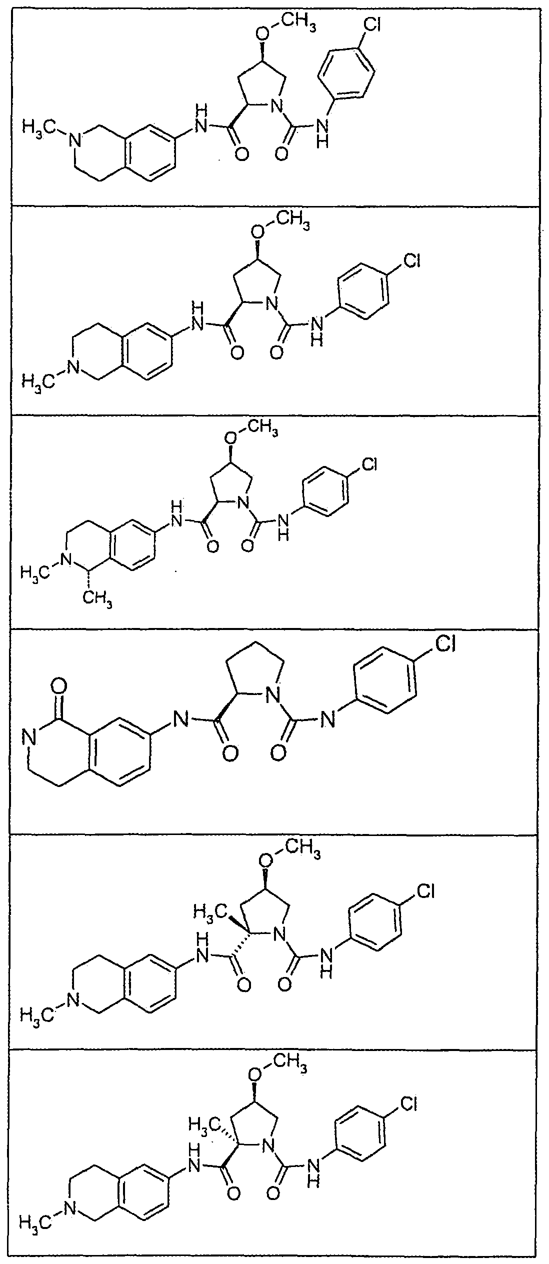 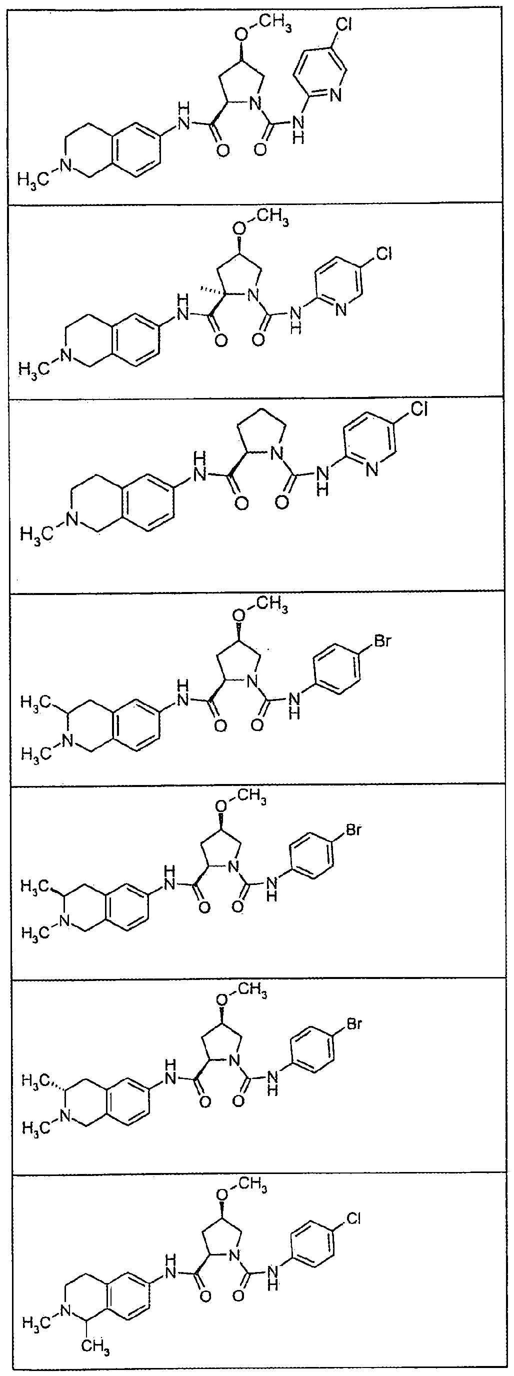 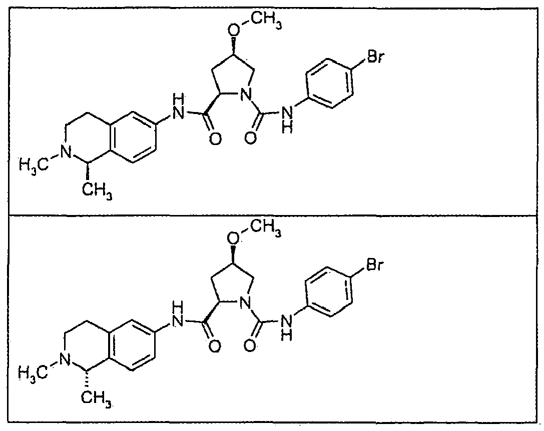 deren Tautomere, deren Enantiomere, deren Diastereomere, deren Gemische und deren Salze.

11. Verbindung gemäß Anspruch 1 ausgewählt aus der Gruppe bestehend aus: 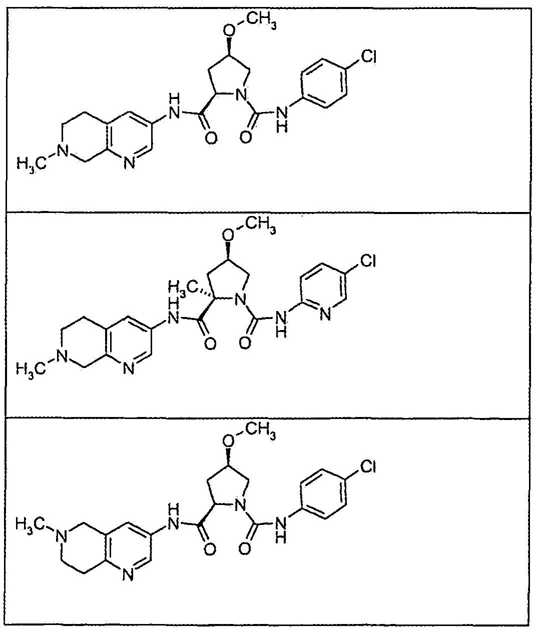 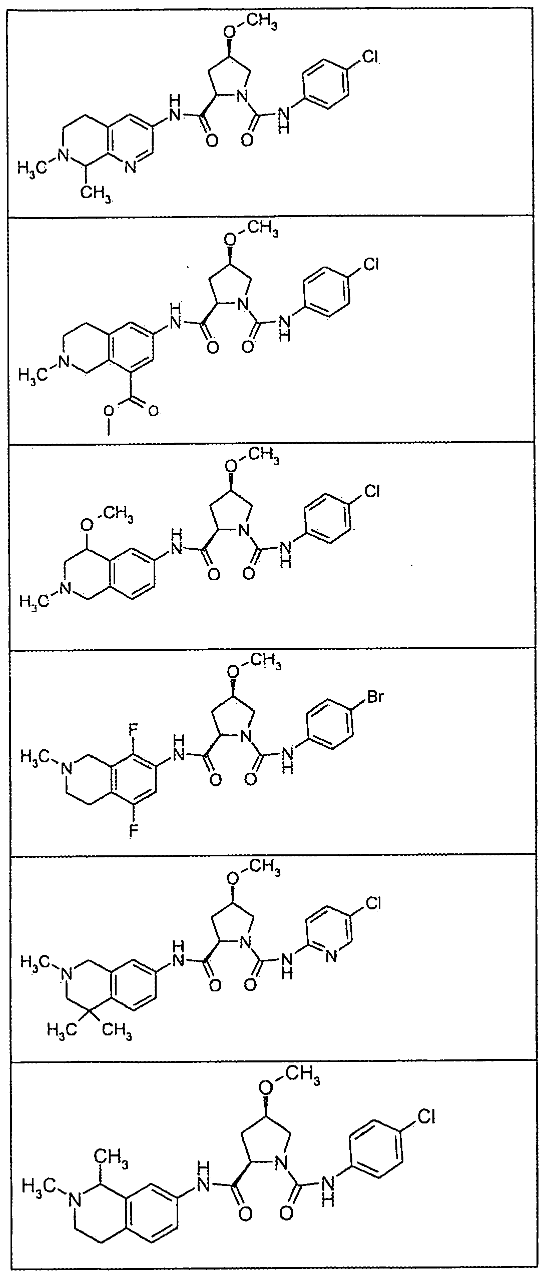 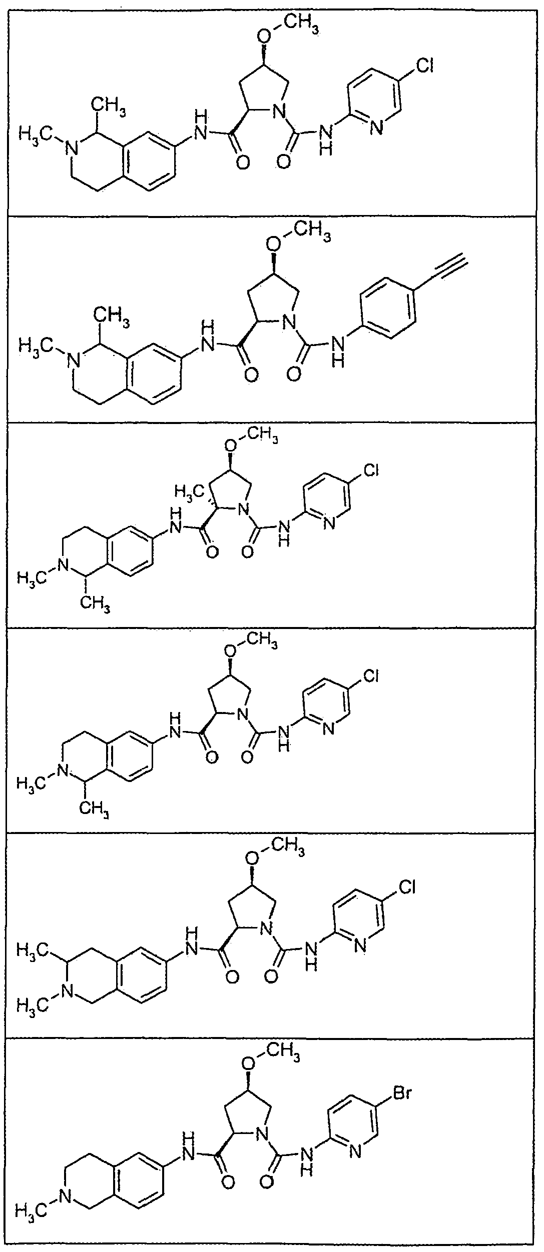 deren Tautomere, deren Enantiomere, deren Diastereomere, deren Gemische und deren Salze.

12. Verbindung gemäß Anspruch 1 ausgewählt aus der Gruppe bestehend aus: 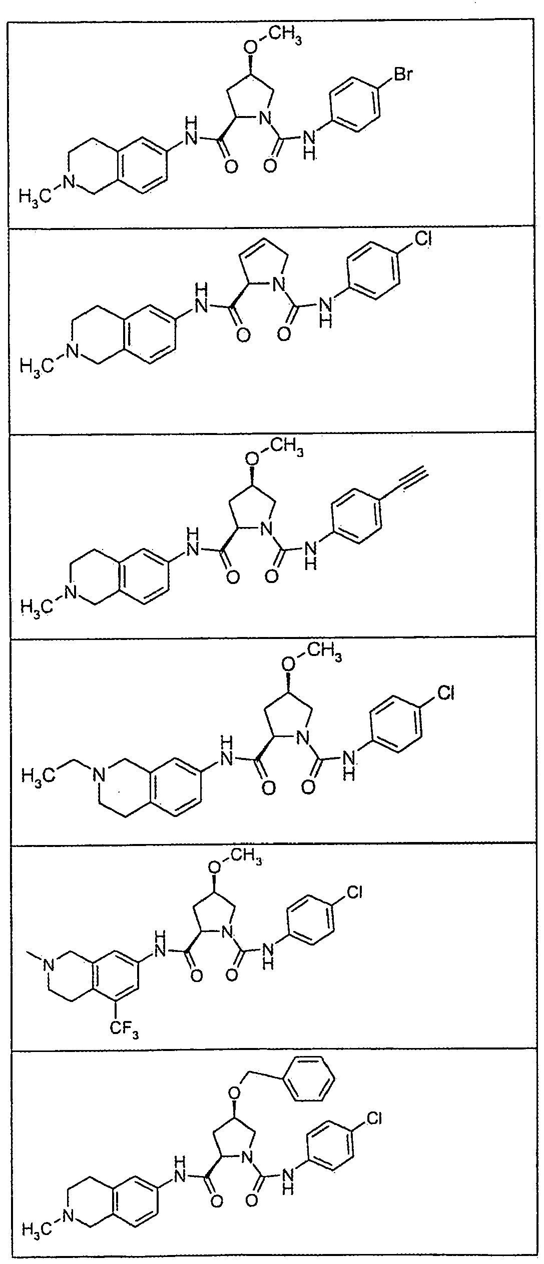 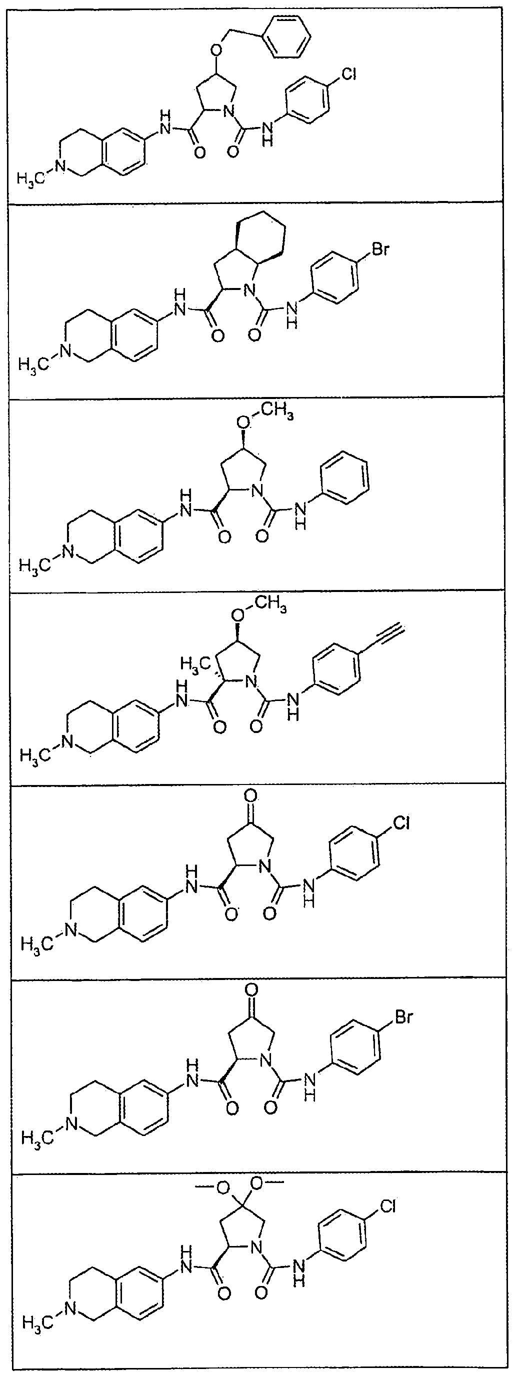 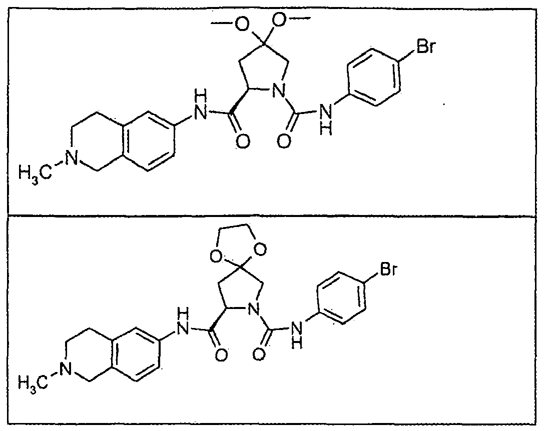 deren Tautomere, deren Enantiomere, deren Diastereomere, deren Gemische und deren Salze.

13. Verbindung gemäß Anspruch 1 ausgewählt aus der Gruppe bestehend aus: 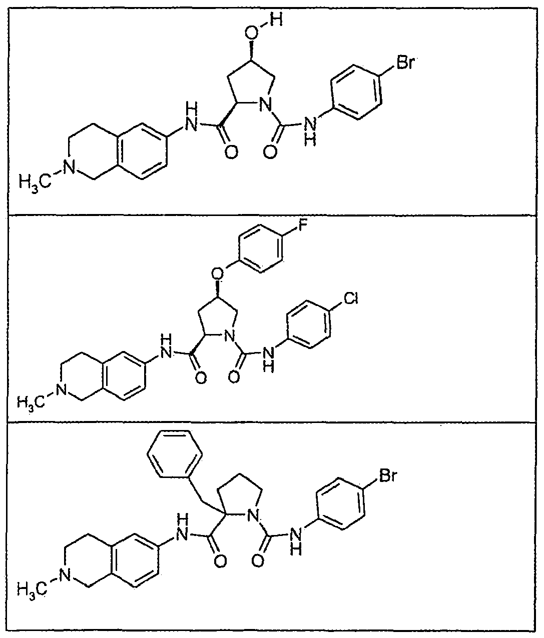 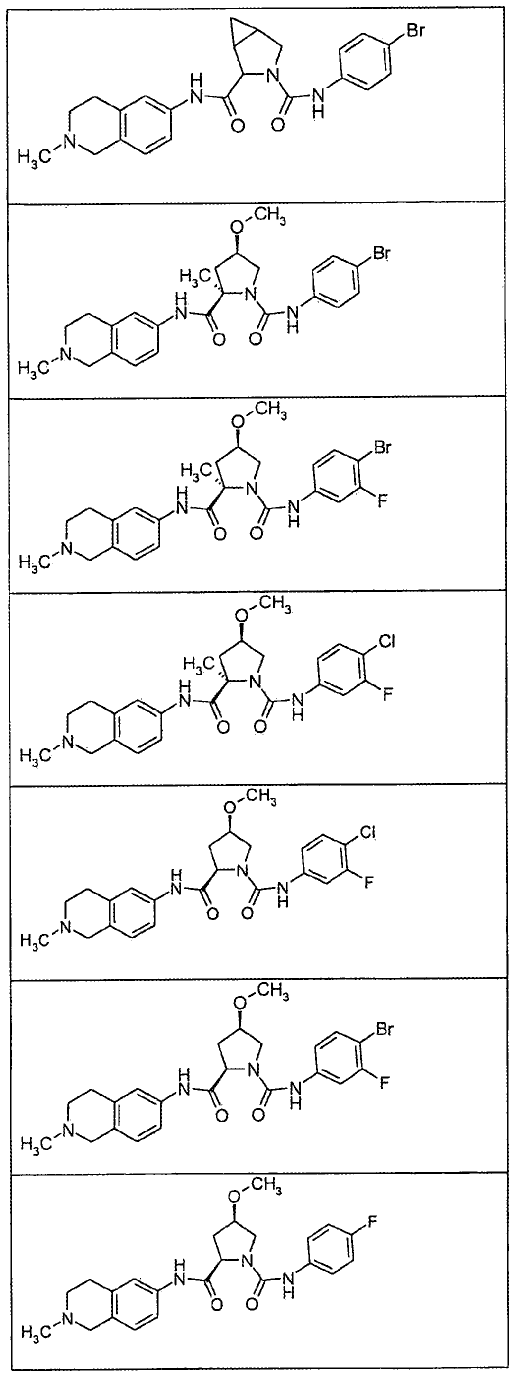 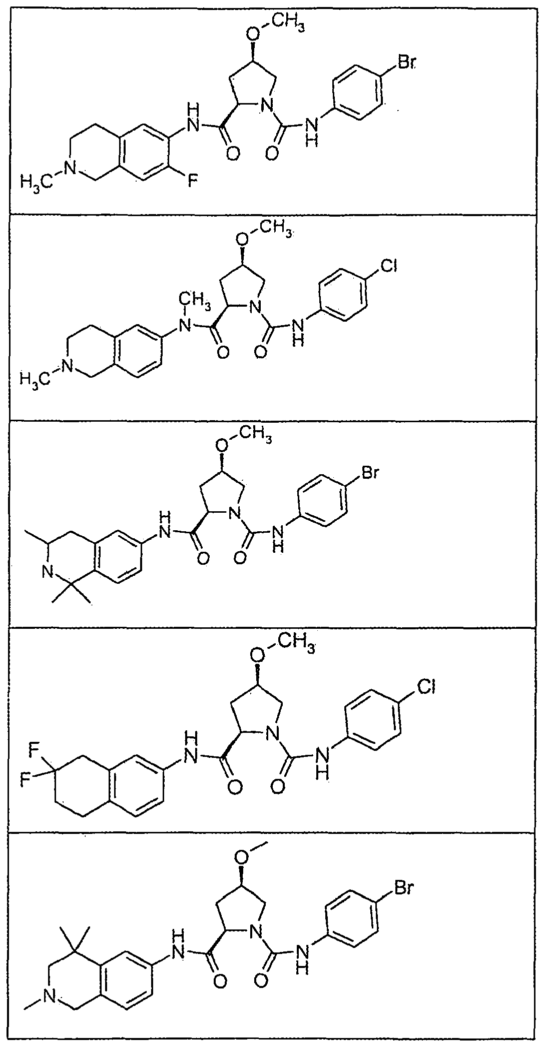 deren Tautomere, deren Enantiomere, deren Diastereomere, deren Gemische und deren Salze.

14. Verbindung gemäß Anspruch 1 ausgewählt aus der Gruppe bestehend aus: 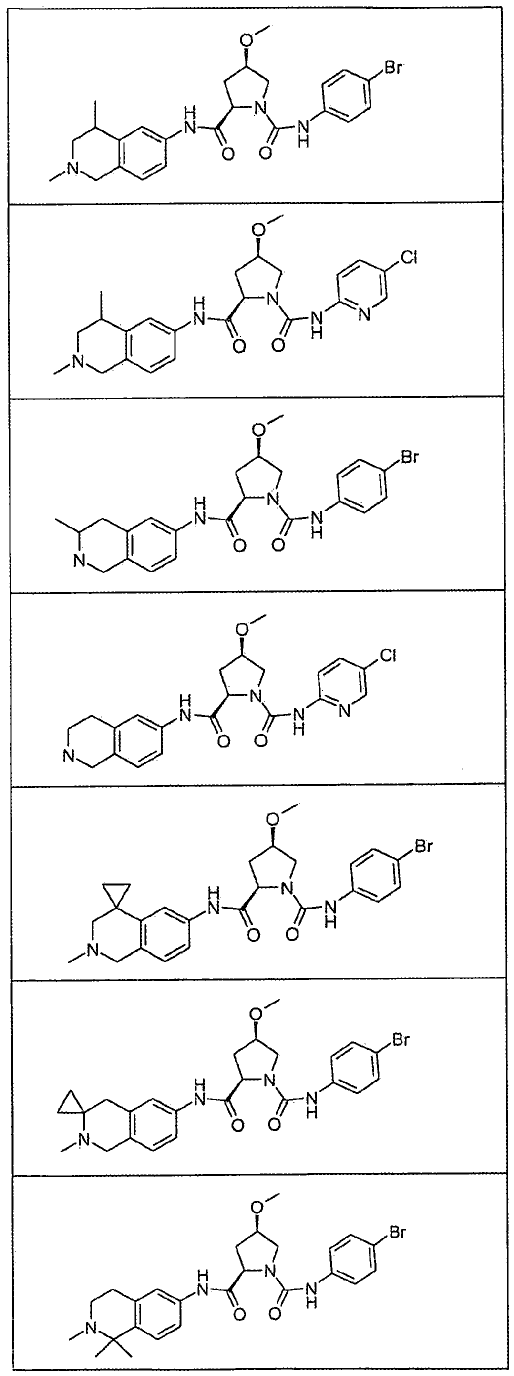 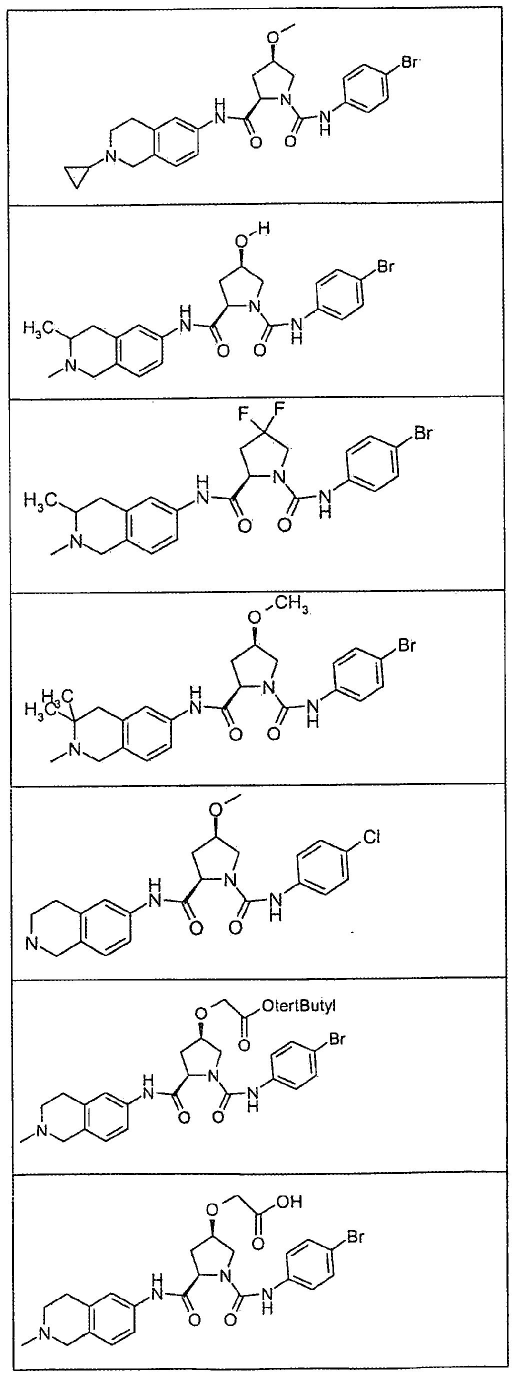 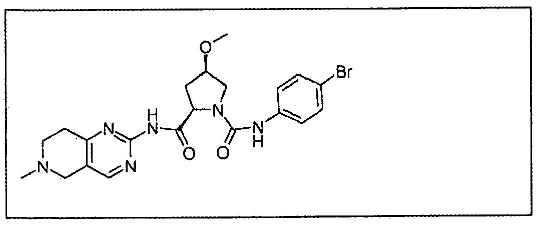 deren Tautomere, deren Enantiomere, deren Diastereomere, deren Gemische und deren Salze.

15. Physiologisch verträgliche Salze der Verbindungen gemäß einem der Ansprüche 1 bis 14.

16. Arzneimittel, enthaltend eine Verbindung nach mindestens einem der Ansprüche 1 bis 14 oder ein physiologisch verträgliches Salz gemäß Anspruch 15. neben gegebenenfalls einem oder mehreren inerten Trägerstoffen und/oder Verdünnungsmitteln.

17. Verbindung nach mindestens einem der Ansprüche 1
bis 14 oder eines physiologisch verträglichen Salzes gemäß Anspruch 15 zur Verwendung als Arzneimittel.

18. Verfahren zur Herstellung eines Arzneimittels gemäß Anspruch 16, **dadurch gekennzeichnet, dass** auf nichtchemischem Wege eine Verbindung nach mindestens einem der Ansprüche 1 bis 14 oder ein physiologisch verträgliches Salz gemäß Anspruch 15 in einen oder mehrere inerte Trägerstoffe und/oder Verdünnungsmittel eingearbeitet wird.

## Claims

1. Compounds of general formula (I) 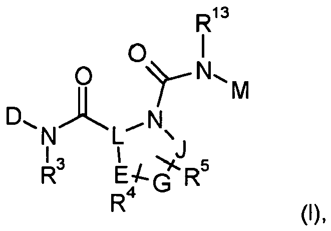 wherein
D denotes a substituted bicyclic ring system of formula 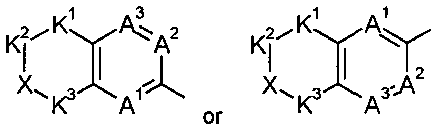 wherein
K¹
denotes a bond, a -CH₂-, -CHR^{7a}-, -CR^{7b}R^{7c}- or -C(O)- group, and wherein
R^{7a}/R^{7b}/R^{7c}
each independently of one another denote a fluorine atom, a hydroxy, C₁₋₅-alkyloxy, amino, C₁₋₅-alkylamino, di-(C₁₋₅-alkyl)-amino, C₃₋₅-cycloalkyleneimino, C₁₋₅-alkylcarbonylamino group,
a C₁₋₅-alkyl group which may be substituted by 1-3 fluorine atoms, a hydroxy-C₁₋₅-alkyl, C₁₋₅-alkyloxy-C₁₋₅-alkyl, amino-C₁₋₅-alkyl, C₁₋₅-alkylamino-C₁₋₅-alkyl, di-(C₁₋₅-alkyl)-amino-C₁₋₅-alkyl, C₄₋₇-cycloalkyleneimino-C₁₋₅-alkyl, carboxy-C₀₋₅-alkyl, C₁₋₅-alkyloxycarbonyl-C₀₋₅-alkyl, aminocarbonyl-C₀₋₅-alkyl, C₁₋₅-alkylaminocarbonyl-C₀₋₅-alkyl, di-(C₁₋₅-alkyl)-aminocarbonyl-C₀₋₅-alkyl or a C₄₋₇-cycloalkyleneiminocarbonyl-C₀₋₅-alkyl group,
while the two groups R^{7b}/R^{7c} may not simultaneously be bound to the cyclic carbon atom via a heteroatom, except where -C(R^{7b}R^{7c})- corresponds to a ―CF₂ group, or
R^{7a} denotes a fluoro-, chloro- , bromo-, methyl-, methoxy-, amino- or nitro-substituted phenyl or monocyclic heteroaryl group, or
two groups R^{7b}/R^{7c} together with the cyclic carbon atom may form a 3-, 4-, 5-, 6- or 7-membered saturated carbocyclic group or a cyclopentene, cyclohexene, oxetan, azetidine, thietan, tetrahydrofuran, pyrrolidine, tetrahydrothiophene, tetrahydropyran, piperidine, pentamethylene sulphide, hexamethyleneimine, 1,3-dioxolan, 1,4-dioxane, hexahydropyridazine, piperazine, thiomorpholine, morpholine, 2-imidazolidinone, 2-oxazolidinone, tetrahydro-2(1H)-pyrimidinone or [1.3]oxazinan-2-one ring,
while the methylene groups thereof may be substituted by 1-2 C₁₋₃-alkyl or CF₃- groups, and/or
the methylene groups thereof, if they are not bound to a heteroatom, may be substituted by 1-2 fluorine atoms, and/or wherein a -CH₂ group besides an N atom may be replaced by a -CO group, and/or
the imino groups of which may each be substituted by a C₁₋₃-alkyl or C₁₋₃-alkylcarbonyl group, and/or
wherein the sulphur atom may be oxidised to form a sulphoxide or sulphone group,
K² and K³
each independently of one another denote a -CH₂, -CHR^{8a}, -CR^{8b}R^{8c} or a -C(O) group , wherein
R^{8a}/R^{8b}/R^{8c}
each independently of one another denote a C₁₋₅-alkyl group which may be substituted by 1-3 fluorine atoms, a hydroxy-C₁₋₅-alkyl, C₁₋₅-alkyloxy-C₁₋₅-alkyl, amino-C₁₋₅-alkyl, C₁₋₅-alkylamino-C₁₋₅-alkyl, di-(C₁₋₅-alkyl)-amino-C₁₋₅-alkyl, C₄₋₇-cycloalkyleneimino-C₁₋₅-alkyl, carboxy-C₀₋₅-alkyl, C₁₋₅-alkyloxycarbonyl-C₀₋₅-alkyl, aminocarbonyl-C₀₋₅-alkyl, C₁₋₅-alkylaminocarbonyl-C₀₋₅-alkyl, di-(C₁₋₅-alkyl)-aminocarbonyl-C₀₋₅-alkyl or a C₄₋₇-cycloalkyleneiminocarbonyl-C₀₋₅-alkyl- group,
or two groups R^{8b}/R^{8c} together with the cyclic carbon atom may form a 3-, 4-, 5-, 6- or 7-membered saturated carbocyclic group or a cyclopentene, cyclohexene, oxetan, azetidine, thietan, tetrahydrofuran, pyrrolidine, tetrahydrothiophene, tetrahydropyran, piperidine, pentamethylene sulphide, hexamethyleneimine, hexahydropyridazine, tetrahydro-2(1H)-pyrimidinone, [1.3]oxazinan-2-one ring,
while the methylene groups thereof may be substituted by 1-2 C₁₋₃-alkyl or CF₃- groups, and/or
the methylene groups thereof, if they are not bound to a heteroatom, may be substituted by 1-2 fluorine atoms, and/or wherein a -CH₂ group besides a nitrogen atom may be replaced by a -CO group, and/or
the imino groups of which may each be substituted by a C₁₋₃-alkyl or C₁₋₃-alkylcarbonyl group, and/or
wherein the sulphur atom may be oxidised to form a sulphoxide or sulphone group,
with the proviso that a heteroatom introduced by R^{8b} or R^{8c} must not be only one carbon atom away from X in formula (I),
and
in all not more than four groups selected from among R^{7a}, R^{7b}, R^{7c}, R^{8a}, R^{8b} and R^{8c} may be present, and
X denotes an oxygen or sulphur atom, a CF₂, sulphene, sulphone or a NR¹ group, wherein
R¹ denotes a hydrogen atom or a hydroxy, C₁₋₃-alkyloxy, amino, C₁₋₃-alkylamino, di-(C₁₋₃-alkyl)-amino, a C₁₋₅-alkyl, C₂₋₅-alkenyl-CH₂, C₂₋₅-alkynyl-CH₂, C₃₋₆-cycloalkyl, C₄₋₆-cycloalkenyl, oxetan-3-yl, tetrahydrofuran-3-yl, benzyl, C₁₋₅-alkyl-carbonyl, trifluoromethylcarbonyl, C₃₋₆-cycloalkyl-carbonyl, C₁₋₅-alkylsulphonyl, C₃₋₆-cycloalkyl-sulphonyl, aminocarbonyl, C₁₋₅-alkylaminocarbonyl, di-(C₁₋₅-alkyl)-aminocarbonyl, C₁₋₅-alkyloxycarbonyl, C₄₋₇-cycloalkyleneiminocarbonyl group,
while the methylene and methyl groups present in the groups mentioned above may additionally be substituted by a C₁₋₃alkyl, carboxy, C₁₋₅-alkoxycarbonyl group, or may be substituted by a hydroxy, C₁₋₅-alkyloxy, amino, C₁₋₅-alkylamino, C₁₋₅-dialkylamino or C₄₋₇-cycloalkyleneimino group,
if the methylene or methyl groups are not directly bound to a heteroatom selected from among O, N or S, and/or one to three hydrogen atoms may be replaced by fluorine atoms, if the methylene or methyl groups are not directly bound to a heteroatom selected from among O, N or S,
and wherein
A¹ denotes either N or CR¹⁰,
A² denotes either N or CR¹¹,
A³ denotes either N or CR¹²,
while R¹⁰, R¹¹ and R¹² each independently of one another denote
a hydrogen, fluorine, chlorine, bromine or iodine atom, or a C₁₋₅-alkyl, CF₃, C₂₋₅ -alkenyl, C₂₋₅-alkynyl, a cyano, carboxy, C₁₋₅-alkyloxycarbonyl, hydroxy, C₁₋₃-alkyloxy, CF₃O, CHF₂O, CH₂FO, amino, C₁₋₅-alkylamino, di-(C₁₋₅-alkyl)-amino or C₄₋₇-cycloalkyleneimino group, and
-L-E-G-J- denotes a ―C-C-C-C- or -C-C=C-C- group which may be substituted by R⁴ and R⁵, and
R³ denotes a hydrogen atom or a C₁₋₃-alkyl group, and
R⁴ denotes a hydrogen atom or
a straight-chain or branched C₁₋₆-alkyl, C₂₋₆-alkenyl or C₂₋₆-alkynyl group,
wherein the hydrogen atoms of the methylene and/or methyl fragments of the straight-chain or branched C₁₋₆-alkyl, C₂₋₆-alkenyl or C₂₋₆-alkynyl group may optionally be wholly or partly replaced by fluorine atoms, and/or
wherein the hydrogen atoms of the methylene and/or methyl fragments of the straight-chain or branched C₁₋₆-alkyl, C₂₋₆-alkenyl or C₂₋₆-alkynyl group may optionally each be substituted independently of one another by one to two substituents selected from among a C₃₋₅-cycloalkyl group, a nitrile, hydroxy or C₁₋₅-alkyloxy group, while the hydrogen atoms of the C₁₋₅-alkyloxy group may optionally be wholly or partly replaced by fluorine atoms, an allyloxy, propargyloxy, benzyloxy, C₁₋₅-alkylcarbonyloxy, C₁₋₅-alkyloxycarbonyloxy, carboxy-C₁₋₅-alkyloxy, C₁₋₅-alkyloxycarbonyl-C₁₋₅-alkyloxy, mercapto, C₁₋₅-alkylsulphanyl, C₁₋₅-alkylsulphinyl, C₁₋₅-alkylsulphonyl, carboxy, C₁₋₅-alkyloxycarbonyl, aminocarbonyl, C₁₋₅-alkylaminocarbonyl, di-(C₁₋₅-alkyl)-aminocarbonyl, C₄₋₇-cycloalkyleneiminocarbonyl, aminosulphonyl, C₁₋₅-alkylaminosulphonyl, di-(C₁₋₅-alkyl)-aminosulphonyl, C₄₋₇-cycloalkyleneiminosulphonyl, amino, C₁₋₅-alkylamino, di-(C₁₋₅-alkyl)-amino, C₁₋₅-alkylcarbonylamino, C₁₋₅-alkylsulphonylamino, *N*-(C₁₋₅-alkylsulphonyl)-C₁₋₅-alkylamino, C₃₋₆-cycloalkylcarbonylamino group, or a morpholinyl, thiomorpholinyl, pyrrolidinyl, piperidinyl, piperazinyl, tetrahydrofuranyl, tetrahydropyranyl group, while the above-mentioned carbocyclic and heterocyclic groups in the ring may each be substituted by 1-4 C₁₋₃-alkyl or C₁₋₃-alkylcarbonyl groups or by 1-2 oxo groups, and/or wherein the hydrogen atoms of the sp²-hybridised carbon atoms of the straight-chain or branched C₂₋₆-alkenyl group may optionally be wholly or partly replaced by fluorine atoms, or
a nitrile, carboxy, aminocarbonyl, C₁₋₅-alkylaminocarbonyl, C₃₋₆-cycloalkylaminocarbonyl, di-(C₁₋₅-alkyl)-aminocarbonyl, C₁₋₅-alkyloxycarbonyl or a C₄₋₇-cycloalkyleneiminocarbonyl group wherein optionally a methylene group may be replaced by a oxygen, sulphur or C₀₋₃-alkyl-substituted nitrogen atom, or
a phenyl, mono- or bicyclic heteroaryl, phenyl-C₁₋₅-alkyl or mono- or bicyclic heteroaryl-C₁₋₅-alkyl group,
which may optionally be mono- to trisubstituted in the phenyl or heteroaryl moiety by identical or different substituents selected from among fluorine, chlorine, bromine and iodine atoms, and C₁₋₅-alkyl, trifluoromethyl, amino, C₁₋₅-alkyl-amino, di-(C₁₋₅-alkyl)-amino, hydroxy, C₁₋₅-alkyloxy, mono-, di- or trifluoromethoxy, carboxy and C₁₋₅-alkyloxycarbonyl group,
and
if -L-E-G-J- denotes a ―C-C-C-C- group, R⁴ at E or G may also denote a fluorine atom or a hydroxy, methoxy, C₃₋₅-alkenyl-oxy, C₃₋₅-alkynyloxy, C₂₋₅-alkyloxy, C₃₋₆-cycloalkyl-oxy, C₁₋₅-alkylaminocarbonyloxy, di(C₁₋₅-alkyl)aminocarbonyloxy or C₄₋₇-cycloalkyleneiminocarbonyloxy, phenyl-C₀₋₃-alkyloxy, heteroaryl-C₀₋₃-alkyloxy, amino, C₁₋₅-alkylamino, di-(C₁₋₅-alkyl)-amino, C₄₋₇-cycloalkyleneimino, C₁₋₃-acylamino, (C₁₋₃-acyl)C₁-₃-alkylamino, C₁₋₅-alkyloxycarbonylamino, C₁₋₅-alkylaminocarbonylamino, di(C₁₋₅-alkyl)aminocarbonylamino or a C₄₋₇-cycloalkyleneiminocarbonyl-amino group,
while the methyl or methylene groups present in the above-mentioned alkyl or cycloalkyl groups may each independently of one another be substituted by a substituent selected from among morpholinyl, thiomorpholinyl, pyrrolidinyl, piperidinyl, piperazinyl, tetrahydrofuranyl, tetrahydropyranyl, dimethylaminocarbonyl, C₁₋₅-alkyloxycarbonyl, carboxy, methyl, hydroxy, methoxy or amino,
and
the above-mentioned phenyl or heteroaryl groups may optionally be mono- to trisubstituted by identical or different substituents selected from among fluorine, chlorine, bromine and iodine atoms, and C₁₋₅-alkyl, trifluoromethyl, amino, C₁₋₅-alkyl-amino, di-(C₁₋₅-alkyl)-amino, hydroxy, C₁₋₅-alkyloxy, mono-, di- or trifluoromethoxy, carboxy- and C₁₋₅-alkyloxycarbonyl group,
with the proviso
that two heteroatoms selected from among oxygen and nitrogen are separated from one another by precisely one optionally substituted CH₂ group, and/or
that two atoms form an ―O-O or ―S-O- bond,
is excluded, and
R⁵ denotes a hydrogen atom, a C₁₋₅ alkyl, C₂₋₅ alkenyl or C₂₋₅ alkynyl or a phenyl-C₀₋₅ alkyl group, wherein the alkyl group may be substituted by a hydroxy, methoxy, hydroxycarbonyl or C₁₋₅-alkoxycarbonyl group,
or if R⁵ is linked to E or G it may also denote a hydroxy or methoxy group, or
R⁴ and R⁵ if they are bound to the same carbon atom, they may form together with the carbon atom a -C=O group or a -CF₂- group, or
R⁴ and R⁵ if they are bound to the same carbon atom or to two adjacent carbon atoms, together with the carbon atom(s) they may form a 3-7-membered carbocyclic group or a monounsaturated 5-7 membered carbocyclic group,
wherein one of the carbon chain members of this cyclic group may be replaced by an oxygen or sulphur atom or a -NH-, -N(C₁₋₅-alkyl)-, -N(C₁₋₄-alkylcarbonyl)- or a carbonyl, sulphinyl or sulphonyl group, and/or
two directly adjacent carbon chain members of these C₄₋₇-carbocyclic groups may together be replaced by a -C(O)NH-, -C(O)N(C₁₋₅-alkyl)-, -S(O)₂NH-, or -S(O)₂N(C₁₋₅-alkyl)- group, and/or
four directly adjacent carbon chain members of these C₅₋₇-carbocyclic groups may together be replaced by an -O-CH₂-CH₂-O group, and/or
1 to 3 carbon atoms of these 3-7-membered cyclic groups may optionally be substituted independently of one another by in each case one or two fluorine atoms or one or two C₁₋₅-alkyl groups or a hydroxy, formyloxy, C₁₋₅-alkyloxy, C₁₋₅-alkylcarbonyloxy, amino, C₁₋₅-alkylamino, di-(C₁₋₅-alkyl)-amino, C₄₋₇-cycloalkyleneimino, C₁₋₅-alkylcarbonylamino, C₃₋₆-cycloalkylcarbonylamino, nitrile, carboxy-C₁₋₅-alkyl, C₁₋₅-alkyloxycarbonyl-C₁₋₅-alkyl, carboxy, C₁₋₅-alkyloxycarbonyl, aminocarbonyl, C₁₋₅-alkylaminocarbonyl, di-(C₁₋₅-alkyl)-aminocarbonyl or C₄₋₇-cycloalkyleneiminocarbonyl group,
with the proviso that a cyclic group of this kind formed from R⁴ and R⁵ together,
wherein two nitrogen atoms or one nitrogen and one oxygen atom in the cyclic group are separated from one another by precisely one optionally substituted CH₂ group, and/or wherein two atoms in the ring form a ―O-O- or ―S-O- bond,
is excluded,
or the fragment 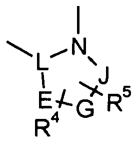 denotes the group 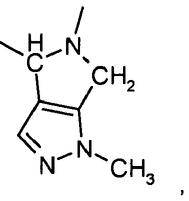 R¹³ denotes a hydrogen atom or a C₁₋₅ alkyl group,
M denotes a phenyl, thienyl or pyridyl ring optionally substituted by R² and R⁶, wherein
R² denotes a fluorine, chlorine, bromine or iodine atom or a methyl, ethyl, vinyl, methoxy, ethynyl, cyano or ―C(O)NH₂ group, and
R⁶ denotes a hydrogen, fluorine, chlorine, bromine or iodine atom or a hydroxy, methoxy, trifluoromethoxy, a C₁₋₃-alkyl optionally substituted by fluorine atoms, a cyano, amino or NH₂C(O)- group,
while, unless stated otherwise, by the term "heteroaryl group" mentioned hereinbefore in the definitions is meant a monocyclic 5- or 6-membered heteroaryl group, wherein
the 6-membered heteroaryl group contains one, two or three nitrogen atoms, and
the 5-membered heteroaryl group contains an imino group optionally substituted by a C₁₋₃-alkyl group, or an oxygen or sulphur atom, or
an imino group optionally substituted by a C₁₋₃-alkyl group or an oxygen or sulphur atom and additionally one or two nitrogen atoms, or
an imino group optionally substituted by a C₁₋₃-alkyl group and three nitrogen atoms,
and moreover a phenyl ring optionally substituted by a fluorine, chlorine or bromine atom, a C₁₋₃-alkyl, hydroxy, C₁₋₃-alkyloxy group, amino, C₁₋₃-alkylamino, di-(C₁₋₃-alkyl)-amino or C₃₋₆-cycloalkyleneimino group may be fused to the above-mentioned monocyclic heteroaryl groups via two adjacent carbon atoms,
and the bond is effected via a nitrogen atom or a carbon atom of the heterocyclic moiety or a fused-on phenyl ring,
and wherein, unless stated otherwise, by the term "halogen atom" mentioned hereinbefore in the definitions is meant an atom selected from among fluorine, chlorine, bromine and iodine,
and wherein the alkyl, alkenyl, alkynyl and alkyloxy groups contained in the previously mentioned definitions which have more than two carbon atoms may, unless stated otherwise, be straight-chain or branched and the alkyl groups in the previously mentioned dialkylated groups, for example the dialkylamino groups, may be identical or different,
and the hydrogen atoms of the methyl or ethyl groups contained in the foregoing definitions, unless stated otherwise, may be wholly or partly replaced by fluorine atoms,
the tautomers, the enantiomers, the diastereomers, the mixtures and the salts thereof.

2. Compounds of general formula (I) according to claim 1, wherein
D denotes a substituted bicyclic ring system of formula 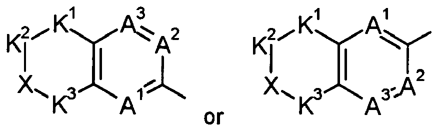 wherein
K¹
denotes a -CH₂-, -CHR^{7a}-, -CR^{7b}R^{7c}- or a -C(O)- group, and wherein R^{7a}/R^{7b}/R^{7c}
each independently of one another denote a fluorine atom, a hydroxy, C₁₋₅-alkyloxy, a C₁₋₅-alkyl group,
while the two groups R^{7b}/^{R7c} may not simultaneously be bound to the cyclic carbon atom via a heteroatom, except where -C(R^{7b}R^{7c})-corresponds to a ―CF₂ group, or
two groups R^{7b}/R^{7c} together with the cyclic carbon atom may form a 3-membered carbocyclic group and
K² and K³
each independently of one another denote a -CH₂-, -CHR^{8a}-, -CR^{8b}R^{8c}-or a -C(O)- group , wherein
R^{8a}/R^{8b}/R^{8c}
each independently of one another denote a C₁₋₅-alkyl group,
and/or
two groups R^{8b}/R^{8c} together with the cyclic carbon atom may form a 3-membered saturated carbocyclic group
and
in all not more than four groups selected from among R^{7a}, R^{7b}, R^{7c}, R^{8a}, R^{8b} and R^{8c} may be present, and
X denotes an oxygen or sulphur atom, a sulphene, sulphone, -CF₂-or an NR¹ group, wherein
R¹ denotes a hydrogen atom or a hydroxy, C₁₋₃-alkyloxy, amino, C₁₋₃-alkylamino, di-(C₁₋₃-alkyl)-amino, a C₁₋₅-alkyl, C₂₋₅-alkenyl-CH₂, C₂₋₅-alkynyl-CH₂ or a C₃₋₆-cycloalkyl group,
and wherein
A¹ denotes either N or CR¹⁰,
A² denotes either N or CR¹¹,
A³ denotes either N or CR¹²,
wherein R¹⁰, R¹¹ and R¹² each independently of one another denote
a hydrogen, fluorine, chlorine, bromine or iodine atom, or a C₁₋₅-alkyl, CF₃, a cyano, carboxy, C₁₋₅-alkyloxycarbonyl, hydroxy, C₁₋₃-alkyloxy, CF₃O, CHF₂O, CH₂FO, amino, C₁₋₅-alkylamino, di-(C₁₋₅-alkyl)-amino or C₄₋₇-cycloalkyleneimino group,
the tautomers, the enantiomers, the diastereomers, the mixtures and the salts thereof.

3. Compounds of general formula (I) according to claim 1 or 2, wherein
X denotes an NR¹ group, wherein
R¹ denotes a hydrogen atom or a C₁₋₅-alkyl, allyl or cyclopropyl group, and
A¹ denotes CR¹⁰,
A² denotes CR¹¹,
A³ denotes either N or CR¹²,
wherein R¹⁰, R¹¹ and R¹² each independently of one another denote a hydrogen, fluorine or chlorine atom, or a methyl, CF₃, cyano, carboxy, C₁₋₅-alkyloxycarbonyl, hydroxy, methoxy, CF₃O, CHF₂O, CH₂FO group,
the tautomers, the enantiomers, the diastereomers, the mixtures and the salts thereof.

4. Compounds of general formula (I) according to one of claims 1, 2 or 3, wherein
R⁴ denotes a hydrogen atom or
a straight-chain or branched C₁₋₆-alkyl group, wherein the hydrogen atoms of the methylene and/or methyl fragments of the straight-chain or branched C₁₋₆-alkyl group may optionally be wholly or partly replaced by fluorine atoms, and/or wherein the hydrogen atoms of the methylene and/or methyl fragments of the straight-chain or branched C₁₋₆alkyl group may optionally each be substituted independently of one another by a substituent selected from among a hydroxy, C₁₋₅-alkyloxy, carboxy, C₁₋₅-alkyloxycarbonyl, aminocarbonyl, C₁₋₅-alkylaminocarbonyl, di-(C₁₋₅-alkyl)-aminocarbonyl, C₄₋₇-cycloalkyleneiminocarbonyl, amino, C₁₋₅-alkylamino, di-(C₁₋₅-alkyl)-amino, C₁₋₅-alkylcarbonylamino, C₁₋₅-alkylsulphonylamino, *N*-(C₁₋₅-alkylsulphonyl)-C₁₋₅-alkylamino, C₃₋₆-cycloalkylcarbonylamino group, or
a nitrile, carboxy, aminocarbonyl, C₁₋₅-alkylaminocarbonyl, C₃₋₆-cycloalkylaminocarbonyl, di-(C₁₋₅-alkyl)-aminocarbonyl, C₁₋₅-alkyloxycarbonyl or a C₄₋₇-cycloalkyleneiminocarbonyl group wherein a methylene group may optionally be replaced by a oxygen, sulphur or C₀₋₃-alkyl-substituted nitrogen atom,
and
if -L-E-G-J- denotes a ―C-C-C-C- group, R⁴ at E or G may also denote a fluorine atom or a hydroxy, methoxy, C₃₋₅-alkenyloxy, C₃₋₅-alkynyloxy, C₂₋₅-alkyl-oxy, C₃₋₆-cycloalkyl-oxy, C₁₋₅-alkylaminocarbonyloxy, di(C₁₋₅-alkyl)aminocarbonyloxy or C₄₋₇-cycloalkyleneiminocarbonyloxy, phenyl-C₀₋₂-alkyloxy group, which may be substituted in the phenyl ring by 1-2 fluorine atoms or methoxy groups, an amino, C₁₋₅-alkylamino, di-(C₁₋₅-alkyl)-amino, C₄₋₇-cycloalkyleneimino, C₁₋₃-acylamino, (C₁₋₃-acyl)C₁₋₃-alkylamino, C₁₋₅-alkyloxycarbonylamino, C₁₋₅-alkylaminocarbonylamino, di(C₁₋₅-alkyl)aminocarbonylamino or a C₄₋₇-cycloalkyleneiminocarbonylamino group,
while the methyl or methylene groups present in the above-mentioned alkyl or cycloalkyl groups may each independently of one another be substituted by a substituent selected from among dimethylaminocarbonyl, C₁₋₅alkyloxycarbonyl, carboxy, methyl, hydroxy, methoxy or amino,
with the proviso
that two heteroatoms selected from among oxygen and nitrogen are separated from one another by precisely one optionally substituted -CH₂- group, and/or
that two atoms form an ―O-O- or ―S-O- bond,
is excluded, and
R⁵ denotes a hydrogen atom or a C₁₋₅alkyl, allyl, propargyl or benzyl group, or if R⁵ is linked to E or G, it may also denote a hydroxy or methoxy group or
R⁴ and R⁵ if they are bound to the same carbon atom, may form together with the carbon atom a -C=O group, or a -CF₂- group, or
R⁴ and R⁵ if they are bound to the same carbon atom or to two adjacent carbon atoms, may form together with the carbon atom(s) a 3-7-membered carbocyclic group,
while one of the carbon chain members of this cyclic group may be replaced by an oxygen or sulphur atom or a -NH-, -N(C₁₋₅-alkyl)-, -N(C₁₋₄-alkylcarbonyl)- or a carbonyl, sulphinyl or sulphonyl group, and/or
two directly adjacent carbon chain members of these C₄₋₇-carbocyclic groups may together be replaced by an -C(O)NH-, -C(O)N(C₁₋₅-alkyl)-, -S(O)₂NH-, or -S(O)₂N(C₁₋₅-alkyl)- group, and/or
four directly adjacent carbon chain members of these C₅₋₇carbocyclic groups may together be replaced by a -O-CH₂-CH₂O- group,
with the proviso that a cyclic group formed from R⁴ and R⁵ together,
wherein two nitrogen atoms or one nitrogen and one oxygen atom in the cyclic group are separated from one another by precisely one optionally substituted CH₂ group, and/or
wherein two atoms in the ring form a ―O-O- or ―S-O- bond,
is excluded,
the tautomers, the enantiomers, the diastereomers, the mixtures and the salts thereof.

5. Compounds of general formula (I) according to one of claims 1, 2, 3 or 4, wherein
-L-E-G-J- denotes a ―C-C-C-C- group which may be substituted by R⁴ and R⁵, which are defined as in claims 1, 2, 3 or 4 hereinbefore,
the tautomers, the enantiomers, the diastereomers, the mixtures and the salts thereof.

6. Compounds of general formula (I) according to one of claims 1, 2, 3, 4 or 5, wherein
D denotes a substituted bicyclic ring system of general formula 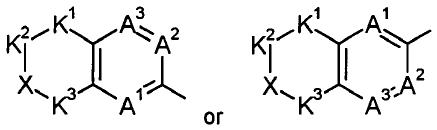 wherein
K¹ denotes a -CH₂-, -CHR^{7a}-, -CR^{7b}R^{7c}- or a -C(O)- group, wherein
R^{7a} denotes a C₁₋₂-alkyl group and
R^{7b}/R^{7c} each independently of one another denote a hydroxy, methoxy or a C₁₋₃-alkyl group,
while the two groups R^{7b}/R^{7c} may not simultaneously be bound to the cyclic carbon atom via an oxygen atom, or
two groups R^{7b}/R^{7c} together with the cyclic carbon atom may form a 3-membered carbocyclic group,
and
K² and K³ in each case independently of one another denote a -CH₂-, -CHR^{8a}- or a -CR^{8b}R^{8c}- group , wherein
R^{8a}/R^{8b}/R^{8c}
each independently of one another denote a C₁₋₃-alkyl group,
and/or
two groups R^{8b}/R^{8c} together with the cyclic carbon atom may form a 3-membered saturated carbocyclic group
and
in all not more than four groups selected from among R^{7a}, R^{7b}, R^{7c}, R^{8a}, R^{8b} and R^{8c} may be present, and
X denotes a NR¹ group, wherein
R¹ denotes a hydrogen atom or a C₁₋₃-alkyl, allyl or cyclopropyl group, and
A¹ denotes CR¹⁰,
A² denotes CR¹¹,
A³ denotes CR¹²,
wherein R¹⁰, R¹¹ and R¹² each independently of one another denote
a hydrogen, fluorine or chlorine atom, or a methyl, CF₃, hydroxy, methoxy, CF₃O, CHF₂O, CH₂FO group,
and
-L-E-G-J- denotes a -C-C-C-C- group, which may be substituted by R⁴ and R⁵, and
R³ denotes a hydrogen atom, and
R⁴ denotes a hydrogen atom or
a straight-chain or branched C₁₋₃-alkyl group,
wherein the hydrogen atoms of the methylene and/or methyl fragments of the straight-chain or branched C₁₋₆alkyl group may optionally be substituted independently of one another by a substituent selected from among a hydroxy, C₁₋₅-alkyloxy, carboxy, C₁₋₅-alkyloxycarbonyl group, or
if R⁴ is bound to E or G it may also denote a fluorine atom or a hydroxy, methoxy, C₃₋₅-alkenyl-oxy, C₂₋₅-alkyl-oxy, C₃₋₆-cycloalkyl-oxy, C₁₋₅-alkylaminocarbonyloxy, di(C₁₋₅-alkyl)aminocarbonyloxy or C₄₋₇-cycloalkyleneiminocarbonyloxy group,
with the proviso
that two heteroatoms selected from among oxygen and nitrogen are separated from one another by precisely one optionally substituted -CH₂- group,
is excluded, and
R⁵ denotes a hydrogen atom or a C₁₋₅alkyl, allyl or benzyl group, or if R⁵ is linked to E or G it may also denote a hydroxy or methoxy group, or
R⁴ and R⁵ if they are bound to the same carbon atom, may form together with
the carbon atom a -C=O- group, or a -CF₂- group, or
R⁴ and R⁵ if they are bound to the same carbon atom or to two adjacent carbon atoms, may form together with the carbon atom(s) a 3-6-membered carbocyclic group,
while four directly adjacent carbon chain members of these C₅₋₆-carbocyclic groups may together be replaced by an -O-CH₂-CH₂O group,
R¹³ denotes a hydrogen atom,
M denotes a phenyl substituted by R² in the 4-position or a pyridyl ring substituted by R² in the 5-position, wherein
R² denotes a fluorine, chlorine, bromine atom, a methoxy or ethynyl group, and
R⁶ denotes a hydrogen or fluorine atom,
the tautomers, the enantiomers, the diastereomers, the mixtures and the salts thereof.

7. Compounds of general formula (I) according to one of claims 1, 2, 3, 4, 5 or 6, wherein the central ring 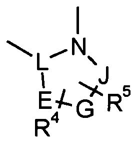 denotes either 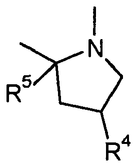 or 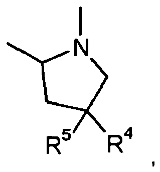 the tautomers, the enantiomers, the diastereomers, the mixtures and the salts thereof.

8. Compounds of general formula (I) according to one of claims 1, 2, 3, 4, 5, 6 or 7, wherein
D denotes a substituted bicyclic ring system of general formula 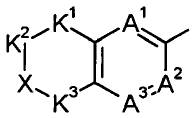 the tautomers, the enantiomers, the diastereomers, the mixtures and the salts thereof.

9. Compounds of general formula (I) according to one of claims 1, 2, 3, 4, 5, 6, 7 or 8 which are in the R configuration at the chain members G and L of the 5-membered central ring,
the tautomers, the enantiomers, the diastereomers, the mixtures and the salts thereof.

10. Compound according to claim 1, selected from among: 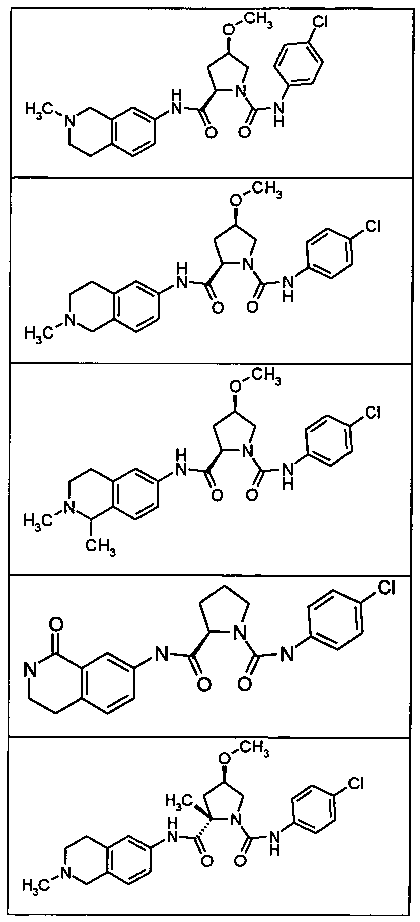 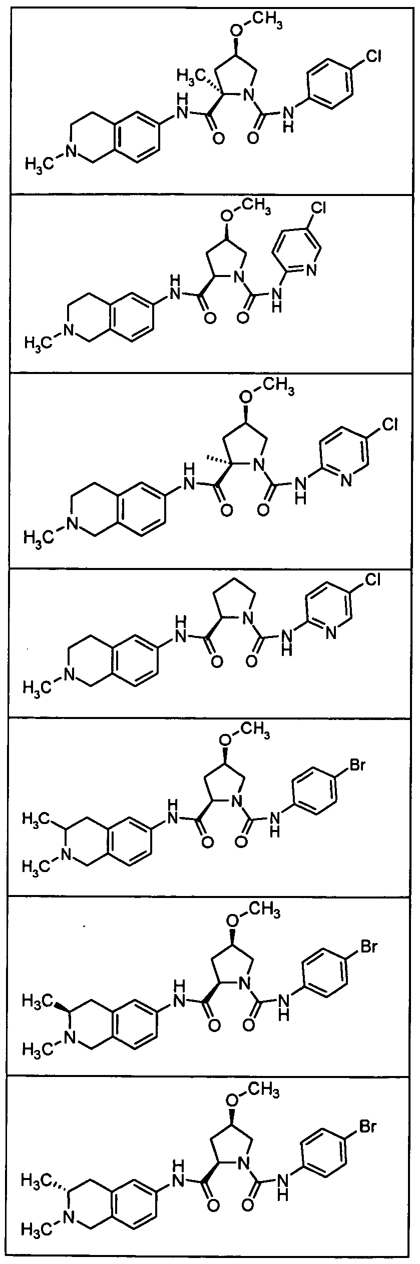 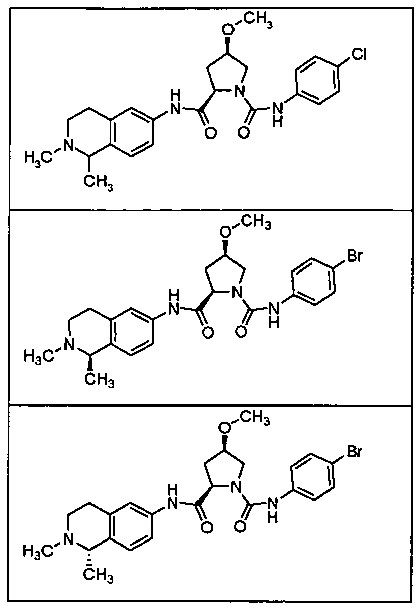 the tautomers, the enantiomers, the diastereomers, the mixtures and the salts thereof.

11. Compound according to claim 1, selected from among: 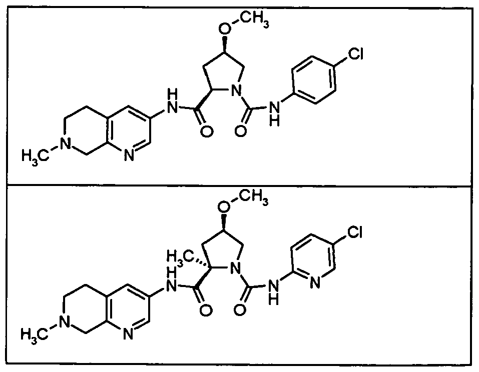 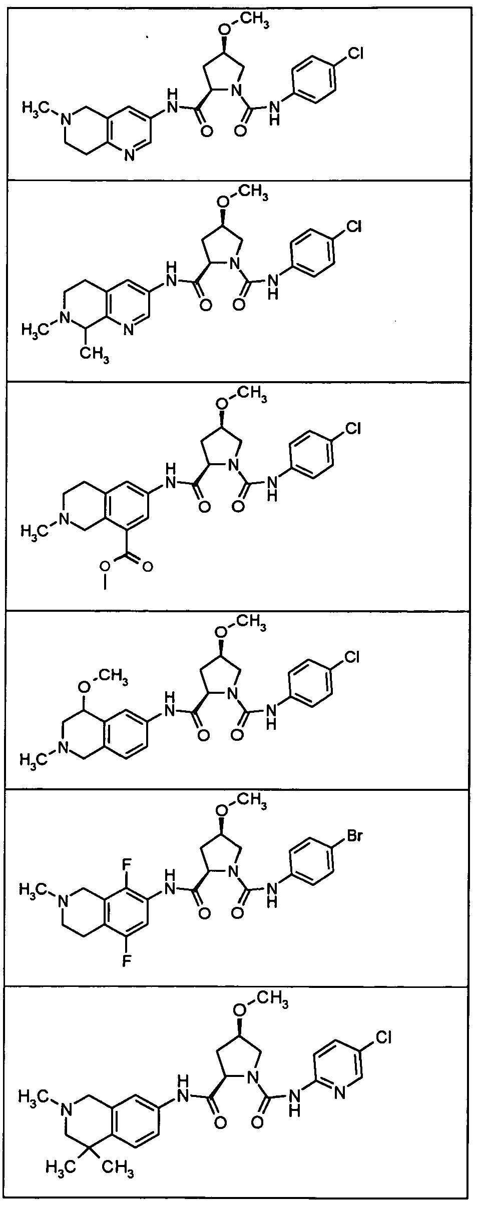 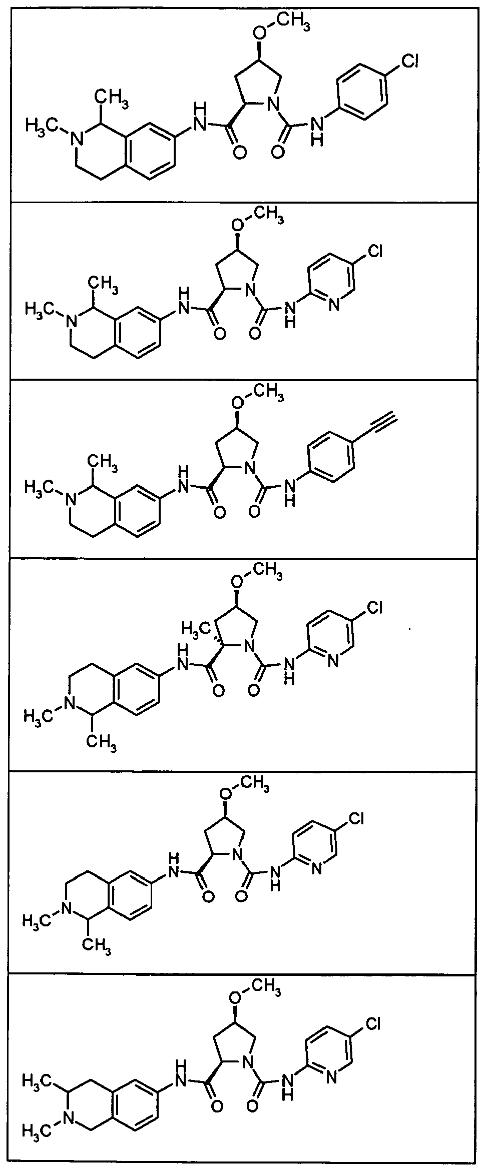 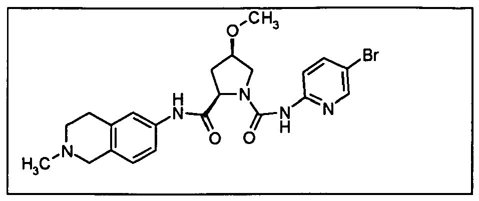 the tautomers, the enantiomers, the diastereomers, the mixtures and the salts thereof.

12. Compound according to claim 1, selected from among: 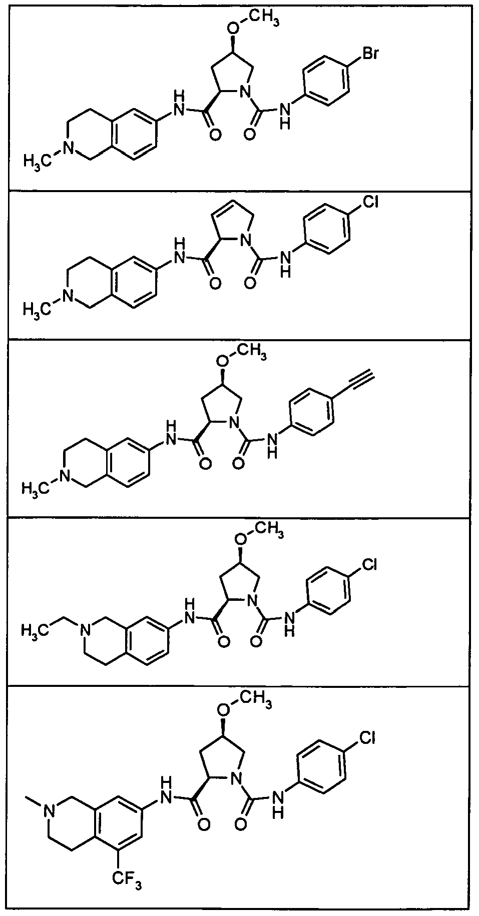 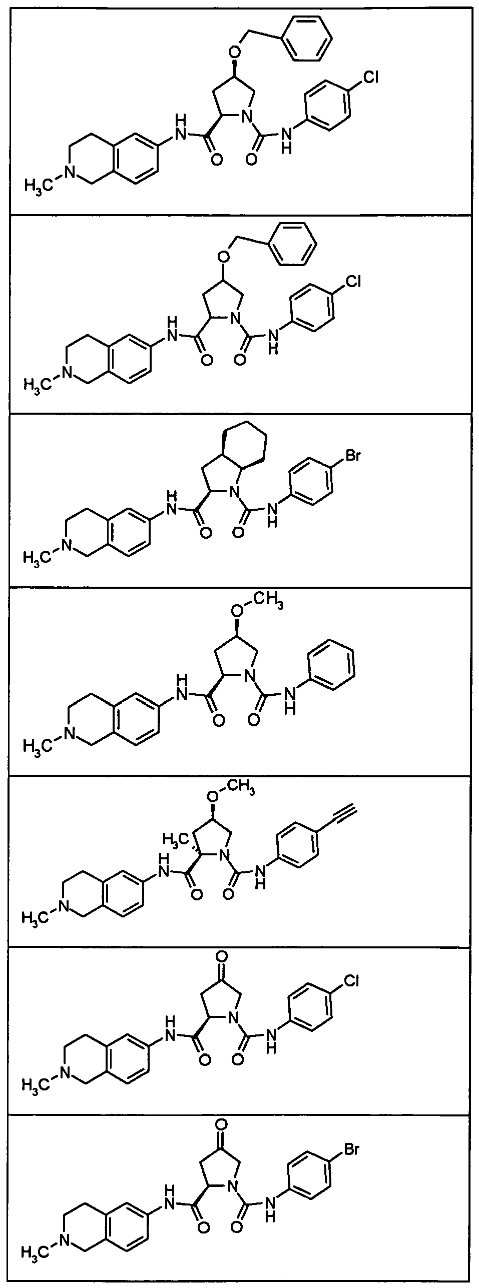 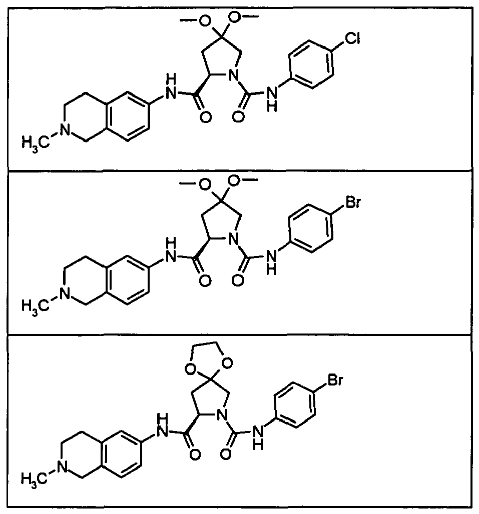 the tautomers, the enantiomers, the diastereomers, the mixtures and the salts thereof.

13. Compound according to claim 1, selected from among: 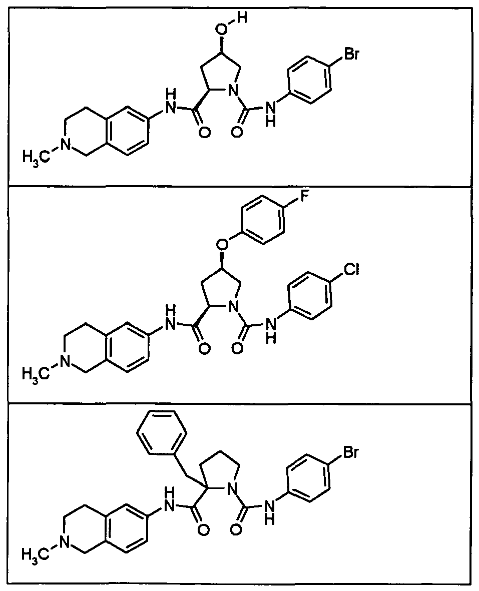 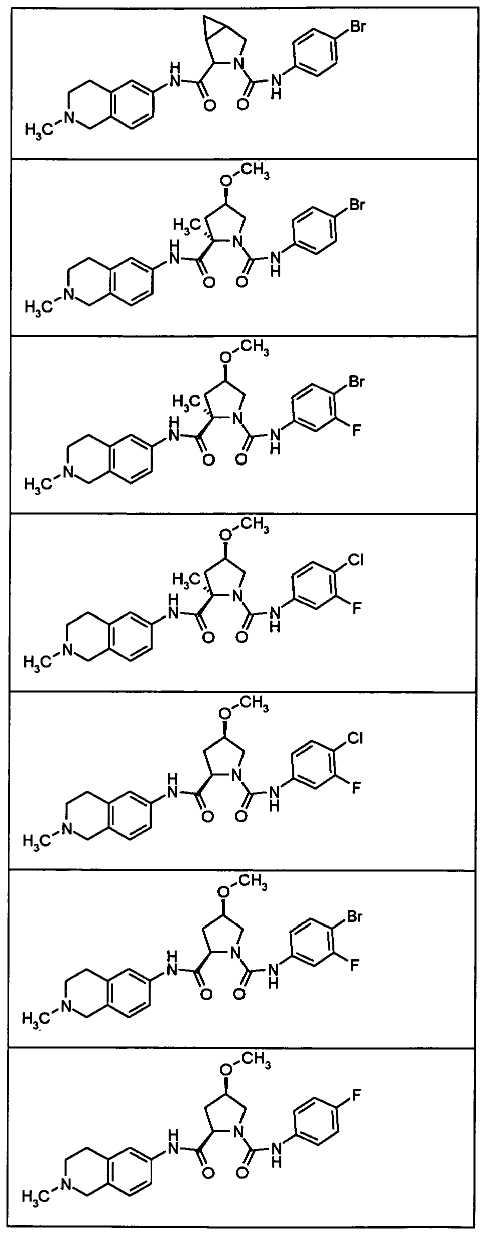 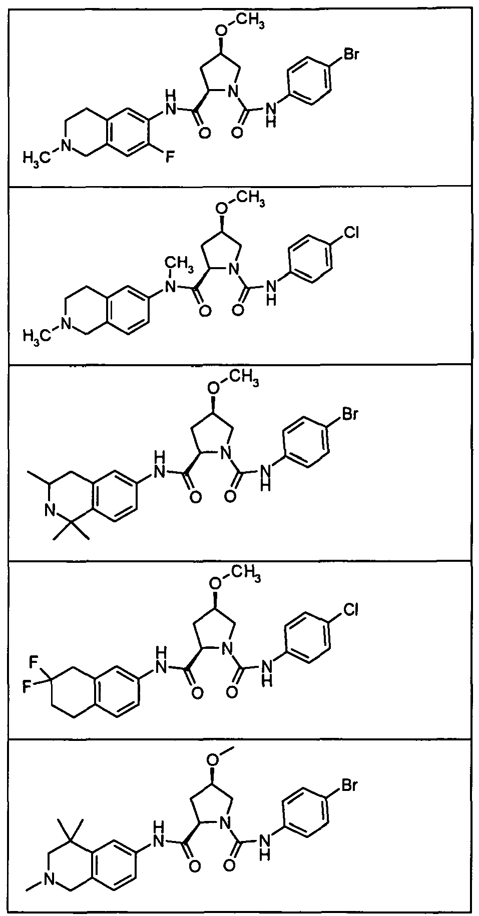 the tautomers, the enantiomers, the diastereomers, the mixtures and the salts thereof.

14. Compound according to claim 1, selected from among: 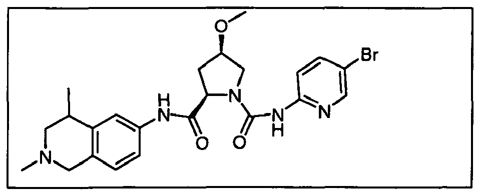 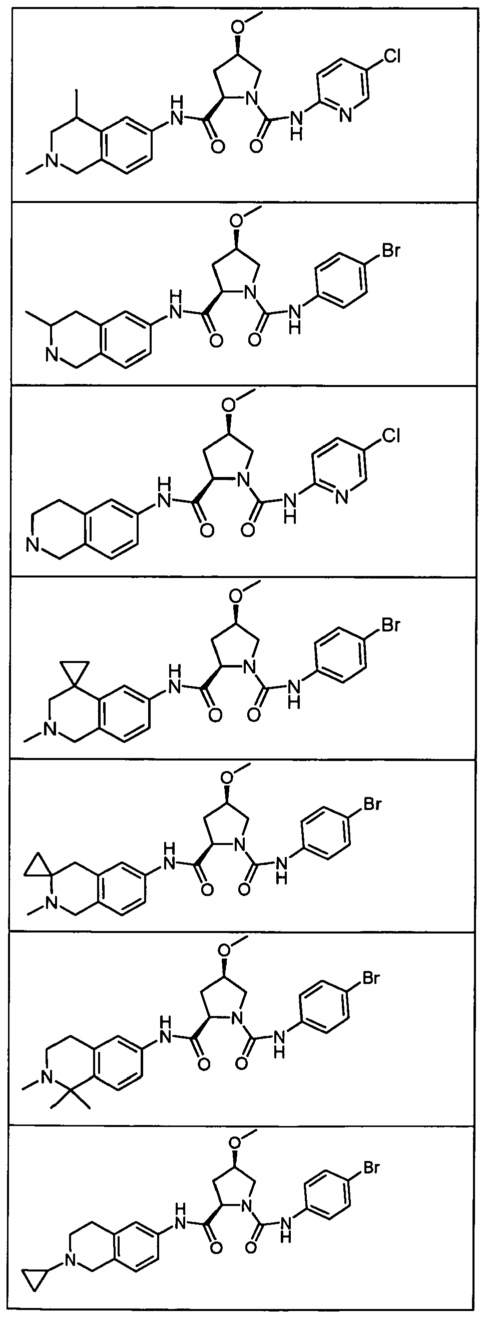 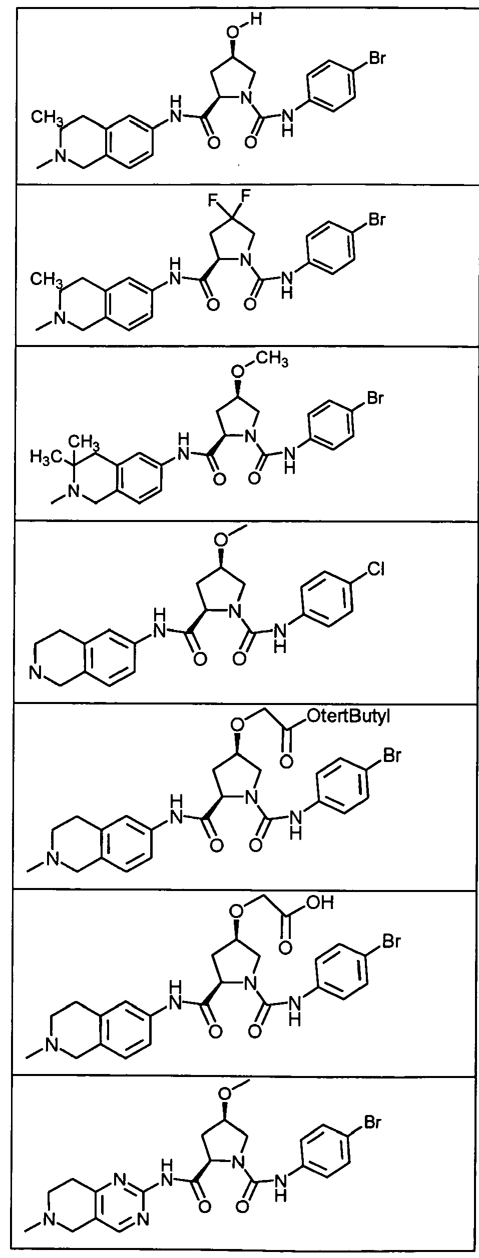 the tautomers, the enantiomers, the diastereomers, the mixtures and the salts thereof.

15. Physiologically acceptable salts of the compounds according to one of claims 1 to 14.

16. Medicaments, containing a compound according to at least one of claims 1 to 14 or a physiologically acceptable salt according to claim 15, optionally in addition to one or more inert carriers and/or diluents.

17. Compound according to at least one of claims 1 to 14 or a physiologically acceptable salt according to claim 15 for use as a medicament.

18. Method of preparing a medicament according to claim 16, **characterised in that** a compound according to at least one of claims 1 to 14 or a physiologically acceptable salt according to claim 15 is incorporated in one or more inert carriers and/or diluents by a non-chemical method.

## Revendications

1. Composés de formule générale (I) 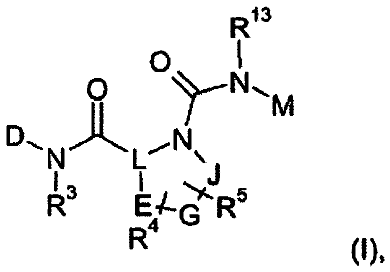 dans lesquels
D est un système cyclique bicyclique substitué de formule 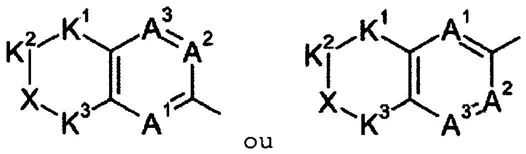 dans lequel
K¹
Désigne une liaison, un groupe -CH₂-, -CHR^{7a}-, -CR^{7b}R^{7c}- ou -C(O)-, et où
R^{7a}/R^{7b}/R^{7c}
désignent respectivement, indépendamment les uns des autres, un atome de fluor, un groupe hydroxy, alkyloxy en C₁ à C₅, amino, alkylamino en C₁ à C₅, di-(alkyle en C₁ à C₅)-amino, cycloalkylène-imino en C₃ à C₅, alkylcarbonylamino en C₁ à C₅,
un groupe alkyle en C₁ à C₅ qui peut être substitué par 1 à 3 atomes de fluor, un groupe hydroxy-alkyle en C₁ à C₅, alkyloxy en C₁ à C₅-alkyle en C₁ à C₅, amino-alkyle en C₁ à C₅, alkylamino en C₁ à C₅-alkyle en C₁ à C₅, di-(alkyle en C₁ à C₅)-amino-alkyle en C₁ à C₅, cycloalkylène-imino en C₄ à C₇- alkyle en C₁ à C₅, carboxy-alkyle en C₀ à C₅, alkyloxycarbonyle en C₁ à C₅-alkyle en C₀ à C₅, aminocarbonyl-alkyle en C₀ à C₅, alkylaminocarbonyle en C₁ à C₅-alkyle en C₀ à C₅, di-(alkyle en C₁ à C₅)-aminocarbonyl-alkyle en C₀ à C₅ ou cycloalkylène-iminocarbonyle en C₄ à C₇-alkyle en C₀ à C₅,
où, de façon non concomitante, les deux radicaux R^{7b}/R^{7c} peuvent être liés à l'atome de carbone cyclique par un hétéroatome, à l'exception de -C (R^{7b}R^{7c}) correspondant à un groupe -CF₂-,
ou
R^{7a} désigne un groupe phényle ou hétéroaryle monocyclique substitué par un atome de fluor, de chlore, de brome, un groupe méthyle, méthoxy, amino ou nitro, ou
deux radicaux R^{7b}/R^{7c} conjointement avec l'atome de carbone cyclique peuvent former un carbocycle à 3, 4, 5, 6 ou 7 chaînons saturé ou un cycle cyclopentane, cyclohexane, oxétane, azétidine, thiétane, tétrahydrofurane, pyrrolidine, tétrahydrothiophène, tétrahydropyrane, pipéridine, pentaméthylsulfure, hexaméthylène-imine, 1,3-dioxane, 1,4-dioxane, hexahydropyridazine, pipérazine, thiomorpholine, morpholine, 2-imidazolidinone, 2-oxazolidinone, tétrahydro-2(1H)-pyrimidone ou [1,3]oxazinan-2-one,
où leurs groupes méthylène peuvent être substitués par 1 à 2 groupes alkyle en C₁ à C₃ ou CF₃, et/ou
leurs groupes méthylène, dans la mesure où ils ne sont pas liés à un hétéroatome, peuvent être substitués par 1 à 2 atomes de fluor, et/ou
dans lequel un groupe -CH₂- à côté d'un atome de N peut être remplacé par un groupe -CO-, et/ou
leurs groupes imino peuvent être substitués respectivement par un groupe alkyle en C₁ à C₃ ou alkylcarbonyle en C₁ à C₃, et/ou dans lequel l'atome de soufre peut être oxydé en un sulfoxyde ou un groupe sulfone,
K² et K³
désignent respectivement, indépendamment l'un de l'autre, un groupe -CH₂-, -CHR^{8a}, -CR^{8b}R^{8c}-ou un groupe -C(O)-, où
R^{8a}/R^{8b}/R^{8c}
désignent respectivement, indépendamment les uns des autres, un groupe alkyle en C₁ à C₅ qui peut être substitué par 1 à 3 atomes de fluor, un groupe hydroxy-alkyle en C₁ à C₅, alkyloxy en C₁ à C₅-alkyle en C₁ à C₅, amino-alkyle en C₁ à C₅, alkylamino en C₁ à C₅-alkyle en C₁ à C₅, di-(alkyle en C₁ à C₅)-amino-alkyle en C₁ à C₅, cycloalkylène-imino en C₄ à C₇-alkyle en C₁ à C₅, carboxy-alkyle en C₀ à C₅, alkyloxycarbonyle en C₁ à C₅-alkyle en Co à C₅, aminocarbonyl-alkyle en C₀ à C₅, alkylaminocarbonyle en C₁ à C₅-alkyle en C₀ à C₅, di-(alkyle en C₁ à C₅)-aminocarbonyl-alkyle en C₀ à C₅ ou cycloalkylène-iminocarbonyle en C₉ à C₇-alkyle en C₀ à C₅, ou deux radicaux R^{8b}/R^{8c} conjointement avec l'atome de carbone cyclique peuvent former un carbocycle à 3, 4, 5, 6 ou 7 chaînons saturé ou un cycle cyclopentène, cyclohexène, oxétane, azétidine, thiétane, tétrahydrofurane, pyrrolidine, tétrahydrothiophène, tétrahydropyrane, pipéridine, pentaméthylènesulfure, hexaméthylène-imine, hexahydropyridazine, tétrahydro-2(1H)-pyrimidinone, [1,3]oxazinan-2-one,
où leurs groupes méthylène peuvent être substitués par 1 à 2 groupes alkyle en C₁ à C₃ ou CF₃, et/ou
leurs groupes méthylène, dans la mesure où ils ne sont pas liés à un hétéroatome, peuvent être substitués par 1 à 2 atomes de fluor, et/ou
dans lequel un groupe -CH₂- à côté d'un atome d'azote peut être remplacé par un groupe -CO-, et/ou
leurs groupes imino peuvent être substitués respectivement par un groupe alkyle en C₁ à C₃ ou alkylcarbonyle en C₁ à C₃, et/ou dans lequel l'atome de soufre peut être oxydé en un groupe sulfoxyde ou un groupe sulfone,
à condition qu'un hétéroatome intégré par R^{8b} ou R^{8c} ne puisse pas être éliminé par uniquement un atome de carbone de X dans la formule (I), et
au total, au maximum quatre radicaux choisis parmi R^{7a}, R^{7b}, R^{7c}, R^{8a}, R^{8b} et R^{8c} peuvent être présents, et
X est un atome d'oxygène ou de soufre, un groupe CF₂, sulfène, sulfone ou NR¹, où
R¹ désigne un atome d'hydrogène ou un groupe hydroxy, alkyloxy en C₁ à C₃, amino, alkylamino en C₁ à C₃, di-(alkyle en C₁ à C₃)-amino, un groupe alkyle en C₁ à C₅, alcényle en C₂ à C₅-CH₂, alcinyle en C₂ à C₅-CH₂, cycloalkyle en C₃ à C₆, cycloalcényle en C₄ à C₆, oxétan-3-yle, tétrahydrofuran-3-yle, benzyle, alkyle en C₁ à C₅-carbonyle, trifluorométhylcarbonyle, cycloalkyle en C₃ à C₆-carbonyle, alkyle en C₁ à C₅-sulfonyle, cycloalkyle en C₃ à C₆-sulfonyle, aminocarbonyle, alkylaminocarbonyle en C₁ à C₅, di-(alkyle en C₁ à C₅)-aminocarbonyle, alkyloxycarbonyle en C₁ à C₅, cycloalkylène-iminocarbonyle en C₄ à C₇,
où les groupes méthylène et méthyle qui se trouvent dans les groupes précédemment cités peuvent être substitués par un groupe alkyle en C₁ à C₃, carboxy, alcoxycarbonyle en C₁ à C₅, ou par un groupe hydroxy, alkyloxy en C₁ à C₅, amino, alkylamino en C₁ à C₅, dialkylamino en C₁ à C₅ ou cycloalkylène-imino en C₄ à C₇, dans la mesure où les groupes méthylène ou méthyle ne sont pas liés directement à un hétéroatome du groupe O, N ou S, et/ou un à trois atomes d'hydrogène peuvent être remplacés par des atomes de fluor, dans la mesure où les groupes méthylène ou méthyle ne sont pas liés directement à un hétéroatome du groupe comprenant O, N ou S,
et dans lequel
A¹ désigne soit N soit CR¹⁰,
A² désigne soit N soit CR¹¹,
A³ désigne soit N soit CR¹²,
où R¹⁰, R¹¹ et R¹² sont respectivement, indépendamment les uns des autres
un atome d'hydrogène, de fluor, de chlore, de brome ou d'iode, ou un groupe alkyle en C₁ à C₅, CF₃, alcényle en C₂ à C₅, alcinyle en C₂ à C₅, cyano, carboxy, alkyloxycarbonyle en C₁ à C₅, hydroxy, alkyloxy en C₁ à C₃, CF₃O, CHF₂O, CH₂FO, amino, alkylamino en C₁ à C₅, di-(alkyle en C₁ à C₅)-amino ou cycloalkylène-imino en C₄ à C₇, et
-L-E-G-J- désigne un groupe -C-C-C-C- ou -C-C=C-C- qui peut être substitué par R⁴ et R⁵, et
R³ désigne un atome d'hydrogène ou un groupe alkyle en C₁ à C₃, et
R⁴ désigne un atome d'hydrogène ou
un groupe alkyle en C₁ à C₆ à chaîne linéaire ou ramifiée, alcényle en C₂ à C₆ ou alcinyle en C₂ à C₆,
où les atomes d'hydrogène des fragments méthylène et/ou méthyle du groupe alkyle en C₁ à C₆ à chaîne linéaire ou ramifiée peuvent être remplacés éventuellement par un groupe alcényle en C₂ à C₆ ou alcinyle en C₂ à C₆, en totalité ou en partie, et/ou
les atomes d'hydrogène des fragments méthylène et/ou méthyle du groupe alkyle en C₁ à C₆ à chaîne linéaire ou ramifiée, du groupe alcényle en C₂ à C₆ ou alcinyle en C₂ à C₆ peuvent être remplacés éventuellement, respectivement, indépendamment les uns des autres par un à deux substituants choisis parmi un groupe cycloalkyle en C₃ à C₅, un groupe nitrile, hydroxy ou alkyloxy en C₁ à C₅, où les atomes d'hydrogène du groupe alkyloxy en C₁ à C₅ peuvent être remplacés éventuellement en totalité ou en partie par des atomes de fluor, un groupe allyloxy, propargyloxy, benzyloxy, alkylcarbonyloxy en C₁ à C₁₅, alkyloxycarbonyloxy en C₁ à C₅, carboxy-alkyloxy en C₁ à C₅, alkyloxycarbonyle en C₁ à C₅-alkyloxy en C₁ à C₅, mercapto, alkylsulfanyle en C₁ à C₅, alkylsulfinyle en C₁ à C₅, alkylsulfonyle en C₁ à C₅, carboxy, alkyloxycarbonyle en C₁ à C₅, aminocarbonyle, alkylaminocarbonyle en C₁ à C₅, di-(alkyle en C₁ à C₅)-aminocarbonyle, cycloalkylène-iminocarbonyle en C₄ à C₇, aminosulfonyle, alkylaminosulfonyle en C₁ à C₅, di-(alkyle en C₁ à C₅)-aminosulfonyle, cycloalkylène-iminosulfonyle en C₄ à C₇, amino, alkylamino en C₁ à C₅, di-(alkyle en C₁ à C₅)-amino, alkylcarbonylamino en C₁ à C₅, alkyle en C₁ à C₅-sulfonylamino, N-(alkylsulfonyle en C₁ à C₅)-alkylamino en C₁ à C₅, un groupe cycloalkylcarbonylamino en C₃ à C₆ ou un groupe morpholinyle, thiomorpholinyle, pyrrolidinyle, pipéridinyle, pipérazinyle, tétrahydrofuranyle, tétrahydropyranyle, où les cycles carbo et hétéro précédemment cités peuvent être substitués respectivement dans le cycle par 1 à 4 groupes alkyle en C₁ à C₃ ou alkylcarbonyle en C₁ à C₃, ou respectivement par 1 à 2 groupes oxo, et/ou
où les atomes d'hydrogène des atomes de carbone hybridés par sp² du groupe alcényle en C₂ à C₆ à chaîne linéaire ou ramifiée peuvent être remplacés en totalité ou en partie par des atomes de fluor, ou
un groupe nitrile, carboxy, aminocarbonyle, alkylaminocarbonyle en C₁ à C₅, cycloalkylaminocarbonyle en C₃ à C₆, di-(alkyle en C₁ à C₅)-aminocarbonyle, alkyloxycarbonyle en C₁ à C₅ ou cycloalkylène-iminocarbonyle en C₄ à C₇ dans lequel éventuellement, un groupe méthylène peut être remplacé par un atome d'oxygène, de soufre ou d'azote substitué par un groupe alkyle en C₀ à C₃, ou
un groupe phényle, hétéroaryle mono- ou bicyclique, phényl-alkyle en C₁ à C₅ ou hétéroaryle mono- ou bicyclique-alkyle en C₁ à C₅,
qui dans la partie phényle ou hétéroaryle peut être substitué éventuellement une à trois fois par des substituants identiques ou différents choisis dans le groupe comprenant les atomes de fluor, de chlore, de brome et d'iode et un groupe alkyle en C₁ à C₅, trifluorométhyle, amino, alkyle en C₁ à C₅-amino, di-(alkyle en C₁ à C₅)-amino, hydroxy, alkyloxy en C₁ à C₅, mono-, di- ou trifluorométhoxy, carboxy et alkyloxycarbonyle en C₁ à C₅, et
lorsque -L-E-G-J- désigne un groupe -C-C-C-C-, R⁴ peut représenter sur E ou G, également un atome de fluor ou un groupe hydroxy, méthoxy, alcényle en C₃ à C₅-oxy, alcinyle en C₃ à C₅-oxy, alkyle en C₂ à C₅-oxy, cycloalkyle en C₃ à C₆-oxy, alkylaminocarbonyloxy en C₁ à C₅, di-(alkyle en C₁ à C₅)-aminocarbonyloxy ou cycloalkylène-iminocarbonyloxy en C₄ à C₇, phénylalkyloxy en C₀ à C₃, hétéroaryl-alkyloxy en C₀ à C₃, amino, alkylamino en C₁ à C₅, di-(alkyle en C₁ à C₅)-amino, cycloalkylène-imino en C₄ à C₇, acylamino en C₁ à C₃, (acyle en C₁ à C₃)-alkylamino en C₁ à C₃, alkyloxycarbonylamino en C₁ à C₅, alkylaminocarbonylamino en C₁ à C₅, di-(alkyle en C₁ à C₅)-aminocarbonylamino ou cycloalkylène-iminocarbonylamino en C₄ à C₇,
où les groupes méthyle ou méthylène présents dans les radicaux alkyle ou cycloalkyle précédemment cités peuvent être substitués respectivement, indépendamment les uns des autres, par un substituant choisi parmi les groupes morpholinyle, thiomorpholinyle, pyrrolidinyle, pipéridinyle, pipérazinyle, tétrahydrofuranyle, tétrahydropyranyle, diméthylaminocarbonyle, alkyloxycarbonyle en C₁ à C₅, carboxy, méthyle, hydroxy, méthoxy ou amino,
et
les radicaux phényle ou hétéroaryle précédemment cités peuvent être substitués éventuellement une à trois fois par des substituants identiques ou différents choisis dans le groupe comprenant des atomes de fluor, de chlore, de brome et d'iode, et un groupe alkyle en C₁ à C₅, trifluorométhyle, amino, alkyle en C₁ à C₅-amino, di-(alkyle en C₁ à C₅)-amino, hydroxy, alkyloxy en C₁ à C₅, mono-, di- ou trifluorométhoxy, carboxy et
alkyloxycarbonyle en C₁ à C₅,
à condition
que deux hétéroatomes du groupe comprenant l'oxygène et l'azote soient séparés l'un de l'autre par exactement un groupe -CH₂- éventuellement substitué, et/ou
que deux atomes forment une liaison -O-O- ou -S-O-,
soit exclus, et
R⁵ désigne un atome d'hydrogène, un groupe alkyle en C₁ à C₅, alcényle en C₂ à C₅ ou alcinyle en C₂ à C₅ ou un groupe phényl-alkyle en C₀ à C₅, où le groupe alkyle pouvant être substitué par un groupe hydroxy, méthoxy, hydroxycarbonyle ou alcoxycarbonyle en C₁ à C₅,
ou dans la mesure où R⁵ est relié à E ou G, un groupe hydroxy ou méthoxy peut également être représenté, ou
R⁴ et R⁵ dans la mesure où ils sont liés au même atome de carbone, peuvent former conjointement avec l'atome de carbone, un groupe -C=O- ou un groupe -CF₂-, ou
R⁴ et R⁵ dans la mesure où ils sont liés au même atome de carbone ou à deux atomes de carbone voisins, peuvent former conjointement avec le ou les atomes de carbone, un carbocycle de 3 à 7 chaînons ou un carbocycle simplement insaturé de 5 à 7 chaînons,
où l'un des chaînons de la chaîne de carbone de ce cycle peut être remplacé par un atome d'oxygène ou de soufre ou un groupe -NH-, -N(alkyle en C₁ à C₅), N-(alkylcarbonyle en C₁ à C₄) ou carbonyle, sulfinyle ou sulfonyle, et/ou
où deux chaînons de la chaîne de carbone directement voisins de ces carbocycles en C₄ à C₇ peuvent être substitués conjointement par un groupe -C(O)NH-, - C(O)N(alkyle en C₁ à C₅), -S(O)₂NH- ou - S(O)₂N(alkyle en C₁ à C₅), et/ou
où quatre chaînons de la chaîne de carbone directement voisins de ces carbocycles en C₅ à C₇ peuvent être substitués conjointement par un groupe -O-CH₂-CH₂-O, et/ou
où 1 à 3 atomes de carbone de ces cycles de 3 à 7 chaînons peuvent être substitués éventuellement, indépendamment les uns des autres par respectivement un ou deux atomes de fluor ou un ou deux groupes alkyle en C₁ à C₅ ou un groupe hydroxy, formyloxy, alkyloxy en C₁ à C₅, alkylcarbonyloxy en C₁ à C₅, amino, alkylamino en C₁ à C₅, di-(alkyle en C₁ à C₅)-amino, cycloalkylène-imino en C₄ à C₇, alkylcarbonylamino en C₁ à C₅, cycloalkyl-carbonylamino en C₃ à C₆, nitrile, carboxy-alkyle en C₁ à C₅, alkyloxycarbonyle en C₁ à C₅-alkyle en C₁ à C₅, carboxy, alkyloxycarbonyle en C₁ à C₅, aminocarbonyle, alkylaminocarbonyle en C₁ à C₅, di-(alkyle en C₁ à C₅)-aminocarbonyle ou cycloalkylène-iminocarbonyle en C₄ à C₇,
à condition qu'un tel cycle formé conjointement par R⁴ et R⁵
dans lequel deux atomes d'azote ou un atome d'azote et un atome d'oxygène dans le cycle sont séparés l'un de l'autre par exactement un groupe -CH₂-éventuellement substitué, et/ou
dans lequel deux atomes du cycle forment une liaison -O-O- ou une liaison -S-O-,
soit exclus,
ou le fragment 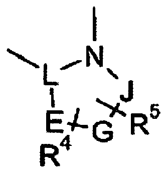 représente le groupe 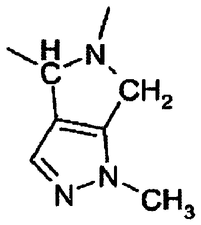 R¹³ désigne un atome d'hydrogène ou un groupe alkyle en C₁ à C₅,
M désigne un cycle phényle, thiényle ou pyridyle éventuellement substitué par R² et R⁶, dans lequel
R² désigne un atome de fluor, de chlore, de brome ou d'iode ou un groupe méthyle, éthyle, vinyle, méthoxy, éthinyle, cyano ou -C(O)NH₂-, et
R⁶ désigne un atome d'hydrogène, de fluor, de chlore, de brome ou d'iode ou un groupe hydroxy, méthoxy, trifluorométhoxy, un groupe alkyle en C₁ à C₃ éventuellement substitué par des atomes de fluor, un groupe cyano, amino ou NH₂C(O)-,
où, sauf indication contraire, l'expression « groupe hétéroaryle » mentionnée précédemment dans les définitions signifie un groupe hétéroaryle monocyclique de 5 ou 6 chaînons, où
le groupe hétéroaryle à 6 chaînons contenant un, deux ou trois atomes d'azote, et
le groupe hétéroaryle à 5 chaînons contient un groupe imino éventuellement substitué par un groupe alkyle en C₁ à C₃, un atome d'oxygène ou un atome de soufre, ou
contient un groupe imino éventuellement substitué par un groupe alkyle en C₁ à C₃ ou un atome d'oxygène ou de soufre et en outre, un ou deux atomes d'azote, ou
contient un groupe imino éventuellement substitué par un groupe alkyle en C₁ à C₃ et trois atomes d'azote,
et en outre, un cycle phényle éventuellement substitué par un atome de fluor, de chlore ou de brome, un groupe alkyle en C₁ à C₃, hydroxy, alkyloxy en C₁ à C₃, amino, alkylamino en C₁ à C₃, di-(alkyle en C₁ à C₃), amino ou cycloalkylène-imino en C₃ à C₆ sur les groupes hétéroaryle monocycliques mentionnés précédemment, peut être condensé par le biais de deux atomes de carbone voisins,
et la liaison s'effectue par le biais d'un atome d'azote ou d'un atome de carbone de la partie hétérocyclique ou d'un cycle phényle condensé,
et où, sauf indication contraire, l'expression « halogénoatome » mentionnée dans les définitions précédentes signifie un atome du groupe comprenant le fluor, le chlore, le brome et l'iode,
et où les groupes alkyle, alcényle, alcinyle et alkyloxy mentionnés dans les définitions précédentes, qui présentent plus de deux atomes de carbone, sauf indication contraire, peuvent être à chaîne linéaire ou ramifiée et les groupes alkyle dans les radicaux dialkylés précédemment cités par exemple les groupes dialkylamino peuvent être identiques ou différents,
et où les atomes d'hydrogène des groupes méthyle ou éthyle contenus dans les définitions précédemment citées, sauf indication contraire, peuvent être remplacés en totalité ou en partie par des atomes de fluor,
leurs tautomères, leurs énantiomères, leurs diastéréomères, leurs mélanges et leurs sels.

2. Composés de formule générale (I) selon la revendication 1, dans lesquels
D est un système cyclique bicyclique substitué de formule 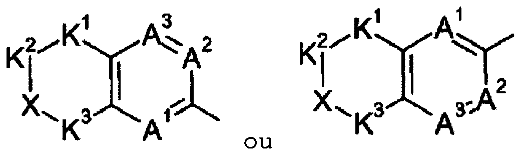 dans lequel
K¹
désigne un groupe -CH₂-, -CHR^{7a}-, -CR^{7b}R^{7c}- ou
-C(O)-, et où
R^{7a}/R^{7b}/R^{7c}
désignent respectivement, indépendamment les uns des autres, un atome de fluor, un groupe hydroxy, alcoxy en C₁ à C₅, alkyle en C₁ à C₅,
où, de façon non concomitante, les deux radicaux R^{7b}/R^{7c} peuvent être liés à l'atome de carbone cyclique par un hétéroatome, à l'exception de -C(R^{7b}R^{7c})- correspondant à un groupe -CF₂-, ou
deux radicaux R^{7b}/R^{7c} conjointement avec l'atome de carbone cyclique peuvent former un carbocycle à 3 chaînons et
K² et K³
désignent respectivement, indépendamment l'un de l'autre, un groupe -CH₂-, -CHR^{8a}, - CR^{8b}R^{8c}- ou un groupe -C(O)-, où R^{8a}/R^{8b}/R^{8c}
peuvent désigner respectivement, indépendamment les uns des autres, un groupe alkyle en C₁ à C₅,
et/ou
deux radicaux R^{8b}/R^{8c} conjointement avec l'atome de carbone cyclique peuvent former un carbocycle à 3 chaînons saturé
et
au total, au maximum quatre radicaux choisis parmi R^{7a}, R^{7b}, R^{7c}, R^{8a}, R^{8b} et R^{8c} peuvent être présents, et
X est un atome d'oxygène ou de soufre, un groupe sulfène, sulfone, -CF₂- ou NR¹, dans lequel R¹ désigne un atome d'hydrogène ou un groupe hydroxy, alkyloxy en C₁ à C₃, amino, alkylamino en C₁ à C₃, di-(alkyle en C₁ à C₃)-amino, un groupe alkyle en C₁ à C₅, alcényle en C₂ à C₅-CH₂, alcinyle en C₂ à C₅-CH₂ ou cycloalkyle en C₃ à C₆,
et dans lequel
A¹ désigne soit N soit CR¹⁰,
A² désigne soit N soit CR¹¹,
A³ désigne soit N soit CR¹²,
où R¹⁰, R¹¹ et R¹² sont respectivement, indépendamment les uns des autres
un atome d'hydrogène, de fluor, de chlore, de brome ou d'iode ou un groupe alkyle en C₁ à C₅, -CF₃-, un groupe cyano, carboxy, alkyloxycarbonyle en C₁ à C₅, hydroxy, alkyloxy en C₁ à C₃, -CF₃O-, -CHF₂O-, -CH₂FO-, amino, alkylamino en C₁ à C₅, di-(alkyle en C₁ à C₅) - amino ou cycloalkylène-imino en C₄ à C₇,
leurs tautomères, leurs énantiomères, leurs diastéréomères, leurs mélanges et leurs sels.

3. Composés de formule générale (I) selon la revendication 1 ou 2, dans lesquels
X désigne un groupe NR¹ dans lequel
R¹ désigne un atome d'hydrogène ou un groupe alkyle en C₁ à C₅, allyle ou cyclopropyle, et
A¹ est CR¹⁰,
A² est CR¹¹,
A³ est soit N soit CR¹²,
où R¹⁰, R¹¹ et R¹² sont respectivement, indépendamment les uns des autres
un atome d'hydrogène, de fluor ou de chlore ou un groupe méthyle, -CF₃-, cyano, carboxy, alkyloxycarbonyle en C₁ à C₅, hydroxy, méthoxy, -CF₃O-,
-CHF₂O-, -CH₂FO-,
leurs tautomères, leurs énantiomères, leurs diastéréomères, leurs mélanges et leurs sels.

4. Composés de formule générale (I) selon l'une quelconque des revendications 1, 2 ou 3, dans lesquels R⁴ est un atome d'hydrogène ou
un groupe alkyle en C₁ à C₆ à chaîne linéaire ou ramifiée,
où les atomes d'hydrogène des fragments méthylène et/ou méthyle du groupe alkyle en C₁ à C₆ à chaîne linéaire ou ramifiée peuvent être remplacés éventuellement, en totalité ou en partie par des atomes de fluor, et/ou
où les atomes d'hydrogène des fragments méthylène et/ou méthyle du groupe alkyle en C₁ à C₆ à chaîne linéaire ou ramifiée peuvent être remplacés éventuellement, respectivement, indépendamment les uns des autres par un substituant choisi parmi un groupe hydroxy, alkyloxy en C₁ à C₅, carboxy, alkyloxycarbonyle en C₁ à C₅, aminocarbonyle, alkylamino-carbonyle en C₁ à C₅, di-(alkyle en C₁ à C₅)-aminocarbonyle, cycloalkylène-iminocarbonyle en C₄ à C₇, amino, alkylamino en C₁ à C₅, di-(alkyle en C₁ à C₅)-amino, alkylcarbonylamino en C₁ à C₅, alkyle en C₁ à C₅-sulfonylamino, N-(alkylsulfonyle en C₁ à C₅)-alkylamino en C₁ à C₅, cycloalkylcarbonylamino en C₃ à C₆, ou
un groupe nitrile, carboxy, aminocarbonyle, alkylaminocarbonyle en C₁ à C₅, cycloalkylaminocarbonyle en C₃ à C₆, di-(alkyle en C₁ à C₅) - aminocarbonyle, alkyloxycarbonyle en C₁ à C₅ ou cycloalkylène-iminocarbonyle en C₄ à C₇ dans lequel éventuellement, un groupe méthylène peut être remplacé par un atome d'oxygène, de soufre ou d'azote substitué par un groupe alkyle en C₀ à C₃, ou
lorsque -L-E-G-J- désigne un groupe -C-C-C-C-, R⁴ peut représenter sur E ou G, également un atome de fluor ou un groupe hydroxy, méthoxy, alcényle en C₃ à C₅-oxy, alcinyle en C₃ à C₅-oxy, alkyle en C₂ à C₅-oxy, cycloalkyle en C₃ à C₆-oxy, alkylaminocarbonyloxy en C₁ à C₅, di-(alkyle en C₁ à C₅)-aminocarbonyloxy ou cycloalkylène-iminocarbonyloxy en C₄ à C₇, phénylalkyloxy en C₀ à C₂, qui peut être substitué dans le cycle phényle par 1 à 2 atomes de fluor ou des groupes méthoxy, un groupe amino, alkylamino en C₁ à C₅, di-(alkyle en C₁ à C₅)-amino, cycloalkylène-imino en C₄ à C₇, acylamino en C₁ à C₃, (acyle en C₁ à C₃) - alkylamino en C₁ à C₃, alkyloxycarbonylamino en C₁ à C₅, alkylaminocarbonylamino en C₁ à C₅, di-(alkyle en C₁ à C₅)-aminocarbonylamino ou un groupe cycloalkylène-iminocarbonylamino en C₄ à C₇,
où les groupes méthyle ou méthylène présents dans les radicaux alkyle ou cycloalkyle précédemment cités peuvent être substitués respectivement, indépendamment les uns des autres, par un substituant choisi parmi les groupes diméthylaminocarbonyle, alkyloxycarbonyle en C₁ à C₅, carboxy, méthyle, hydroxy, méthoxy ou amino,
à condition
que deux hétéroatomes du groupe comprenant l'oxygène et l'azote séparés l'un de l'autre par exactement un groupe -CH₂- éventuellement substitué, et/ou
que deux atomes formant une liaison -O- O- ou -S-O-
soit exclus, et
R⁵ désigne un atome d'hydrogène ou un groupe alkyle en C₁ à C₅, allyle, propargyle ou benzyle, ou dans la mesure où R⁵ est relié à E ou G, un groupe hydroxy ou méthoxy peut également être représenté, ou
R⁴ et R⁵ dans la mesure où ils sont liés au même atome de carbone, peuvent former conjointement avec l'atome de carbone, un groupe -C=O- ou un groupe -CF₂-, ou
R⁴ et R⁵ dans la mesure où ils sont liés au même atome de carbone ou à deux atomes de carbone voisins, peuvent former conjointement avec le ou les atomes de carbone, un carbocycle de 3 à 7 chaînons,
où l'un des chaînons de la chaîne de carbone de ce cycle peut être remplacé par un atome d'oxygène ou de soufre ou un groupe -NH-, - N(alkyle en C₁ à C₅), N(alkylcarbonyle en C₁ à C₄) ou carbonyle, sulfinyle ou sulfonyle, et/ou
où deux chaînons de la chaîne de carbone directement voisins de ces carbocycles en C₄ à C₇ peuvent être substitués conjointement par un groupe -C(O)NH-, -C(O)N(alkyle en C₁ à C₅), -S(O)₂NH- ou -S(O)₂N(alkyle en C₁ à C₅), et/ou
où quatre chaînons de la chaîne de carbone directement voisins de ces carbocycles en C₅ à C₇ peuvent être substitués conjointement par un groupe -O-CH₂-CH₂-O,
à condition qu'un tel cycle formé conjointement par R⁴ et R⁵,
dans lequel deux atomes d'azote ou un atome d'azote et un atome d'oxygène dans le cycle sont séparés l'un de l'autre par exactement un groupe -CH₂- éventuellement substitué, et/ou
dans lequel deux atomes du cycle forment une liaison -O-O- ou une liaison -S-O-
soit exclus,
leurs tautomères, leurs énantiomères, leurs diastéréomères, leurs mélanges et leurs sels.

5. Composés de formule générale (I) selon l'une quelconque des revendications 1, 2, 3 ou 4, dans lesquels
-L-E-G-J- désigne un groupe -C-C-C-C- qui peut être substitué par R⁴ et R⁵ qui sont tels que définis dans les revendications 1, 2, 3 ou 4,
leurs tautomères, leurs énantiomères, leurs diastéréomères, leurs mélanges et leurs sels.

6. Composés de formule générale (I) selon l'une quelconque des revendications 1, 2, 3, 4 ou 5, dans lesquels
D désigne un système cyclique bicyclique substitué de formule générale 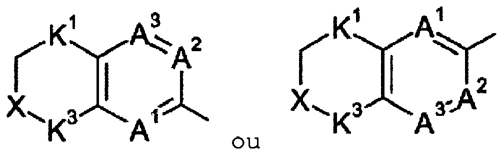 dans lequel
K¹ est un groupe -CH₂-, -CHR^{7a}-, -CR^{7b}R^{7c}- ou -C(O)-, où
R^{7a} est un groupe alkyle en C₁ à C₂ et
R^{7b}/R^{7c} désignent respectivement,
indépendamment les uns des autres, un groupe hydroxy, méthoxy ou alkyle en C₁ à C₃,
où, de façon non concomitante, les deux radicaux R^{7b}/R^{7c} peuvent être liés à l'atome de carbone cyclique par un atome d'oxygène, ou
deux radicaux R^{7b}/R^{7c} conjointement avec l'atome de carbone cyclique peuvent former un carbocycle à 3 chaînons,
et
K² et K³ désignent respectivement, indépendamment l'un de l'autre, un groupe -CH₂-, -CHR^{8a} ou - CR^{8b}R^{8c}-, où
R^{8a}/R^{8b}/R^{8c}
peuvent désigner respectivement, indépendamment les uns des autres, un groupe alkyle en C₁ à C₃,
et/ou
deux radicaux R^{8b}/R^{8c} conjointement avec l'atome de carbone cyclique peuvent former un carbocycle à 3 chaînons saturé
et
au total, au maximum quatre radicaux choisis parmi R^{7a}, R^{7b}, R^{7c}, R^{8a}, R^{8b} et R^{8c} pouvant être présents,
et
X est un groupe NR¹, dans lequel
R¹ désigne un atome d'hydrogène ou un groupe alkyle en C₁ à C₃, allyle ou cyclopropyle, et
A¹ est CR¹⁰,
A² est CR¹¹,
A³ est CR¹²,
où R¹⁰, R¹¹ et R¹² sont respectivement, indépendamment les uns des autres
un atome d'hydrogène, de fluor ou de chlore, ou un groupe méthyle, CF₃, hydroxy, méthoxy, CF₃O, CHF₂O, CH₂FO,
et
-L-E-G-J- est un groupe -C-C-C-C- qui peut être substitué par R⁴ et R⁵, et
R³ est un atome d'hydrogène, et
R⁴ est un atome d'hydrogène ou
un groupe alkyle en C₁ à C₃ à chaîne linéaire ou ramifiée,
où les atomes d'hydrogène des fragments méthylène et/ou méthyle du groupe alkyle en C₁ à C₆ à chaîne linéaire ou ramifiée peuvent être remplacés éventuellement, indépendamment les uns des autres par un substituant choisi parmi les groupes hydroxy, alkyloxy en C₁ à C₅, carboxy, alkyloxycarbonyle en C₁ à C₅, ou
si R⁴ est lié à E ou G, peut représenter également un atome de fluor ou un groupe hydroxy, méthoxy, alcényle en C₃ à C₅-oxy, alkyle en C₂ à C₅-oxy, cycloalkyle en C₃ à C₆-oxy, alkylaminocarbonyloxy en C₁ à C₅, di-(alkyle en C₁ à C₅)-aminocarbonyloxy ou cycloalkylène-iminocarbonyloxy en C₄ à C₇
à condition
que deux hétéroatomes du groupe comprenant l'oxygène et l'azote séparés l'un de l'autre par exactement un groupe
-CH₂- éventuellement substitué,
soit exclus, et
R⁵ est un atome d'hydrogène ou un groupe alkyle en C₁ à
C₅, allyle ou benzyle ou dans la mesure où R⁵ est relié à E ou G, un groupe hydroxy ou méthoxy peut également être représenté, ou
R⁴ et R⁵ dans la mesure où ils sont liés au même atome de carbone, peuvent former conjointement avec l'atome de carbone, un groupe -C=O- ou un groupe -CF₂-, ou
R⁴ et R⁵ dans la mesure où ils sont liés au même atome de carbone ou à deux atomes de carbone voisins, peuvent former conjointement avec le ou les atomes de carbone, un carbocycle de 3 à 6 chaînons,
où quatre chaînons de la chaîne de carbone directement voisins de ces carbocycles en C₅ à C₆ peuvent être substitués conjointement par un groupe -O-CH₂-CH₂O-,
et
R¹³ est un atome d'hydrogène,
M est un cycle phényle substitué en position 4 par R²
ou un cycle pyridyle substitué en position 5 par R², dans lequel
R² désigne un atome de fluor, de chlore, de brome, un groupe méthoxy ou éthinyle, et
R⁶ désigne un atome d'hydrogène ou de fluor,
leurs tautomères, leurs énantiomères, leurs diastéréomères, leurs mélanges et leurs sels.

7. Composés de formule générale (I) selon l'une quelconque des revendications 1, 2, 3, 4, 5 ou 6, dans lesquels le cycle central 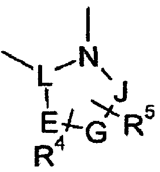 est soit 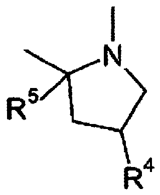 soit 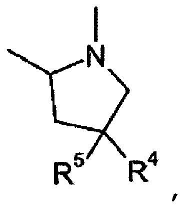 leurs tautomères, leurs énantiomères, leurs diastéréomères, leurs mélanges et leurs sels.

8. Composés de formule générale (I) selon l'une quelconque des revendications 1, 2, 3, 4, 5, 6 ou 7, dans lesquels
D est un système cyclique bicyclique substitué de formule générale 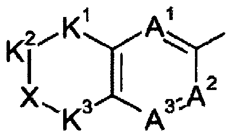 leurs tautomères, leurs énantiomères, leurs diastéréomères, leurs mélanges et leurs sels.

9. Composés de formule générale (I) selon l'une quelconque des revendications 1, 2, 3, 4, 5, 6, 7 ou 8, qui ont une configuration R sur les chaînons G et L du cycle central à 5 chaînons,
leurs tautomères, leurs énantiomères, leurs diastéréomères, leurs mélanges et leurs sels.

10. Composé selon la revendication 1, choisi dans le groupe comprenant : 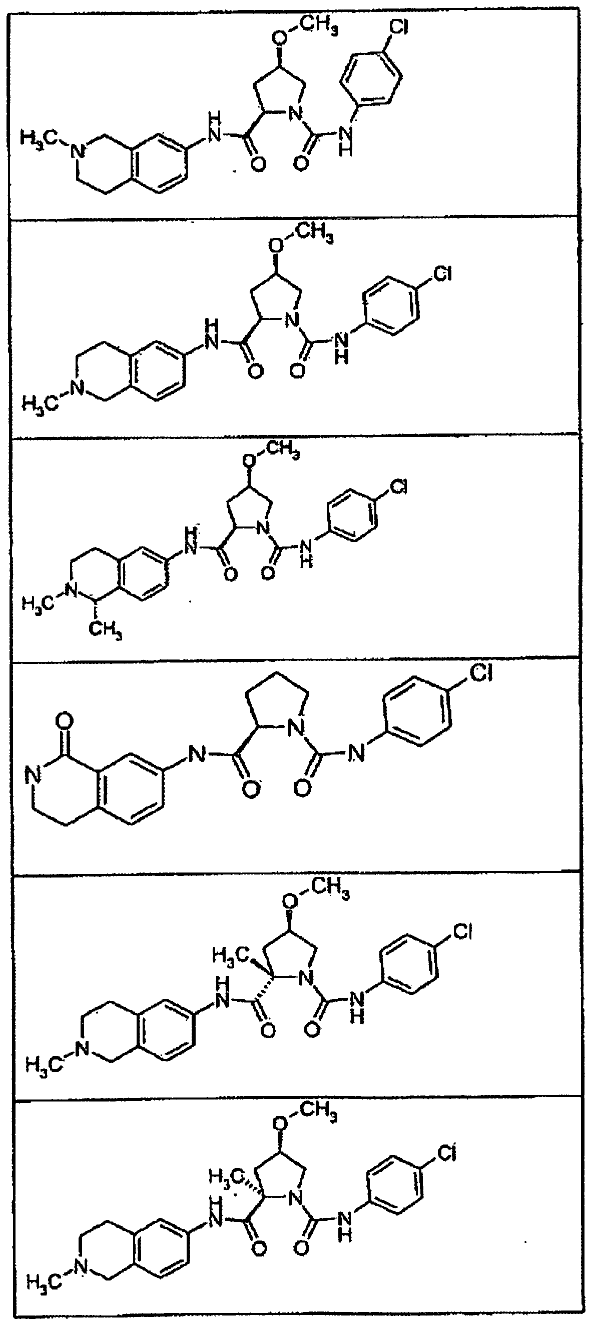 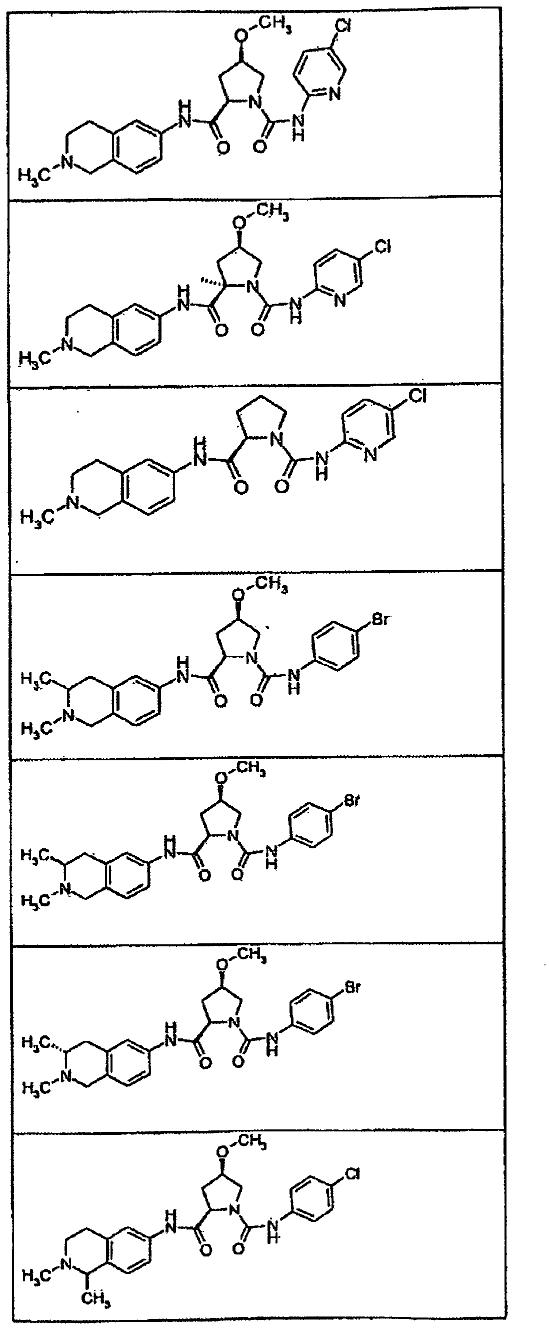 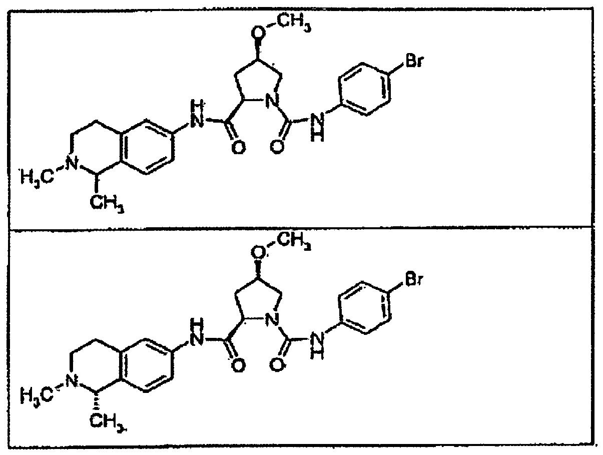 leurs tautomères, leurs énantiomères, leurs diastéréomères, leurs mélanges et leurs sels.

11. Composé selon la revendication 1, choisi dans le groupe comprenant : 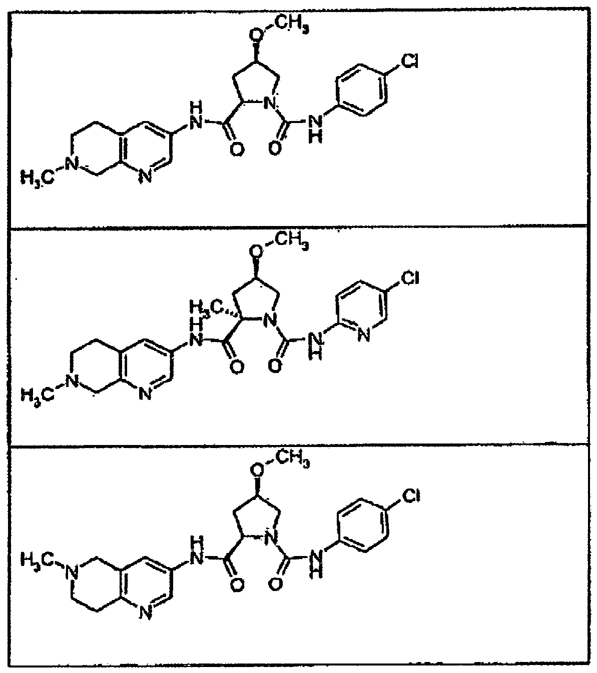 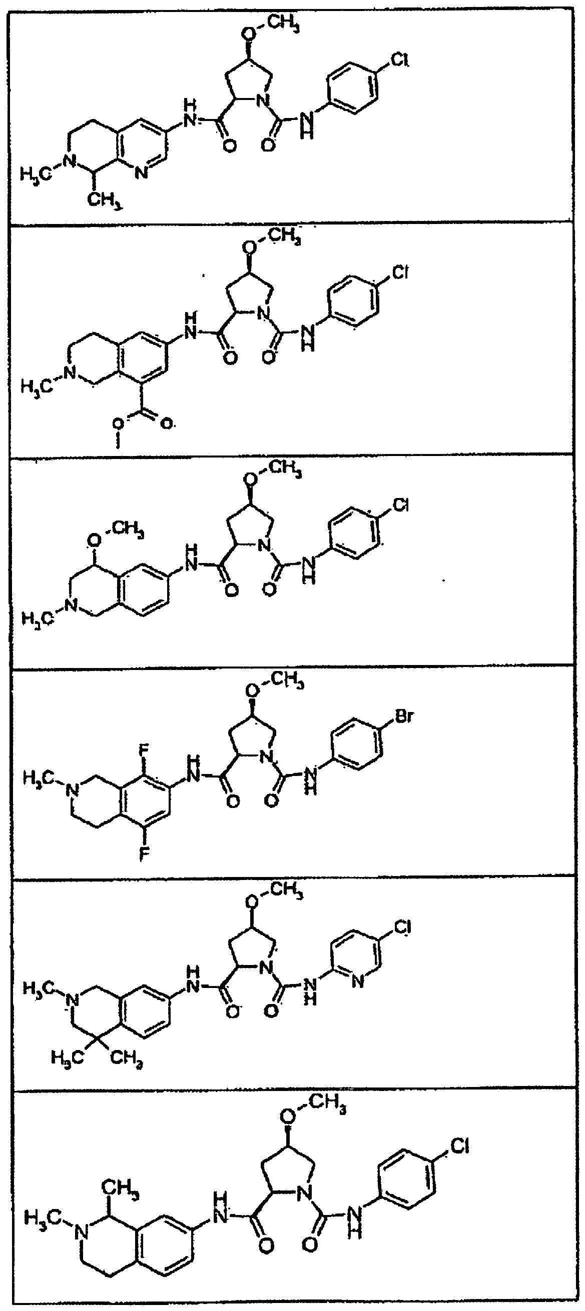 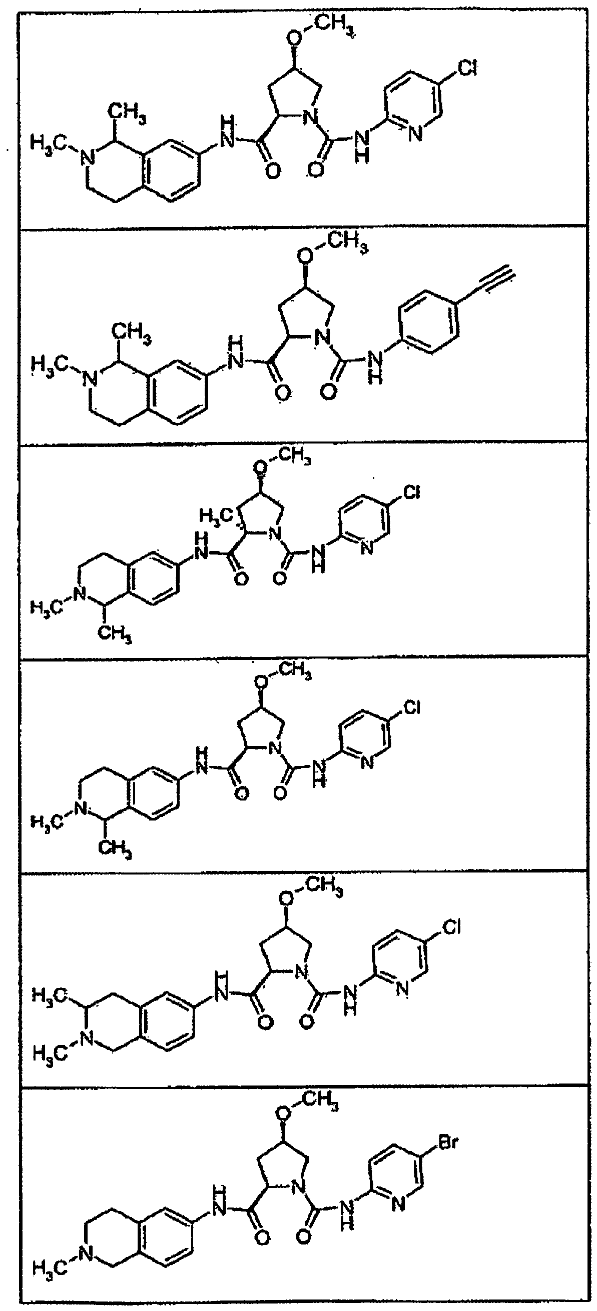 leurs tautomères, leurs énantiomères, leurs diastéréomères, leurs mélanges et leurs sels.

12. Composé selon la revendication 1, choisi dans le groupe comprenant : 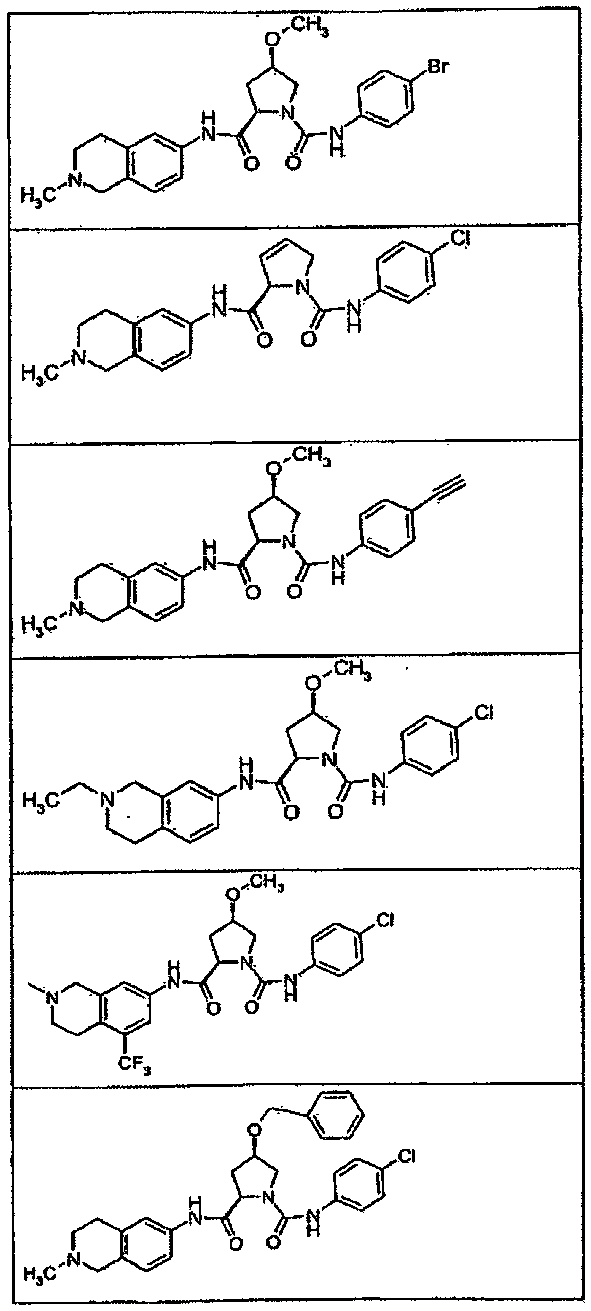 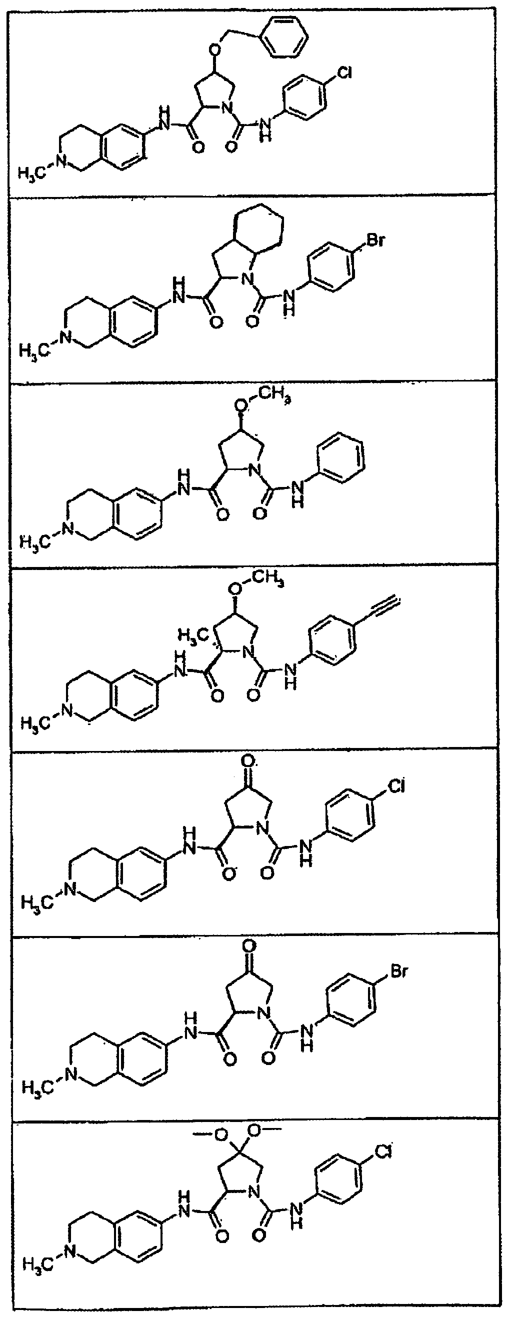 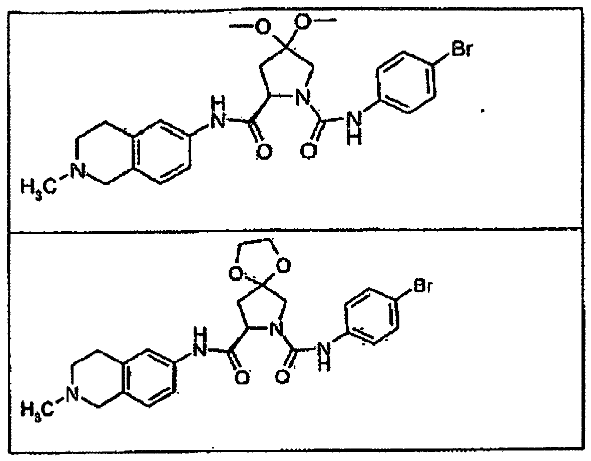 leurs tautomères, leurs énantiomères, leurs diastéréomères, leurs mélanges et leurs sels.

13. Composé selon la revendication 1, choisi dans le groupe comprenant : 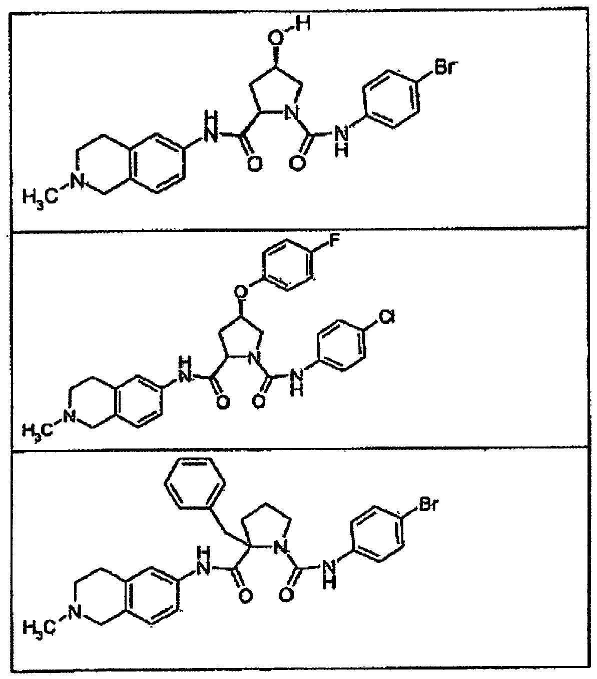 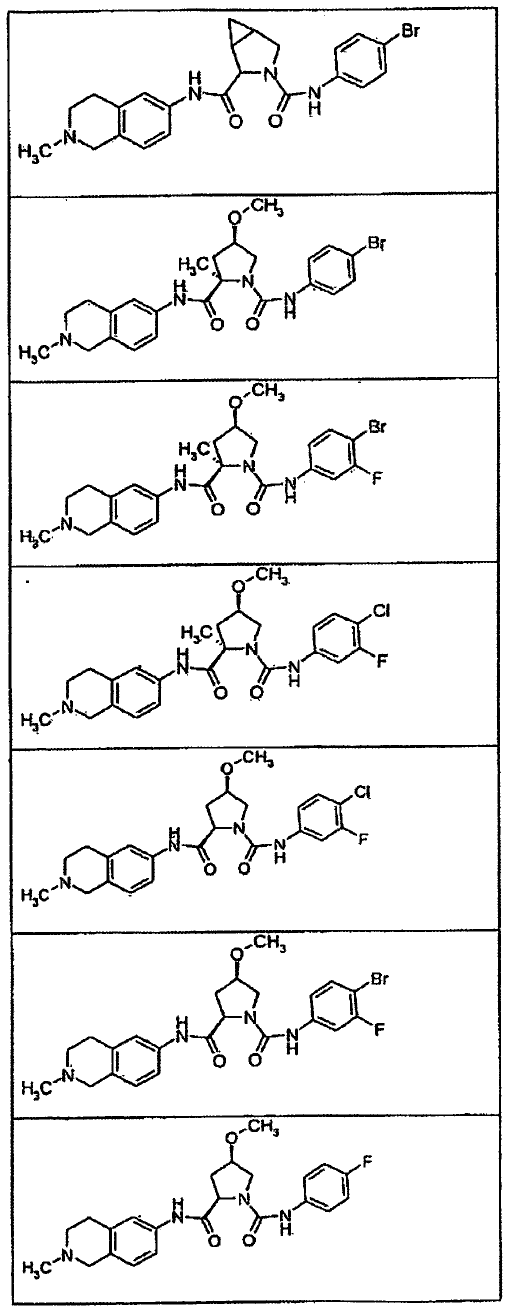 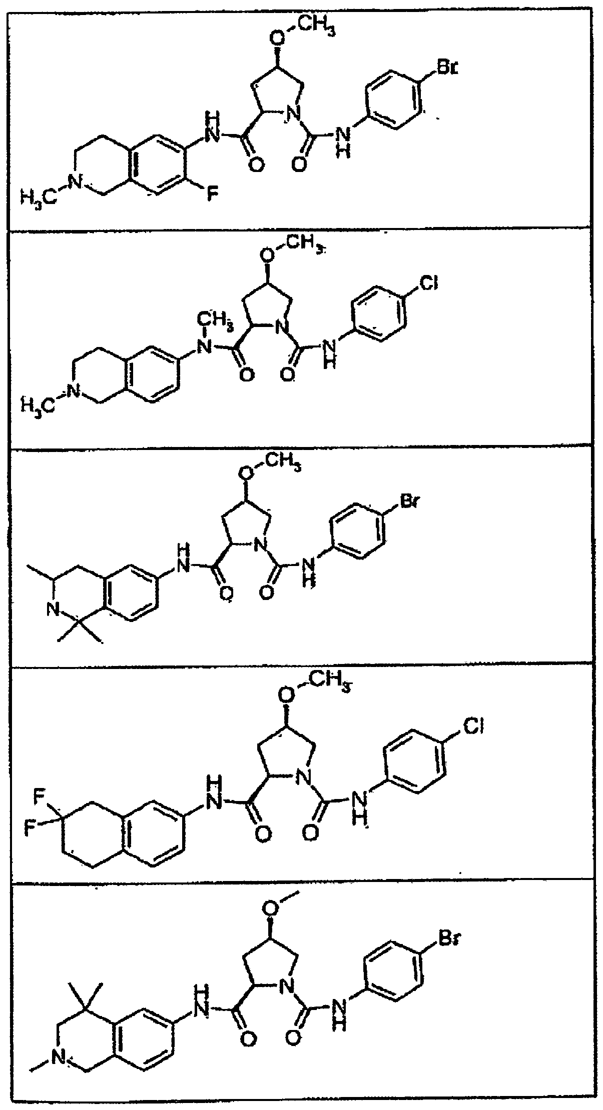 leurs tautomères, leurs énantiomères, leurs diastéréomères, leurs mélanges et leurs sels.

14. Composé selon la revendication 1, choisi dans le groupe comprenant : 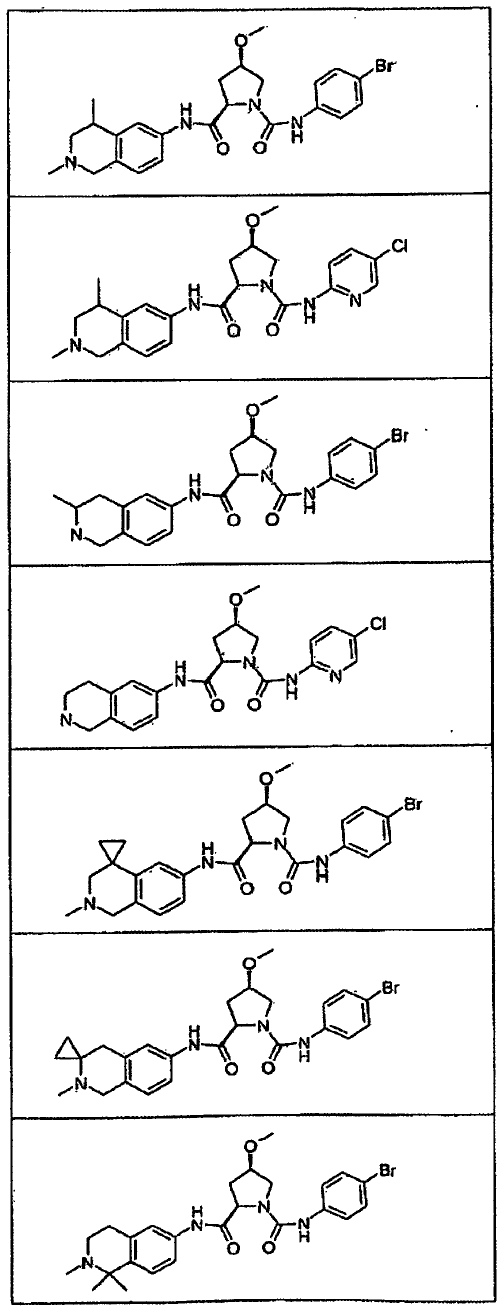 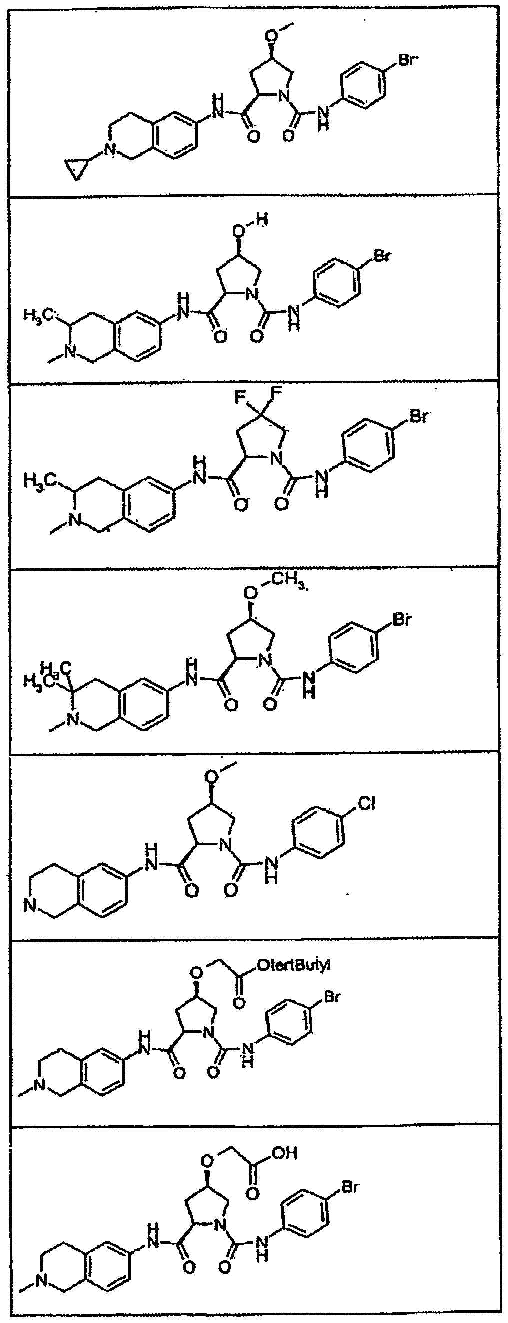 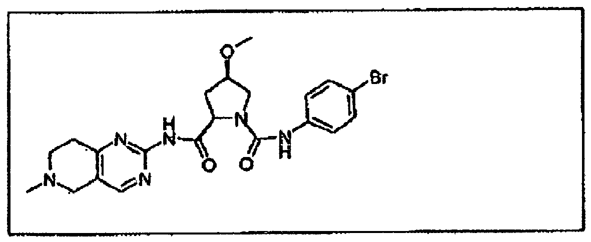 leurs tautomères, leurs énantiomères, leurs diastéréomères, leurs mélanges et leurs sels.

15. Sels physiologiquement acceptables des composés selon l'une quelconque des revendications 1 à 14.

16. Médicament contenant un composé selon au moins l'une quelconque des revendications 1 à 14 ou un sel physiologiquement acceptable selon la revendication 15, parallèlement à éventuellement un ou plusieurs véhicules et/ou diluants inertes.

17. Composé selon au moins l'une quelconque des revendications 1 à 14 ou sel physiologiquement acceptable selon la revendication 15 pour l'utilisation comme médicament.

18. Procédé de production d'un médicament selon la revendication 16, **caractérisé en ce que**, de façon non chimique, un composé selon au moins l'une quelconque des revendications 1 à 14 ou un sel physiologiquement acceptable de celui-ci selon la revendication 15, est incorporé dans un ou plusieurs véhicules et/ou diluants inertes.
